# EUROPÄISCHE PATENTSCHRIFT

(11) **EP 1 492 771 B1**
(45) Veröffentlichungstag und Bekanntmachung des Hinweises auf die Patenterteilung: **28.02.2007**
(21) Anmeldenummer: 03745195.2
(22) Anmeldetag: 29.03.2003
(51) Int. Cl.: C07D 215/40, C07D 405/12, C07D 217/24, A61K 31/47, A61P 29/00

(54) **CHINOLIN- UND ISOCHINOLIN-DERIVATE, EIN VERFAHREN ZU IHRER HERSTELLUNG UND IHRE VERWENDUNG ALS ENTZÜNDUNGSHEMMER**
QUINOLINE AND ISOQUINOLINE DERIVATIVES, METHOD FOR THE PRODUCTION THEREOF AND USE THEREOF AS ANTI-INFLAMMATORY AGENTS
DERIVES DE QUINOLEINE ET D'ISOQUINOLEINE, PROCEDE DE PRODUCTION DE CES DERIVES ET LEUR UTILISATION COMME AGENTS ANTI-INFLAMMATOIRES

(30) Priorität: 02.04.2002 DE 10215316
(43) Veröffentlichungstag der Anmeldung: 05.01.2005
(73) Patentinhaber: Schering Aktiengesellschaft, 13353 Berlin (DE)
(72) Erfinder: JAROCH, Stefan, 10629 Berlin (DE); LEHMANN, Manfred, 12305 Berlin (DE); SCHMEES, Norbert, 13469 Berlin (DE); BERGER, Markus, 13347 Berlin (DE); REHWINKEL, Hartmut, 10961 Berlin (DE); KROLIKIEWICZ, Konrad, 12357 Berlin (DE); SKUBALLA, Werner, 13465 Berlin (DE); SCHÄCKE, Heike, 10115 Berlin (DE); SCHOTTELIUS, Arndt, Belvedere, CA 94920 (US)
(74) Vertreter: Seuss, Thomas
(86) Internationale Anmeldenummer: PCT/EP2003/003298
(87) Internationale Veröffentlichungsnummer: WO 2003/082827

(56) Entgegenhaltungen:
- WO-A-00/32584
- AOKI Y ET AL: "Inhibitory effect of a novel quinolinone derivative, TA-270, on asthmatic inflammatory responses in sensitized guinea pigs" EUROPEAN JOURNAL OF PHARMACOLOGY, AMSTERDAM, NL, Bd. 409, 15. Dezember 2000 (2000-12-15), Seiten 325-330, XP002219698 ISSN: 0014-2999

## Beschreibung

Die Erfindung betrifft Chinolin- und Isochinolin-Derivate, ein Verfahren zu ihrer Herstellung und ihre Verwendung als Entzündungshemmer.

Aus dem Stand der Technik DE 100 38 639 und WO02/10143 sind Entzündungshemmer der allgemeinen Formel 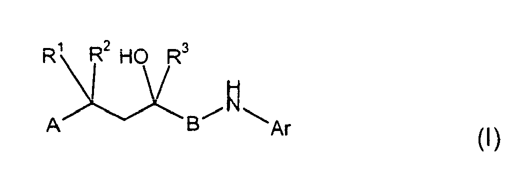 bekannt, wobei der Ar-Rest Phthalide, Thiophthalide, Benzoxazinone oder Phthalazinone umfasst. Diese Verbindungen zeigen im Experiment Wirkdissoziationen zwischen antiinflammatorischen und unerwünschten metabolischen Wirkungen und sind den bisher beschriebenen, nichtsteroidalen Glucocorticoiden überlegen oder weisen zumindest eine ebenso gute Wirkung auf.

Des weiteren sind Entzündungshemmer mit Chinolinstruktur aus AOKl. Y. et al, Europ. J. of Pharmacology, 409, 2009 p. 325-330 bekannt.

Die Selektivität der Verbindungen des Standes der Technik gegenüber den anderen Steroidrezeptoren ist jedoch noch verbesserungsbedürftig.
Daher war es Aufgabe der vorliegenden Erfindung, Verbindungen zur Verfügung zu stellen, deren Selektivität gegenüber den anderen Steroidrezeptoren verbessert ist.
Diese Aufgabe wird von den Verbindungen gemäß der Patentansprüche gelöst.

Die vorliegende Erfindung betrifft daher Verbindungen der allgemeinen Formel I 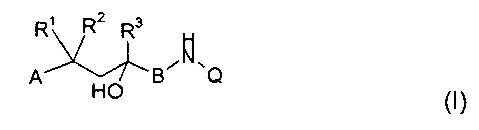 worin
- A: für eine Aryl-, eine Benzyl- oder eine Phenethylgruppe steht, wobei die Aryl-, Benzyl- oder Phenethylgruppe gegebenenfalls substituiert sein kann durch einen oder mehrere Reste aus der Gruppe C₁-C₅-Alkyl, C₁-C₅-Alkoxy, C₁-C₅-Alkylthio, C₁-C₅-Perfluoralkyl, Halogen, Hydroxy, Cyano, Nitro, -O-(CH₂)ₙ-O-, -O-(CH₂)ₙ-CH₂-, -O-CH=CH-, -(CH₂)ₙ₊₂-wobei n = 1 oder 2 ist und die endständigen Sauerstoffatome und/oder Kohlenstoffatome mit direkt benachbarten Ring-Kohlenstoffatomen verknüpft sind,
oder NR⁴R⁵,
wobei R⁴ und R⁵ unabhängig voneinander Wasserstoff, C₁-C₅-Alkyl oder (CO)-C₁-C₅-Alkyl sein können,
- R¹ und R²: unabhängig voneinander ein Wasserstoffatom, eine Methyl- oder Ethylgruppe oder gemeinsam mit dem Kohlenstoffatom der Kette einen C₃-C₆-Cycloalkylring,
- R³: eine C₁-C₃-Alkylgruppe oder eine gegebenenfalls teilweise oder vollständig fluorierte C₁-C₃-Alkylgruppe,
- B: eine gegebenenfalls durch eine Methyl- oder Ethylgruppe substituierte Methylengruppe oder eine Carbonylgruppe und
- Q: eine über eine beliebige Position verknüpfte Chinolinyl- oder Isochinolinylgruppe bedeutet, die gegebenenfalls substituiert sein kann durch einen oder mehrere Reste aus der Gruppe C₁-C₅-Alkyl, die gegebenenfalls substituiert sein kann durch 1-3 Hydroxygruppen und/oder 1-3 COOR⁶-Gruppen, C₁-C₅-Alkoxy, C₁-C₅-Alkylthio, C₁-C₅-Perfluoralkyl, Halogen, Hydroxy, ein Carbonylsauerstoffatom, Cyano, Nitro, oder NR⁴R⁵, wobei R⁴ und R⁵ unabhängig voneinander Wasserstoff, C₁-C₅-Alkyl oder (CO)-C₁-C₅-Alkyl sein können,
COOR⁶, wobei R⁶ Wasserstoff oder eine C₁-C₅-Alkylgruppe bedeuten,
(CO)NR⁷R⁸, wobei R⁷ und R⁸ unabhängig voneinander Wasserstoff oder eine C₁-C₅-Alkylgruppe bedeuten,
oder eine (C₁-C₅-Alkylen)-O-(CO)-(C₁-C₅)alkylgruppe bedeuten,
sowie deren Racemate oder getrennt vorliegenden Stereoisomeren, und gegebenenfalls deren physiologisch verträgliche Salze.

Als Gruppe A ist die Arylgruppe bevorzugt.

Die Arylgruppe umfaßt Phenyl und Naphthyl. Phenyl ist bevorzugt.

Die substituierten Aryl, Benzyl- oder Phenethylgruppen tragen am Ring 1 - 3 Substituenten, bevorzugt 2 Substituenten zusätzlich zu der Verknüpfung mit der Kette.

Folgende Substitutionsmuster am Ring A sind ein besonderer Gegenstand der Erfindung: 2,5-disubstituierte Phenylderivate und 2,4-disubstituierte Phenylderivate, wobei die 1-Position von der Verknüpfung zur Kette besetzt ist.

Ein besonderer Gegenstand der Erfindung sind Verbindungen, die am Arylrest einen oder mehrere Substituenten tragen ausgewählt aus der Gruppe C₁-C₅-Alkyl, C₁-C₅-Alkoxy, C₁-C₅-Perfluoralkyl, Halogen, Hydroxy, Nitro, -O-(CH₂)ₙ-O-, -O-(CH₂)ₙ-CH₂-, -O-CH=CH-, -(CH₂)ₙ₊₂-wobei n = 1 oder 2 ist und die endständigen Sauerstoffatome und/oder Kohlenstoffatome mit direkt benachbarten Ring-Kohlenstoffatomen verknüpft sind.

Ganz besonders bevorzugt sind Verbindungen der Formel I, worin A einen Phenylring bedeutet, der durch eine Hydroxy- oder Methoxygruppe und eine Halogengruppe substituiert ist, zusätzlich zu der Verknüpfung mit der Kette.

Die C₁-C₅-Alkylgruppen in A R³, R⁴, R⁵, R⁶, R⁷ und R⁸ können geradkettig oder verzweigt sein und für eine Methyl-, Ethyl-, n-Propyl-, iso-Propyl-, n-Butyl, isoButyl, tert.-Butyl- oder n-Pentyl-, 2,2-Dimethylpropyl-, 2-Methylbutyl- oder 3-Methylbutylgruppe stehen. Eine Methyl- oder Ethylgruppe ist bevorzugt. Sie können gegebenenfalls substituiert sein durch 1-3 Hydroxy- und/oder 1-3-COOR⁶ Gruppen. Bevorzugt sind Hydroxygruppen.

Unter einer (C₁-C₅-Alkylen)-O-(CO)-(C₁-C₅)alkylgruppe ist zum Beispiel eine -CH₂-O-CO-CH₃-Gruppe zu verstehen.

Für eine teilweise oder vollständig fluorierte C₁-C₃-Alkylgruppe kommen die teilweise oder vollständig fluorierten folgenden Gruppen in Betracht: Fluormethyl, Difluormethyl, Trifluormethyl, Fluorethyl, 1,1-Difluorethyl, 1,2-Difluorethyl, 1,1,1-Trifluorethyl, Tetrafluorethyl, Pentafluorethyl. Von diesen bevorzugt sind die Trifluormethyl- oder die Pentafluorethylgruppe.

Die Alkylreste R¹ und R² können zusammen mit dem Kohlenstoffatom der Kette einen 3 bis 6-gliedrigen Ring bilden. Bevorzugt ist der Cyclopropylring.

Die C₁-C₅-Alkoxygruppen in A und Q können geradkettig oder verzweigt sein und für eine Methoxy-, Ethoxy-, n-Propoxy-, iso-Propoxy-, n-Butoxy, iso-Butoxy, tert.-Butoxy- oder n-Pentoxy-, 2,2-Dimethylpropoxy-, 2-Methylbutoxy- oder 3-Methylbutoxygruppe stehen. Eine Methoxy- oder Ethoxygruppe ist bevorzugt.

Die C₁-C₅-Alkylthiogruppen in A und Q können geradkettig oder verzweigt sein und für eine Methylthio-, Ethylthio-, n-Propylthio-, iso-Propylthio-, n-Butylthio, iso-Butylthio, tert.-Butylthio- oder n-Pentylthio-, 2,2-Dimethylpropylthio-, 2-Methylbutylthio- oder 3-Methylbutylthiogruppe stehen. Eine Methylthio- oder Ethylthiogruppe ist bevorzugt.

Die Bezeichnung Halogenatom oder Halogen bedeutet ein Fluor-, Chlor-, Brom- oder lodatom. Bevorzugt ist ein Fluor-, Chlor- oder Bromatom.

Die NR⁴R⁵-Gruppe kann beispielsweise NH₂, N(H)CH₃, N(CH₃)₂, N(H)(CO)CH₃, N(CH₃)(CO)CH₃, N[(CO)CH₃]₂, N(H)CO₂CH₃, N(CH₃)CO₂CH₃, N(CO₂CH₃)₂, bedeuten.
Als Alkylreste R⁴ und R⁵ sind C₁-C₃-Alkyl bevorzugt.
Als Acylreste R⁴ und R⁵ sind (CO)-C₁-C₃-Alkyl bevorzugt.

Für die NR⁷R⁸-Gruppe kommt beispielsweise NH₂, N(H)CH₃, N(CH₃)₂, N(C₂H₅)₂, NH(C₂H₅), N(C₃H₇)₂, N(C₄H₉)₂, N(C₅H₁₁)₂ in Frage.

Für den Rest B sind die unsubstituierte Methylengruppe und die Carbonylgruppe bevorzugt. Besonders bevorzugt ist die Methylengruppe.

Ein Gegenstand der Erfindung sind Verbindungen der Formel I, worin Q eine über eine beliebige Position verknüpfte, gegebenenfalls substituierte Chinolinylgruppe ist.

Der Rest Q kann über jedes Ring-Kohlenstoffatom mit der (NH)-Gruppe der Kette verknüpft sein. Bevorzugt sind für den Chinolinring die 4-, 5-, und 8-Position und für den Isochinolinring die 1-Position.

Ein weiterer Gegenstand der Erfindung sind die Verbindungen der allgemeinen Formel I worin
- A: für eine Aryl-, eine Benzyl- oder eine Phenethylgruppe steht, wobei die Aryl-, Benzyl- oder Phenethylgruppe gegebenenfalls substituiert sein kann durch einen oder mehrere Reste aus der Gruppe C₁-C₅-Alkyl, C₁-C₅-Alkoxy, C₁-C₅-Alkylthio, C₁-C₅-Perfluoralkyl, Halogen, Hydroxy, Cyano, Nitro,
-O-(CH₂)ₙ-O-, -O-(CH₂)ₙ-CH₂-, -O-CH=CH-, -(CH₂)ₙ₊₂-wobei n = 1 oder 2 ist und die endständigen Sauerstoffatome und/oder Kohlenstoffatome mit direkt benachbarten Ring-Kohlenstoffatomen verknüpft sind,
oder NR⁴R⁵,
wobei R⁴ und R⁵ unabhängig voneinander Wasserstoff, C₁-C₅-Alkyl oder (CO)-C₁-C₅-Alkyl sein können,
- R¹ und R²: unabhängig voneinander ein Wasserstoffatom, eine Methyl- oder Ethylgruppe oder gemeinsam mit dem Kohlenstoffatom der Kette einen C₃-C₆-Cycloalkyfring,
- R³: eine C₁-C₃-Alkylgruppe oder eine gegebenenfalls teilweise oder vollständig fluorierte C₁-C₃-Alkylgruppe,
- B: eine gegebenenfalls durch eine Methyl- oder Ethylgruppe substituierte Methylengruppe oder eine Carbonylgruppe und
- Q: eine über eine beliebige Position verknüpfte Chinolinyl- oder Isochinolinylgruppe bedeutet, die gegebenenfalls substituiert sein kann durch einen oder mehrere Reste aus der Gruppe C₁-C₅-Alkyl, C₁-C₅-Alkoxy, C₁-C₅-Alkylthio, C₁-C₅-Perfluoralkyl, Halogen, Hydroxy, Cyano, Nitro, oder NR⁴R⁵,
wobei R⁴ und R⁵ unabhängig voneinander Wasserstoff, C₁-C₅-Alkyl oder (CO)-C₁-C₅-Alkyl sein können
sowie deren Racemate oder getrennt vorliegenden Stereoisomeren, und gegebenenfalls deren physiologisch verträgliche Salze.

Ein weiterer Gegenstand der Erfindung sind Verbindungen der allgemeinen Formel I worin Q eine über eine beliebige Position verknüpfte Chinolinyl- oder Isochinolinylgruppe bedeutet, die gegebenenfalls substituiert sein kann durch einen oder mehrere Reste aus der Gruppe C₁-C₅-Alkyl, die gegebenenfalls substituiert sein kann durch 1-3 Hydroxygruppen oder 1-3 COOR⁶-Gruppen,
ein Carbonylsauerstoffatom,
COOR⁶, wobei R⁶ Wasserstoff oder eine C₁-C₅-Alkylgruppe bedeuten, (CO)NR⁷R^{B}, wobei R⁷ und R⁸ unabhängig voneinander Wasserstoff oder eine C₁-C₅-Alkylgruppe bedeuten,
oder eine (C₁-C₅-Alkyl)-O-(CO)-(C₁-C₅)alkylgruppe bedeutet.

Ein Gegenstand der Erfindung sind Verbindungen der allgemeinen Formel I worin Q eine einfach substituierte Chinolinylgruppe darstellt.

Ein Gegenstand der Erfindung sind Verbindungen in denen B eine Methylengruppe und Q eine gegebenenfalls substituierte Chinolinylgruppe bedeutet.

Ein weiterer Gegenstand der Erfindung sind verbindungen, in denen A eine gegebenenfalls substituierte Phenylgruppe, Q eine gegebenenfalls substituierte Chinolinylgruppe und B eine Methylengruppe bedeutet.

Die erfindungsgemäßen Verbindungen der allgemeinen Formel 1 können durch das Vorhandensein von Asymmetriezentren als unterschiedliche Stereoisomere vorliegen. Sowohl die Racemate als auch die getrennt vorliegenden Stereoisomere gehören zum Gegenstand der vorliegenden Erfindung.

Ein besonderer Gegenstand der vorliegenden Erfindung sind die getrennt vorliegenden Stereoisomere, d.h. (+)-Enantiomere und (-)-Enantiomere.

Ferner zeichnen sich die erfindungsgemäßen Verbindungen, wenn sie eine Hydroxygruppe in α-Position zum Chinolinyl- oder Isochinolinyl-Stickstoffatom enthalten, durch das Vorliegen einer Keto-Enol-Tautomerie aus. Im erfindungsgemäßen Sinne gehören beide Formen zum Gegenstand der Erfindung, selbst wenn, z.B. im experimentellen Teil, nur eine der beiden tautomeren Formen aufgeführt worden ist.

Die vorliegende Erfindung betrifft weiterhin Verfahren zur Herstellung der Verbindungen der Formel I, sowie deren Verwendung zur Herstellung von Arzneimitteln. Die Verwendung zur Herstellung von Arzneimitteln zur Behandlung von Erkrankungen, deren Ursache entzündliche Prozesse sind, ist ebenfalls ein Gegenstand der Erfindung.

Die Verfahren zur Herstellung der Verbindungen aus WO98/54159, WO00/32584 und WO02/10143 können auch für die Herstellung der erfindungsgemäßen Verbindungen verwendet werden. Für die Anknüpfung der für die erfindungsgemäßen Verbindungen charakteristischen Chinolin- oder Isochinolingruppe können folgende Verfahrensschritte durchgeführt werden:
A1)
   für B = CO 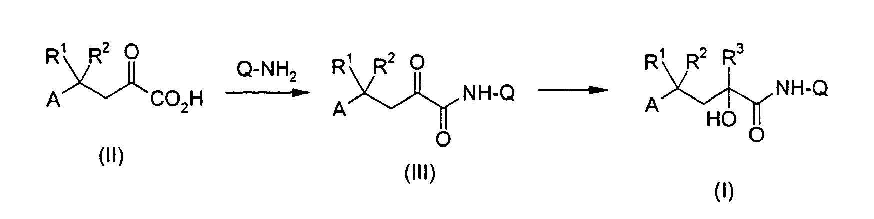
   Eine α-Ketosäure der allgemeinen Formel (II), worin A, R¹ und R² die für Formel (I) angegebenen Bedeutungen haben, wird mit einem Aminochinolin, einem Aminoisochinolin oder einem (partial-)hydrierten Chinolin- bzw. Isochinolin-Derivat (Q-NH₂) ins α-Ketoamid (III), wobei A, R¹ und R² die oben angegebene Bedeutung zukommt, in der dem Fachmann bekannten Weise übergeführt. Beispielsweise wird unter Verwendung von dehydratisierenden Kupplungsreagenzien, wie sie aus der Peptidchemie bekannt sind, z.B. Dicyclohexylcarbodiimid, oder durch vorgeschaltete Umwandlung der Säure in ein Säurechlorid, z.B. mit Thionylchlorid oder POCl₃ und nachfolgende Umsetzung mit Q-NH₂ das α-Ketoamid (III) erhalten. 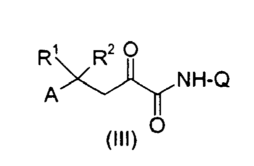
   Verbindung (III) wird entweder mit einer Alkylmetallverbindung, beispielsweise einem Grignardreagenz oder einem Lithiumalkyl, oder durch Reaktion mit Verbindung (IV),

   (R⁹)₃Si-R³ (IV)

   wobei R³ die oben angegebene Bedeutung hat und R⁹ eine C₁-C₅-Alkylgruppe bezeichnet, in Gegenwart eines Katalysators, z.B. Fluorid-Salzen oder Basen, wie etwa Alkalicarbonaten (*J. Am. Chem. Soc.* **1989,** *111*, 393), zur Titelverbindung (I) umgesetzt.
A2)
   fürB=CO 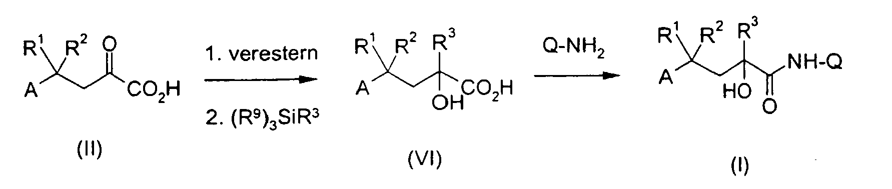
   Alternativ können auch α-Ketosäuren (II) zu Verbindungen (V), 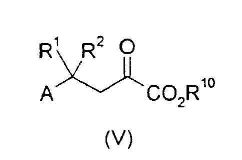 worin A, R¹ und R² wie oben beschrieben definiert sind und R¹⁰ C₁-C₄-Alkyl ist, nach den üblichen Methoden, z.B. mit Thionylchlorid in Methanol oder Ethanol oder mit Methyliodid und Alkalicarbonat, verestert werden und in Analogie zur Reaktionsfolge A**1**) von (III) in (I) in Verbindung (VI) umgewandelt werden. 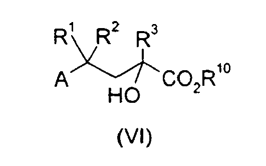
   Der Ester wird unter Standardbedingungen, etwa wäßriger Alkalihydroxidlösung, zur Säure (VI; R¹⁰ = H) verseift. Diese wird zur Kupplung mit einem Aminochinolin oder Aminoisochinolin oder einem (partial-)hydrierten Chinolin- bzw. lsochinolin-Derivat (Q-NH₂) unter Verwendung eines gängigen Aktivierungsreagenzes, z.B. Thionylchlorid, gegebenenfalls in der Gegenwart eines Katalysators wie Dimethylaminopyridin, zur Titelverbindung (I) umgesetzt.
B)
   für B = eine gegebenenfalls durch Methyl oder Ethyl substituierte Methylengruppe

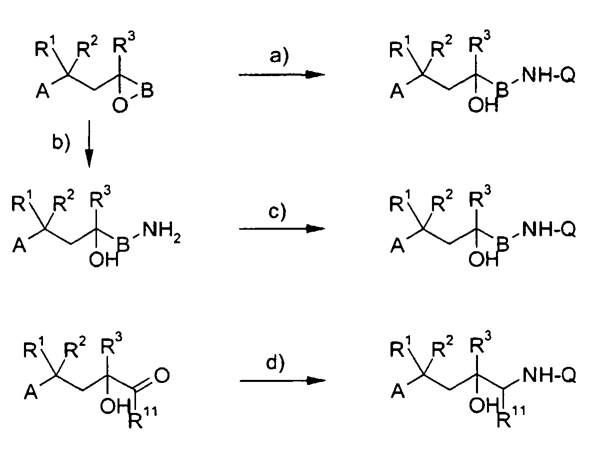

a)
   Eine Verbindung der allgemeinen Formel (VII) oder (VIII), 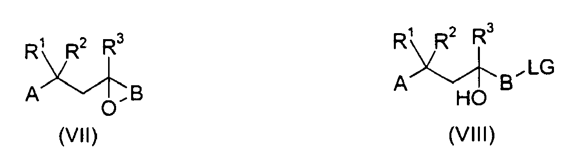 worin A, B und R¹, R² und R³ die oben angegebene Bedeutung haben und LG eine beliebige Fluchtgruppe wie Halogenid oder Sulfonat bedeutet, wird mit einer Verbindung der allgemeinen Formel (IX) oder (X) 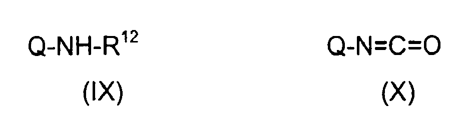 worin R¹² ein Wasserstoffatom, eine C₁-C₅-Acylgruppe oder Alkoxy- oder Aryloxycarbonylgruppe bedeutet und Q die oben angegebene Bedeutung hat, umgesetzt, wobei der Rest R¹² abgespalten oder ein intermediär gebildetes Oxazolidinon (vgl. z.B. S.J. Brickner, D.K. Hutchinson, M.R. Barbachyn, P.R.
   Manninen, D.A. Ulanowicz, S.A. Garmon, K.C. Grega, S.K. Hendges, D.S. Toops, C.W. Ford, G.E. Zurenko *J. Med. Chem.* **1996**, 39, 673) beispielsweise mit wäßrigen Alkalihydroxiden gespalten wird, um zur Titelverbindung (I) zu gelangen.
b)
   Ein anderer Weg besteht darin, Verbindungen der Formel (VII) oder (VIII) mit Stickstoffnucleophilen, beispielsweise Azid-Salzen oder Ammoniak umzusetzen, wobei sich im ersten Falle eine Reduktion in der dem Fachmann bekannten Weise, z.B. mit komplexen Hydridreagenzien, wie Lithiumaluminiumhydrid, oder durch eine Übergangsmetall-katalysierte Hydrogenolyse anschließt, um zu Verbindungen der Formel (Xl) zu gelangen. 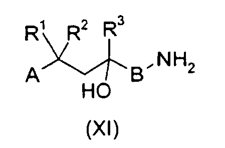 Den Resten R¹-R³, A und B kommt die gleiche Bedeutung wie oben angegeben zu.
c)
   Verbindung (XI) kann unter Basenkatalyse, z.B. in Gegenwart tertiärer Aminbasen oder Alkalicarbonaten oder -hydroxiden, oder unter Übergangsmetallkatalyse, z.B. Palladiumkatalyse (J.P. Wolfe, S. Wagaw, J.-F. Marcoux, S.L. Buchwald *Acc. Chem. Res.* **1998**, *31*, 805; J.F. Hartwig *Acc*. *Chem. Res*. **1998,** *31,* 852), mit einem halogenierten Chinolin oder Isochinolin in die Titelverbindung (I) übergeführt werden.
d)
   Schließlich läßt sich die Titelverbindung (I) auch durch reduktive Aminierung einer Verbindung der Formel (XII) mir Q-NH₂ synthetisieren, wobei z.B Natriumcyanoborhydrid oder Natriumtriacetoxyborhydrid als Reduktionsmittel in Betracht kommen. 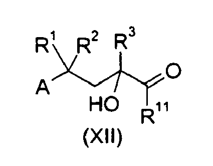 R¹¹ bedeutet Methyl oder Ethyl gemäß der für die Methylengruppe in B definierten Substituenten.

Im Falle, daß die Verbindungen der allgemeinen Formel I als Salze vorliegen, kann dies beispielsweise in der Form des Hydrochlorids, Sulfats, Nitrats, Phosphats, Pivalats, Maleats, Fumarats, Tartrats, Benzoats, Mesylats, Citrats oder Succinats sein.

Wenn die erfindungsgemäßen Verbindungen als racemische Gemische vorliegen, können sie nach dem Fachmann geläufigen Methoden der Racemattrennung in die reinen, optisch aktiven Formen aufgetrennt werden. Beispielsweise lassen sich die racemischen Gemische durch Chromatographie an einem selbst optisch aktiven Trägermaterial (CHIRALPAK AD^{®}) in die reinen Isomere trennen. Es ist auch möglich, die freie Hydroxygruppe in einer racemischen Verbindung der allgemeinen Formel I mit einer optisch aktiven Säure zu verestern und die erhaltenen diastereoisomeren Ester durch fraktionierte Kristallisation oder chromatographisch zu trennen und die getrennten Ester jeweils zu den optisch reinen Isomeren zu verseifen. Als optisch aktive Säure kann beispielsweise Mandelsäure, Camphersulfonsäure oder Weinsäure verwendet werden.

Die Bindung der Substanzen an den Glucocorticoid-Rezeptor (GR) und weitere Steroidhormon-Rezeptoren (Mineralcorticoid-Rezeptor (MR), Progesteron-Rezeptor (PR) und Androgen-Rezeptor (AR)) wird mit Hilfe rekombinant hergestellter Rezeptoren überprüft. Cytosolpräparationen von Sf9 Zellen, die mit rekombinanten Baculoviren, die für den GR kodieren, infiziert worden waren, werden für die Bindungsuntersuchungen eingesetzt. Im Vergleich zur Bezugssubstanz [³H]-Dexamethason zeigen die Substanzen eine hohe bis sehr hohe Affinität zum GR.

Darüberhinaus zeigen die hier beschriebenen Chinoline und lsochinoline der Formel (1) eine hohe Selektivität für den Glucocorticoid-Rezeptor. So zeigt Beispiel 4 z.B. folgendes Profil: IC₅₀(GR) = 0.6-1.3 nM; lC₅₀(MR), lC₅₀(PR), lC₅₀(AR) > 1 µM.

Als wesentlicher, molekularer Mechanismus für die anti-inflammatorische Wirkung von Glucocorticoiden wird die durch den GR vermittelte Hemmung der Transkription von Cytokinen, Adhäsionsmolekülen, Enzymen und anderer pro - inflammatorischen Faktoren angesehen. Diese Hemmung wird durch eine Interaktion des GR mit anderen Trankriptionsfaktoren, z.B. AP-1 und NF-kappa-B, bewirkt (zur Übersicht siehe Cato, ACB and Wade E, BioEssays 18, 371-378 1996).

Die erfindungsgemäßen Verbindungen der allgemeinen Formel I hemmen die durch Lipopolysacchard (LPS) ausgelöste Sekretion des Cytokins IL-8 in der menschlichen Monozytenzelline THP-1. Die Konzentration der Cytokine wurde im Überstand mittels kommerziell erhältlicher ELISA-Kits bestimmt.

Die anti - inflammatorische Wirkung der Verbindungen der allgemeinen Formel I wurden im Tierexperiment durch Testen in der Crotonöl - induzierten Entzündung in der Ratte und der Maus getestet (J. Exp. Med. (1995), 182, 99-108). Hierzu wurde den Tieren Crotonöl in ethanolischer Lösung topisch auf die Ohren appliziert. Die Testsubstanzen wurden gleichzeitig oder zwei Stunden vor dem Crotonöl ebenfalls topisch oder systemisch appliziert. Nach 16-24 Stunden wurden das Ohrgewicht als Maß für das entzündliche Ödem, die Peroxidaseaktivität als Maß für die Einwanderungen von Granulozyten und die Elastaseaktivität als Maß für die Einwanderung von neutrophilen Granulozyten gemessen. Die Verbindungen der allgemeinen Formel I hemmen in diesem Test sowohl nach topischer, als auch nach systemischer Applikation die drei oben genannten Entzündungsparameter.

Eine der häufigsten unerwünschten Wirkungen einer Glucocorticoid - Therapie ist der sogenannte "Steroiddiabetes" [vgl. Hatz, HJ, Glucocorticoide:

Immunologische Grundlagen, Pharmakologie und Therapierichtlinien, Wissenschafliche Verlagsgesellschaft mbH, Stuttgart, 1998]. Ursache hierfür ist die Stimulation der Gluconeogenese in der Leber durch Induktion der hierfür verantwortlichen Enzyme und durch freie Aminosäuren, die aus dem Abbau von Proteinen (katabole Wirkung der Glucocorticoide) entstehen. Ein Schlüsselenzym des katabolen Stoffwechsels in der Leber ist die Tyrosinaminotranferase (TAT). Die Aktivität dieses Enzyms kann photometrisch aus Leberhomogenaten bestimmt werden und stellt ein gutes Maß für die unerwünschten metabolischen Wirkungen der Glucocorticoide dar. Zur Messung der TAT - Induktion werden die Tiere 8 Stunden nach Gabe der Testsubstanzen getötet, die Leber entnommen und die TAT - Aktivität im Homogenat gemessen. Die Verbindungen der allgemeinen Formel I induzieren in diesem Test in Dosen, in denen sie anti - inflammatorisch wirksam sind, nicht oder nur in geringem Maße die Tyrosinaminotransferase.

Aufgrund ihrer anti-inflammatorischen und zusätzlichen anti-allergischen, immunsuppressiven und anti-proliferativen Wirkung können die erfindungsgemäßen Verbindungen der allgemeinen Formel I als Medikamente zur Behandlung oder Prophylaxe folgender Krankheitszustände bei Säugetieren und Menschen Verwendung finden: Dabei steht der Begriff "ERKRANKUNG" für die folgenden Indikationen:
(i) Lungenerkrankungen, die mit entzündlichen, allergischen und / oder proliferativen Prozessen einhergehen:
   - Chronisch obstruktive Lungenerkrankungen jeglicher Genese, vor allem Asthma bronchiale
   - Bronchitis unterschiedlicher Genese
   - Alle Formen der restriktiven Lungenerkrankungen, vor allem allergische Alveolitis,
   - Alle Formen des Lungenödems, vor allem toxisches Lungenödem
   - Sarkoidosen und Granulomatosen, insbesondere Morbus Boeck
(ii) Rheumatische Erkrankungen / Autoimmunerkrankungen / Gelenkerkrankungen, die mit entzündlichen, allergischen und / oder proliferativen Prozessen einhergehen:
   - Alle Formen rheumatischer Erkrankungen, insbesondere rheumatoide Arthritis, akutes rheumatisches Fieber, Polymyalgia rheumatica
   - Reaktive Arthritis
   - Entzündliche Weichteilerkrankungen sonstiger Genese
   - Arthritische Symtome bei degenerativen Gelenkerkrankungen (Arthrosen)
   - Traumatische Arthritiden
   - Kollagenosen jeglicher Genese, z.B. systemischer Lupus erythematodes, Sklerodermie, Polymyositis, Dermatomyositis- Sjögren-Syndrom, Still-Syndrom, Felty-Syndrom
(iii) Allergien, die mit entzündlichen, und / oder proliferativen Prozessen einhergehen:
   - Alle Formen allergischer Reaktionen, z.B. Quincke Ödem, Heuschnupfen, Insektenstich, allergische Reaktionen auf Arzneimittel, Blutderivate, Kontrastmittel etc., Anaphylaktischer Schock, Urtikaria, Kontakdermatitis
(iv) Gefäßentzündungen (Vaskulitiden)
   - Panarteriitis nodosa, Arteriitis temporalis, Erythema nodosum
(v) Dermatologische Erkrankungen, die mit entzündlichen, allergischen und / oder proliferativen Prozessen einhergehen:
   - Atopische Dermatitis (vor allem bei Kindern)
   - Psoriasis
   - Pityriasis rubra pilaris
   - Erythematöse Erkrankungen, ausgelöst durch unterschiedlichen Noxen, z.B. Strahlen, Chemikalien, Verbrennungen etc.
   - Bullöse Dermatosen
   - Erkrankungen des lichenoiden Formenkreises,
   - Pruritus (z. B. allergischer Genese)
   - Seborrhoisches Ekzem
   - Rosacea
   - Pemphigus vulgaris
   - Erythema exsudativum multiforme
   - Balanitis
   - Vulvitis
   - Haarausfall wie Alopecia areata
   - Cutane T - Zell - Lymphome
(vi) Nierenerkrankungen, die mit entzündlichen, allergischen und / oder proliferativen Prozessen einhergehen:
   - Nephrotisches Syndrom
   - Alle Nephritiden
(vii) Lebererkrankungen, die mit entzündlichen, allergischen und / oder proliferativen Prozessen einhergehen:
   - akuter Leberzellzerfall
   - akute Hepatitis unterschiedlicher Genese, z.B. viral, toxisch, arzneimittelinduziert
   - chronisch aggressive und / oder chronisch intermittierende Hepatitis
(viii) Gastrointestinale Erkrankungen, die mit entzündlichen, allergischen und / oder proliferativen Prozessen einhergehen:
   - regionale Enteritis (Morbus Crohn)
   - Colitis Ulcerosa
   - Gastritis
   - Refluxoesophagitis
   - Gastroenteritiden anderer Genese, z.B. einheimische Sprue
(ix) Proktologische Erkrankungen, die mit entzündlichen, allergischen und / oder proliferativen Prozessen einhergehen:
   - Analekzem
   - Fissuren
   - Hämorrhoiden
   - idiopathische Proktitis
(x) Augenerkrankungen, die mit entzündlichen, allergischen und / oder proliferativen Prozessen einhergehen:
   - allergische Keratitis, Uveitis, Iritis,
   - Konjunktivitis
   - Blepharitis
   - Neuritis nervi optici
   - Chorioditis
   - Ophtalmia sympathica
(xi) Erkrankungen des Hals-Nasen-Ohren-Bereiches, die mit entzündlichen, allergischen und / oder proliferativen Prozessen einhergehen:
   - allergische Rhinitis, Heuschnupfen
   - Otitis externa, z.B. bedingt durch Kontaktexem, Infektion etc.
   - Otitis media
(xii) Neurologische Erkrankungen, die mit entzündlichen, allergischen und / oder proliferativen Prozessen einhergehen:
   - Hirnödem, vor allem Tumor-bedingtes Hirnödem
   - Multiple Sklerose
   - akute Encephalomyelitis
   - Meningitis
   - verschieden Formen von Krampfanfällen, z.B. BNS-Krämpfe
(xiii) Bluterkrankungen, die mit entzündlichen, allergischen und / oder proliferativen Prozessen einhergehen:
   - Erworbene hämolytische Anämie
   - Idopathische Thrombocytopenia
(xiv) Tumorerkrankungen, die mit entzündlichen, allergischen und / oder proliferativen Prozessen einhergehen:
   - Akute lymphatische Leukämie
   - Maligne Lymphome
   - Lymphogranulomatosen
   - Lymphosarkome
   - Ausgedehnte Metastasierungen, vor allem bei Mamma- Bronchial- und Prostatakarzinom
(xv) Endokrine Erkrankungen, die mit entzündlichen, allergischen und / oder proliferativen Prozessen einhergehen:
   - Endokrine Orbitopathie
   - Thyreotoxische Krise
   - Thyreoiditis de Quervain
   - Hashimoto Thyreoiditis
   - Morbus Basedow
(xvi) Organ- und Gewebstransplantationen , Graft-versus-host-disease(xvii) Schwere Schockzustände, z.B anaphylaktischer Schock , systemic inflammatory response syndrome (SIRS)
(xviii) Substitutionstherapie bei:
   - angeborene primäre Nebenniereninsuffizienz, z.B. kongenitales adrenogenitales Syndrom
   - erworbene primäre Nebenniereninsuffizienz, z.B. Morbus Addison, autoimmune Adrenalitits, postinfektiös, Tumoren, Metastasen etc.
   - angeboren sekundäre Nebeniereninsuffizienz, z.B. kongenitaler Hypopitutitarismus
   - erworbene sekundäre Nebenniereninsuffizienz, z.B. postinfektiös, Tumoren etc.
(xix) Emesis, die mit entzündlichen, allergischen und / oder proliferativen Prozessen einhergehen:
   - z.B. in Kombination mit einem 5-HT3-Antagonisten bei Zytostika - bedingten Erbrechen.
(xx) Schmerzen bei entzündlicher Genese, z.B. Lumbago

Darüber hinaus können die erfindungsgemäßen Verbindungen der allgemeinen Formel I zur Therapie und Prophylaxe weiterer oben nicht genannter Krankheitszustände eingesetzt werden, für die heute synthetische Glucocorticoide verwendet werden (siehe dazu Hatz, HJ, Glucocorticoide: Immunologische Grundlagen, Pharmakologie und Therapierichtlinien, Wissenschafliche Verlagsgesellschaft mbH, Stuttgart, 1998).

Alle zuvor genannten Indikationen (i) bis (xx) sind ausführlich beschrieben in Hatz, HJ, Glucocorticoide: Immunologische Grundlagen, Pharmakologie und Therapierichtlinien, Wissenschafliche Verlagsgesellschaft mbH, Stuttgart, 1998.

Für die therapeutische Wirkungen bei den oben genannten Krankheitszuständen ist die geeignete Dosis unterschiedlich und hängt beispielsweise von der Wirkstärke der Verbindung der allgemeinen Formel I, dem Wirt, der Art der Verabreichung und der Art und der Schwere der zu behandelnden Zustände, sowie der Verwendung als Prophylaktikum oder Therapeutikum ab.

Die Erfindung liefert weiterhin
(i) die Verwendung eines der erfindungsgemäßen Verbindung gemäß Formel I oder deren Gemisch zur Herstellung eines Medikaments zur Behandlung von einer ERKRANKUNG;
(ii) ein Verfahren zur Behandlung von einer ERKRANKUNG, welches Verfahren eine Verabreichung einer Verbindungsmenge gemäß der Erfindung umfaßt, wobei die Menge die Krankheit unterdrückt, und wobei die Verbindungsmenge einem Patienten gegeben wird, der ein solches Medikament benötigt;
(iii) eine pharmazeutische Zusammensetzung zur Behandlung von einer ERKRANKUNG, welche Behandlung eines der erfindungsgemäßen Verbindungen oder deren Gemisch und wenigstens einen pharmazeutischen Hilfs- und/oder Trägerstoff umfaßt.

Im allgemeinen sind bei Tieren zufriedenstellende Resultate zu erwarten, wenn die täglichen Dosen einen Bereich von 1 µg bis 100.000 µg der erfindungsgemäßen Verbindung pro kg Körpergewicht umfassen. Bei größeren Säugetieren, beispielsweise dem Menschen, liegt eine empfohlene tägliche Dosis im Bereich von 1 µg bis 100.000 µg pro kg Körpergewicht. Bevorzugt ist eine Dosis von 10 bis 30.000 µg pro kg Körpergewicht, mehr bevorzugt eine Dosis von 10 bis 10.000 µg pro kg Körpergewicht. Zum Beispiel wird diese Dosis zweckmäßigerweise mehrmals täglich verabreicht. Zur Behandlung eines akuten Schocks (z.B. anaphylaktischer Schock) können Einzeldosen gegeben werden, die deutlich über den oben genannten Dosen liegen.

Die Formulierung der pharmazeutischen Präparate auf Basis der neuen Verbindungen erfolgt in an sich bekannter Weise, indem man den Wirkstoff mit den in der Galenik gebräuchlichen Trägersubstanzen, Füllstoffen, Zerfallsbeeinflussern, Bindemitteln, Feuchthaltemitteln, Gleitmitteln, Absorptionsmitteln, Verdünnungsmitteln, Geschmackskorrigentien, Färbemitteln usw., verarbeitet und in die gewünschte Applikationsform überführt. Dabei ist auf Remington's Pharmaceutical Science, 15th ed. Mack Publishing Company, East Pennsylvania (1980) hinzuweisen.

Für die orale Applikation kommen insbesondere Tabletten, Dragees, Kapseln, Pillen, Pulver, Granulate, Pastillen, Suspensionen, Emulsionen oder Lösungen in Frage.

Für die parenterale Applikation sind Injektion- und Infusionszubereitungen möglich.

Für die intraartikulären Injektion können entsprechend zubereitet Kristallsuspensionen verwendet werden.

Für die intramuskuläre Injektion können wässrige und ölige Injektionslösungen oder Suspensionen und entprechende Depotpräparationen Verwendung finden.

Für die rektale Applikation können die neuen Verbindungen in Form von Suppositorien, Kapseln, Lösungen (z.B. in Form von Klysmen) und Salben sowohl zur systemischen, als auch zur lokalen Therapie verwendet werden.

Zur pulmonalen Applikation der neuen Verbindungen können diese in Form von Aerosolen und Inhalaten verwendet werden.

Für die lokale Anwendung an Augen, äußerem Gehörgang, Mittelohr, Nasenhöhle und Nasennebenhöhlen können die neuen Verbindungen als Tropfen, Salben und Tinkturen in entsprechenden pharmazeutischen Zubereitungen verwendet werden.

Für die topische Auftragung sind Formulierungen in Gelen, Salben, Fettsalben, Cremes, Pasten, Puder, Milch und Tinkturen möglich. Die Dosierung der Verbindungen der allgemeinen Formel I sollte in diesen Zubereitungen 0.01% - 20% betragen, um eine ausreichende pharmakologische Wirkung zu erzielen.

Die Erfindung umfaßt ebenfalls die erfindungsgemäßen Verbindungen der allgemeinen Formel I als therapeutischen Wirkstoff. Weiterhin gehört zur Erfindung die erfindungsgemäßen Verbindungen der allgemeinen Formel I als therapeutischen Wirkstoff zusammen mit pharmazeutisch verträglichen und annehmbaren Hilfsstoffen und Trägerstoffen.
Ebenfalls umfaßt die Erfindung eine pharmazeutische Zusammensetzung, die eine der pharmazeutisch aktiven, erfindungsgemäßen Verbindungen oder deren Gemisch oder deren pharamzeutisch verträgliches Salz und ein pharmazeutisch verträgliches Salz oder pharmazeutisch verträgliche Hilfsstoffe und Trägerstoffe enthält.

Die nachstehenden Beispiele dienen der näheren Erläuterung der Erfindung ohne sie darauf beschränken zu wollen. Die Synthesen von wichtigen Vorstufen, die im Rahmen des experimentellen Teils nicht offenbart sind, sind bereits Stand der Technik, und können zum Beispiel aus der WO 98/54159 und WO 02/10143 entnommen werden.

### Experimenteller Teil

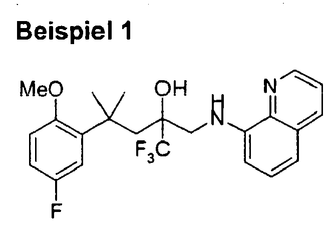

### 1-(Chinolin-8-ylamino)-4-(5-fluor-2-methoxyphenyl)-4-methyl-2-(trifluormethyl)pentan-2-ol

200 mg (0.68 mmol) 2-[2-(5-Fluor-2-methoxyphenyl)-2-methylpropyl)-2-(trifluormethyl)oxiran (WO 00/32584) und 99 mg (0.68 mmol) 8-Aminochinolin werden in 0,2 mL 3,4,5,6-Tetrahydro-2-(1 H)-pyrimidon (DMPU) 20 h auf 130 °C erwärmt. Nach Reinigung des Reaktionsgemischs an Kieselgel mit Hexan-Essigester (0-20 %) werden 230 mg des Produkts erhalten.
'H-NMR (CDCl₃) ; δ = 1.47 (s, 3H), 1.65 (s, 3H), 2.25 (d, 1 H), 2.85 (d, 1 H), 3.30 (AB-System, 2H), 3.85 (s, 3H), 6.13 (br., d, 1 H), 6.80 (dd, 1 H), 6.95 (ddd, 1H), 7.13 (d, 1 H), 7.20 (dd, 1 H), 7.32 (z, 1 H), 7.45 (m, 1 H), 8.18 (m, 1 H), 8.72 (dd, 1 H).

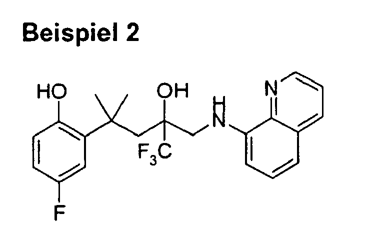

### 1-(Chinolin-8-ylamino)-4-(5-fluor-2-hydroxyphenyl)-4-methyl-2-(trifluormethyl)pentan-2-ol

200 mg (0.46 mmol) 1-(Chinolin-8-ylamino)-4-(5-fluor-2-methoxyphenyl)-4-methyl-2-(trifluormethyl)pentan-2-ol in 20 ml CH₂Cl₂ werden bei 0 °C mit 9 ml 1 M Bortribromid-CH₂Cl₂-Lösung versetzt. Nach 20 h bei Raumtemperatur wird der Ansatz in gesättigte NaHCO₃-Lösung gegossen, 20 Minuten gerührt und mit CH₂Cl₂ extrahiert. Die vereinigten organischen Extrakte werden mit Wasser gewaschen, getrocknet (Na₂SO₄) und im Vakuum eingeengt. Chromatographie mit Hexan-Essigester (0-25 %) an Kieselgel liefert 190 mg des Produkts.
'H-NMR (CDCl₃); δ = 1.48 (s, 3H), 1.62 (s, 3H), 2.20 (d, 1 H), 3.18 (d, 1 H), 3.35 (s, 2H), 6.45 (d, 1 H), 6.52 (dd, 1 H), 6.65 (ddd, 1 H), 7.08 (dd, 1 H), 7.15 (d, 1 H), 7.35 (t, 1 H), 7.50 (dd, 1 H), 8.25 (d, 1 H), 8.83 (dd, 1 H).

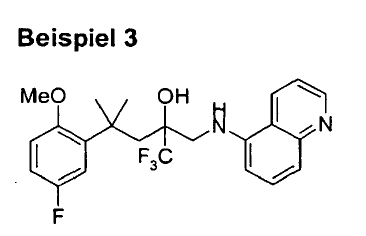

### 1-(Chinolin-5-ylamino)-4-(5-fluor-2-methoxyphenyl-4-methyl-2-(trifluormethyl)pentan-2-ol

Analog zu Beispiel 1 werden 200 mg (0.48 mmol) 2-[2-(5-Fluor-2-methoxyphenyl)-2-methylpropyl]-2-(trifluormethyl)oxiran mit 99 mg (0.68 mmol) 5-Aminochinolin umgesetzt. Nach Chromatographie an Kieselgel mit Hexan-Essigester (0-70 %) werden 58 mg des Produkts erhalten.
'H-NMR (CDCl₃); δ = 1.47 (s, 3H), 1.56 (s, 3H), 2.38 (d, 1 H), 2.78 (d, 1 H), 3.15 (dd, 1 H), 3.33 (dd, 1 H), 3.85 (s, 3H), 4.65 (br., 1H), 6.10 (d, 1 H), 6.80 (dd, 1 H), 6.93 (ddd, 1H), 7.10 (dd, 1H), 7.37 (dd, 1H), 7.50 (t, 1H), 7.61 (d, 1H), 8.18 (d, 1H), 8.80 (dd, 1 H).

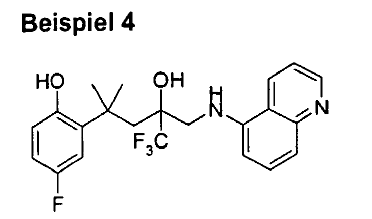

### 1-(Chinolin-5-ylamino)-4-(5-fluor-2-methoxyphenyl)-4-methyl-2-(trifluormethyl)pentan-2-ol

Analog zu Beispiel 2 werden 58 mg (0.13 mmol) 1-(Chinolin-5-ylamino)-4-(5-fluor-2-methoxyphenyl)-4-methyl-2-(trifluormethyl)pentan-2-ol in 6 ml CH₂Cl₂ mit 2.6 ml 1M Bortribromid-CH₂Cl₂-Lösung umgesetzt. Nach Chromatographie an Kieselgel mit Hexan-Essigester (0-70 %) werden 22 mg des Produkts erhalten.
'H-NMR (CDCl₃); δ = 1.48 (s, 3H), 1.54 (s, 3H), 2.45 (d, 1H), 2.82 (d, 1 H), 3.20 (dd, 1H), 3.40 (dd, 1H), 5.05 (br., 1 H), 6.25 (d, 1H), 6.70 (m, 1H), 6.85 (dd, 1H), 6.95 (dd, 1H), 7.45 (dd, 11H), 7.53 (d, 1H), 7.58 (d, 1H), 8.32 (d, 1H), 8.68 (d, 1 H).

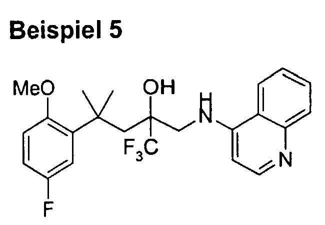

### 1-(Chinolin-4-ylamino)-4-5-fluor-2-methoxyphenyl)-4-methyl-2-(trifluormethyl)pentan-2-ol

### 1-Amino-4-(5-fluor-2-methoxyphenyl)-4-methyl-2-(trifluormethyl)propan-2-ol

1.0 g (3.4 mmol) 2-[2-(5-Fluor-2-methoxyphenyl-2-methylpropyl]-2-(trifluormethyl)oxiran in 68 ml THF werden mit 1.1 g Natriumazid und 180 mg Ammoniumchlorid in 14 ml Wasser und 26 ml Ethanol 6 h unter Rückfluß erhitzt. Der Ansatz wird eingeengt, mit Ether verdünnt, mit Wasser gewaschen, getrocknet (Na₂SO₄) und eingeengt. Chromatographie an Kieselgel mit Hexan-Essigester (0-15 %) liefert 950 mg 1-Azido-4-(5-fluor-2-methoxyphenyl)-4-methyl-2-(trifluormethyl)propan-2-ol. Diese werden in 29 ml THF gelöst und bei 0 °C portionsweise mit 270 mg Lithiumaluminiumhydrid versetzt. Nach 1 h wird der Ansatz mit Essigester und Wasser behandelt und über Celite filtriert. Die Essigesterphase wird getrocknet (Na₂SO₄) und im Vakuum eingeengt. 920 mg Amin werden erhalten.
¹H-NMR (CDCl₃): δ = 1.4 (s, 3H), 1.5 (s, 3H), 2.15 (d, 1 H), 2.45 (d, 1 H), 2.55 (d, 1 H), 2.75 (d, 1 H), 2.80 (m), 3.8 (s, 3H), 6.8 (dd, 1 H), 6.9 (td, 1 H), 7.05 (dd, 1 H)

### 1-(Chinolin-4-ylamino)-4-(5-fluor-2-methoxyphenyl)-4-methyl-2-(trifluormethyl)pentan-2-ol

500 mg (1.6 mmol) 1-Amino-4-(5-fluor-2-methoxyphenyl)-4-methyl-2-(trifluormethyl)propan-2-ol, 265 mg (1.6 mmol) 4-Chlorchinolin und 183 mg (1.6 mmol) Diazabicyclo-[2.2.2]octan werden 3 h auf 150 °C erhitzt. Der Ansatz wird in CH₂Cl₂ und Wasser gelöst. Die wäßrige Phase wird mit CH₂Cl₂ extrahiert, die vereinigten organischen Extrakte mit Wasser gewaschen, getrocknet (Na₂SO₄) und i. Vak. eingeengt. Chromatographie an Kieselgel mit CH₂Cl₂-Methanol (0-10 %) liefert 305 mg Produkt.
¹H-NMR (D₆-DMSO); δ = 1.40 (s, 3H), 1.57 (s, 3H), 2.10 (d, 1H), 2.88 (d, 1H), 3.05 (dd, 1H), 3.15 (dd, 1H), 3.80 (s, 3H), 5.96 (d, 1H), 6.00 (s, 1H), 6.26 (br. t, 1H), 6.98 (d, 1H), 7.02 (td, 1H), 7.10 (dd, 1H), 7.45 (t, 1H), 7.60 (t, 1H), 7.77 (d, 1H), 7.95 (d, 1H), 8.30 (d, 1H).

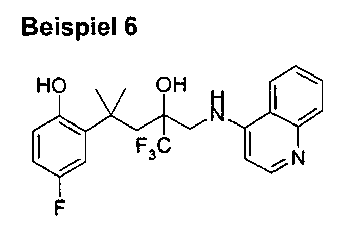

### 1-(Chinolin-4-ylamino)-4-(5-fluor-2-hydroxypheny)-4-methyl-2-(trifluormethyl)pentan-2-ol

Analog zu Beispiel 2 werden 200 mg (0.46 mmol) 1-(Chinotin-4-ylamino)-4-(5-fluor-2-methoxyphenyl)-4-methyl-2-(trifluormethyl)pentan-2-ol und 9 ml 1M Bortribromid-CH₂Cl₂-Lösung umgesetzt. Nach Chromatographie an Kieselgel mit CH₂Cl₂-Methanol (0-15 %) werden 138 mg Produkt erhalten.
'H-NMR (D₆-DMSO); δ = 1.40 (s, 3H), 1.58 (s, 3H), 1.95 (d, 1H), 3.00 - 3.50 (m, 3H), 6.05 (m+d, 2H), 6.65 (br., 1H), 6.80 (dd, 1H), 6.86 (td, 1H), 7.05 (dd, 1H), 7.50 (t, 1H), 7.65 (t, 1H), 7.80 (d, 1H), 8.02 (d, 1H), 8.32 (d, 1H), 9.82 (br., 1H).

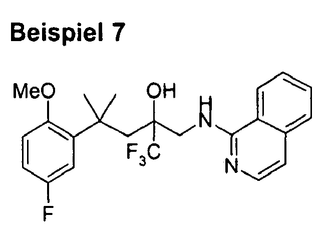

### 4-(5-Fluor-2-methoxyphenyl)-1-(isochinolin-1-ylamino)-4-methyl-2-(trifluormethyl)pentan-2-ol

Analog zu Beispiel 1 werden 200 mg (0.68 mmol) 2-[2-(5-Fluor-2-methoxyphenyl)-2-methylpropyl]-2-(trifluormethyl)oxiran und 99 mg (0.68 mmol) 1-Aminoisochinolin umgesetzt. Nach Chromatographie an Kieselgel mit Hexan-Essigester (0-60%) werden 65 mg Produkt erhalten.
'H-NMR (CDCl₃); δ = 1.45 (s, 3H), 1.72 (s, 3H), 2.40 (d, 1 H), 3.00 (d, 1 H), 3.40 (d, 1 H), 3.88 (s, 3H), 4.23 (d, 1 H), 6.40 (d, 1 H), 6.63 (d, 1 H), 6.82 (dd, 1 H), 6. 89 (td, 1 H), 7.15 (dd, 1H), 7.45 (d, 1 H), 7.55 (t, 1 H), 7.65 (t, 1 H), 8.45 (br., 1 H).

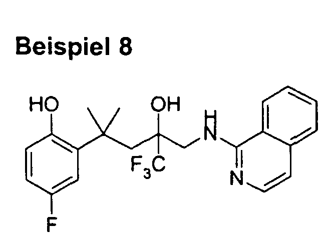

### 4-(5-Fluor-2-hydroxyphenyl)-1-(isochinolin-1-ylamino)-4-methy-2-(trifluormethyl)pentan-2-ol

Analog zu Beispiel 2 werden 65 mg (0.15 mmol) 4-(5-Fluor-2-methoxyphenyl)-1-(isochinolin-1-ylamino)-4-methyl-2-(trifluormethyl)pentan-2-ol mit 2.7 ml 1M Bortribromid-CH₂Cl₂-Lösung umgesetzt. Nach Chromatographie an Kieselgel mit Hexan-Essigester (0-80 %) erhält man 33 mg des Produkts.
'H-NMR (CDCl₃); δ = 1.40 (s, 3H), 1.60 (s, 3H), 2.00 (d, 1 H), 2.87 (d, 1 H), 3.53 (d, 1 H), 4.23 (d, 1 H), 6.25 (d, 1 H), 6.70- 6.90 (m, 3H), 7.00 (dd, 1 H), 7.42 (t, 1 H), 7.48 (d, 1 H), 7.58 (t, 1 H), 8.05 (br., 1 H), 8.22 (d, 1 H), 9.80 (br., 2H).

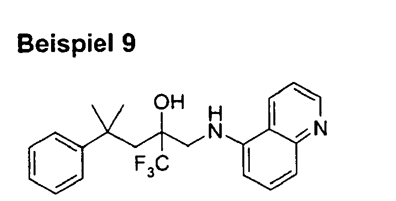

### 1-(Chinolin-5-ylamino)-4-methyl-4-phenyl-2-(trifluormethyl)pentan-2-ol

### 2-(2-Methyl-2-phenylpropyl)-2-(trifluormethyl)oxiran

10.4 g 4-Methyl-2-oxo-4-phenylpentansäure (WO98/54159) in 250 ml Dimethylformamid werden bei -5°C mit 4.1 ml Thionylchlorid und nach 15 min mit 4 ml Methanol versetzt. Nach 15 h bei Raumtemperatur wird der Ansatz mit Wasser verdünnt und mit Essigester extrahiert. Die organischen Extrakte werden mit Wasser gewaschen, getrocknet (Na₂SO₄) und eingeengt, wobei 9.3 g 4-Methyl-2-oxo-4-phenylpentansäure-methylester erhalten werden. Diese werden in 558 ml DMF bei -5°C mit 15.5 ml (104.63 mmol) (Trifluormethyl)trimethylsilan und 20.5 g (63.28 mmol) Caesiumcarbonat versetzt und 16 h bei Raumtemperatur gerührt. Man gibt Wasser hinzu, extrahiert mit Ethylacetat, wäscht die organische Phase mit Wasser und trocknet (Na₂SO₄). Das eingeengte Zwischenprodukt wird in 200 ml THF aufgenommen und 50 ml einer 1 M Lösung von Tetrabutylammoniumfluorid in THF werden zugegeben. Man rührt 2 Stunden, gibt Wasser zu, extrahiert mit Ethylacetat, wäscht die organische Phase mit Wasser und trocknet (Na₂SO₄). Nach Chromatographie an Kieselgel mit Hexan-Essigester (0-30%) werden 8.35 g 2-Hydroxy-4-methyl-4-phenyl-2-(trifluormetyl)pentansäure-methylester erhalten. Der Ester (8.3 g, 28.59 mmol) wird in 180 ml THF gelöst, und über einen Zeitraum von 2,5 Stunden werden 1.52 g (36.20 mmol) Lithiumaluminiumhydrid in kleinen Portionen zugegeben. Nach vollständigem Umsatz werden 5 ml Ethylacetat zugetropft und nach weiteren 10 min werden vorsichtig 10 ml Wasser zugegeben. Man filtriert vom gebildeten Niederschlag ab und wäscht sorgfältig mit Ethylacetat. Nach Chromatographie an Kieselgel mit Hexan-Essigester (0-35%) werden 5.40 g 4-Methyl-4-phenyl-2-(trifluormetyl)pentan-1,2-diol erhalten. Zum Diol (5.40 g, 20.59 mmol) in 43 ml THF werden 5.6 g (21.35 mmol) Triphenylphosphin und unter Eiskühlung 4.3 ml (27.31 mmol) Azodicarbonsäure-diethylester gegeben. Die Reaktionsmischung wird 3 Stunden unter Rückfluß erhitzt und nach dem Abkühlen eingeengt. Nach Chromatographie an Kieselgel mit Hexan-Essigester (0-15%) werden 4.18 g Produkt erhalten.
'H-NMR (CDCl₃); δ = 1.37 (s, 3H), 1.41 (s, 3H), 2.20 (m, 1 H), 2.27 (d, 1 H), 2.55 (d, 1 H), 2.67 (d, 1 H), 7.18-7.35 (m, 5H).

### 1-(Chinolin-5-ylamino)-4-methyl-4-phenyl-2-(trifluormethyl)pentan-2-ol

Analog zu Beispiel 1 werden 300 mg (1.22 mmol) 2-(2-Methyl-2-phenylpropyl)-2-(trifluormethyl)oxiran und 882 mg (6.12 mmol) 5-Aminochinolin umgesetzt. Nach Chromatographie an Kieselgel mit Hexan-Essigester (0-75%) werden 85 mg Produkt erhalten.
'H-NMR (CDCl₃); δ = 1.45 (s, 3H), 1.60 (s, 3H), 2.30 (d, 1 H), 2.36 (d, 1 H), 3.02 (dd, 1 H), 3.05 (s, 1 H), 3.25 (dd, 1H), 4.24 (dd, 1 H), 6.11 (d, 1H), 7.28-7.56 (m, 8H), 8.04 (d, 1 H), 8.86 (dd, 1 H).

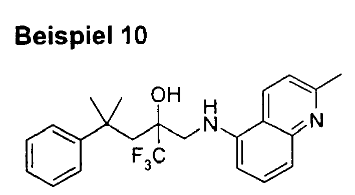

### 4-Methyl-1-(2-methylchinolin-5-ylamino)-4-phenyl-2-(trifluormethyl)pentan-2-ol

Analog zu Beispiel 1 werden 500 mg (2.05 mmol) 2-(2-Methyl-2-phenylpropyl)-2-(trifluormethyl)oxiran und 650 mg (4.10 mmol) 5-Amino-2-methylchinolin umgesetzt. Nach Chromatographie an Kieselgel mit Hexan-Essigester (0-70%) werden 485 mg Produkt erhalten.
¹H-NMR (CDCl₃); δ = 1.44 (s, 3H), 1.59 (s, 3H), 2.30 (d, 1 H), 2.35 (d, 1 H), 2.72 (s, 3H), 3.01 (dd, 1 H), 3.04 (s, 1 H), 3.23 (dd, 1 H), 4.18 (dd, 1 H), 6.04 (d, 1H), 7.21 (d, 1 H), 7.30 (dt, 1 H), 7.37-7.51 (m, 6H), 7.92 (d, 1 H).

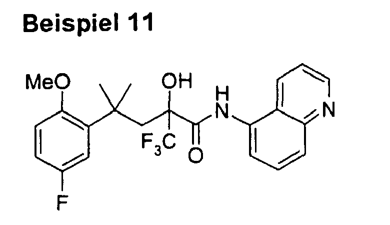

### N-(Chinolin-5-yl)-4-(5-fluor-2-methoxyphenyl)-2-hydroxy-4-methyl-2-(trifluormethyl)pentansäureamid

540 mg (2.13 mmol) 4-(5-Fluor-2-methoxyphenyl)-4-methyl-2-oxopentansäure (WO 00/32584) in 15 ml DMF werden bei -5°C unter Argon mit 0.18 ml Thionylchlorid versetzt. Nach 20 min Rühren bei -3°C bis +3°C werden 470 mg (3.26 mmol) 5-Aminochinolin zugegeben. Man lässt auf Raumtemperatur erwärmen, rührt weitere 16 Stunden, versetzt mit 10%iger Zitronensäure, extrahiert mit Ethylacetat, wäscht die organische Phase mit Wasser und trocknet (Na₂SO₄). Nach Chromatographie an Kieselgel mit Hexan-Essigester (0-75%) werden 680 mg N-(Chinolin-4-yl)-4-(5-fluor-2-methoxyphenyl)-4-methyl-2-oxopentansäureamid erhalten, die in 22 ml DMF gelöst und auf 0°C gekühlt werden. Die Lösung wird mit 1.80 ml (Trifluormethyl)trimethylsilan und 2.43 g Cäsiumcarbonat versetzt und 16 h bei Raumtemperatur gerührt. Man gibt Wasser hinzu, extrahiert mit Ethylacetat, wäscht die organische Phase mit Wasser und trocknet über Natriumsulfat. Das eingeengte Zwischenprodukt wird in 10 ml THF aufgenommen und 5.5 ml einer 1 M Lösung von Tetrabutylammoniumfluorid werden zugegeben. Man rührt 1.5 Stunden, gibt Wasser zu, extrahiert mit Ethylacetat, wäscht die organische Phase mit Wasser und trocknet über Natriumsulfat. Nach Chromatographie an Kieselgel mit Hexan-Essigester (0-70%) werden 420 mg Produkt erhalten.
'H-NMR (CDCl₃); δ = 1.44 (s, 3H), 1.46 (s, 3H), 2.80 (d, 1 H), 3.10 (d, 1 H), 3.57 (s, 1 H), 3.89 (s, 3H), 6.81 (dd, 1 H), 6.89 (m, 1 H), 6.99 (dd, 1H), 7.45 (dd, 1 H). 7.73 (t, 1 H), 7.95 (d, 1 H), 7.99 (d, 1 H), 8.00 (d, 1 H), 8.83 (br., 1H), 8.95 (dd, 1 H).

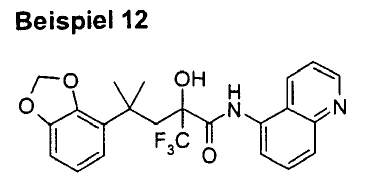

### 4-(1,3-Benzodioxol-4-yl)-N-(chinolin-5-yl)-2-hydroxy-4-methyl-2-(trifluormethyl)pentanamid

### 1,3-Benzodioxol-4-carbonsäure-methylester:

50 g 2,3-Dihydroxybenzoesäure in 450 ml Methanol werden bei Raumtemp. tropfenweise mit 50 ml Thionylchlorid versetzt. Anschließend wird die Lösung für 5h auf 60°C erhitzt und noch über Nacht bei Raumtemp. gerührt. Das Lösungsmittel wird vollständig im Vakuum entfernt und das verbleibende Öl in Diethylether aufgenommen und mit ges. Bicarbonatlösung extrahiert. Nach Waschen mit Sole, Trocknen mit Natriumsulfat und Entfernen des Lösungsmittels im Vakuum erhält man 46 g 2,3-Dihydroxybenzoesäuremethylester. Dieser wird in 575 ml DMF und 20.2 ml Dibrommethan mit 56.7 g Kaliumcarbonat versetzt und er wird 5 h unter Argon auf 100°C erwärmt. Anschließend wird noch über Nacht bei Raumtemp. gerührt. Es wird dann mit Wasser versetzt und dreimal mit Essigester extrahiert. Die organische Phase wird mehrfach mit Wasser gewaschen und über Natriumsulfat getrocknet. Das Lösungsmittel wird im Vakuum entfernt und man erhält 50.2 g 1,3-Benzodioxol-4-carbonsäure-methylester als braunen Feststoff. Fp.: 55-57°C

### 4-(1,3-Benzodioxot-4-yl)-4-methyl-2-oxopentansäure

4.76 g 1,3-Benzodioxol-4-carbonsäure-methylester in 65 ml trockenen THF werden bei Raumtemp. zu einer Lösung von 21 ml 3 M Methylmagnesiumchlorid in THF unter Argon zugetropft. Es wird 3h gerührt und dann langsam mit 1 N Salzsäure versetzt. Nach Extraktion mit Essigester und Waschen der organischen Phase mit Wasser wird mit Natriumsulfat getrocknet und das Lösungsmittel im Vakuum entfernt. Man erhält 5.0 g 1-(1,3-Benzodioxol-4-yl)-1-methylethanol als braunes Öl. Davon werden 3.6 g und 5.4 g 2-(Trimethylsilyloxy)-acrylsäureethylester in 80 ml Dichlormethan bei -70°C mit 18 ml Zinntetrachlorid versetzt. nach 15 min. rühren bei -70°C wird die Lösung auf halbgesättigte Natriumcarbonatlösung gegossen, mit Essigester versetzt und stark gerührt. Die Phasen werden getrennt und die Wasserphase zweimal mit Essigester extrahiert. Die organische Phase wird mit Sole gewaschen, mit Natriumsulfat getrocknet und das Lösungsmittel im Vakuum entfernt. Man erhält ein gelbes Öl, welches mit 60 ml 1 N Natronlauge und 120 ml Methanol versetzt wird und 3h bei Raumtemp. gerührt wird. Das Methanol wird im Vakuum entfernt und die Wasserphase mit Diethylether extrahiert. Dann wird die Wasserphase durch Zugabe von 120 ml 1N Salzsäure angesäuert und 3 Mal mit Diethylether extrahiert. Die Etherphase wird getrocknet und das Lösungsmittel im Vakuum entfernt. Man erhält 4.2 g 4-(1,3-Benzodioxol-4-yl)-4-methyl-2-oxopentansäure als gelbes Öl. MS (EI): M⁺= 250 (M=250)

### 4-(1,3-Benzodioxol-4-yl)-N-(chinolin-5-yl)-4-methyl-2-oxopentanamid

100 mg 4-(1,3-Benzodioxol-4-yl)-4-methyl-2-oxopentansäure in 1 ml Dimethylacetamid werden bei 0°C mit 0.034 ml Thionylchlorid versetzt und 20 min. gerührt. Dann werden 61 mg 5-Aminochinolin zugegeben und es wird über Nacht bei Raumtemp. gerührt. Es wird auf Natriumhydrogencarbonatlösung gegeben und 3 Mal mit Essigester extrahiert. Es wird mit Sole gewaschen, getrocknet und das Lösungsmittel im Vakuum entfernt. Das verbleibende Öl wird durch Dickschichtchromatographie (Kieselgel, Aceton / Hexan 1:1) getrennt. Man erhält 25 mg 4-(1,3-Benzodioxol-4-yl)-N-(chinolin-5-yl)-4-methyl-2-oxopentanamid als gelben Schaum. MS (El): M⁺ = 376.3 (M=376.4)

### 4-(1,3-Benzodioxol-4-yl)-N-(chinolin-5-yl)-2-hydroxy-4-methyl-2-(trifluormethyl)pentanamid

22 mg 4-(1,3-Benzodioxol-4-yl)-N-(chinolin-5-yl)-4-methyl-2-oxopentanamid und 0.04 ml Trifluormethyltrimetyhlsilan in 1 ml DMF werden bei 0°C mit 11 mg Cäsiumcarbonat versetzt. Nach 2h wird eine Spatelspitze Tetrabutylammoniumfluorid zugegeben und nach weiteren 20 min. wird die Reaktion auf Wasser gegeben. Es wird 3 Mal mit Essigester extrahiert, mit Wasser und Sole gewaschen, getrocknet und das Lösungsmittel im Vakuum entfernt. Das verbleibende Öl wird durch Chromatographie an Kieselgel getrennt. Man erhält 11 mg 4-(1,3-Benzodioxol-4-yl)-N-(chinolin-5-yl)-2-hydroxy-4-methyl-2-(trifluormethyl)pentanamid als Feststoff. Fp.: 164-167°C

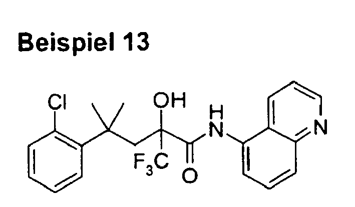

### N-(Chinolin-5-yl)-4-(2-chlorphenyl)-2-hydroxy-4-methyl-2-(trifluormethyl)pentanamid

### N-(Chinolin-5-yl)-4-(2-chlorphenyl)-4-methyl- 2-oxopentanamid

Aus 200 mg 4-(2-Chlorphenyl)-4-methyl-2-oxopentansäure (WO00/32584) werden nach der Vorschrift für 4-(1,3-Benzodioxol-4-yl)-N-(chinolin-5-yl)-4-methyl-2-oxopentanamid nach Chromatographie an Kieselgel 152 mg N-(Chinolin-5-yl)-4-(2-chlorphenyl)-4-methyl-2-oxopentanamid erhalten. MS (El):
M⁺ = 366, 368 (3:1); (M=366.8)

### N-(Chinolin-5-yl)-4-(2-chlorphenyl)-2-hydroxy-4-methyl-2-(trifluormethyl)pentanamid

Aus 142 mg N-(Chinolin-5-yl)-4-(2-chlorphenyl)-4-methyl-2-oxopentanamid werden nach der Vorschrift für 4-(1,3-Benzodioxol-4-yl)-N-(chinolin-5-yl)-2-hydroxy-4-methyl-2-(trifluormethyl)pentanamid (Beispiel 12) nach Chromatographie an Kieselgel 82 mg N-(Chinolin-5-yl)-4-(2-chlorphenyl)-2-hydroxy-4-methyl-2-(trifluormethyl)pentanamid erhalten. Fp.: 210-214°C

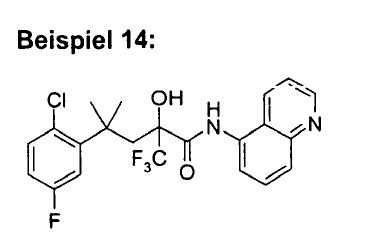

### N-(Chinolin-5-yl)-4-(2-chlor-5-fluorphenyl)-2-hydroxy-4-methyl-2-(trifluormethyl)pentanamid

### N-(Chinolin-5-yl)-4-(2-chlor-5-fluorphenyl)-4-methyl-2-oxopentanamid

Aus 520 mg 4-(2-Chlor-5-fluorphenyl)-4-methyl-2-oxopentansäure (WO 02/10143) werden nach der Vorschrift für 4-(1,3-Benzodioxol-4-yl)-N-(chinolin-5-yl)-4-methyl-2-oxopentanamid (Beispiel 12) nach Chromatographie an Kieselgel 400 mg N-(Chinolin-5-yl)-4-(2-chlor-5-fluorphenyl)-4-methyl-2-oxopentanamid erhalten. Fp.:145-146°C

### N-(Chinolin-5-yl)-4-(2-chlor-5-fluorphenyl)-2-hydroxy-4-methyl-2-(trifluormethyl)pentanamid

Aus 384 mg N-(Chinolin-5-yl)-4-(2-chlor-5-fluorphenyl)-4-methyl-2-oxopentanamid werden nach der Vorschrift für 4-(1,3-Benzodioxol-4-yl)-N-(chinolin-5-yl)-2-hydroxy-4-methyl-2-(trifluormethyl)pentanamid (Beispiel 12) nach Chromatographie an Kieselgel 60 mg N-(Chinolin-5-yl)-4-(2-chlor-5-fluorphenyl)-2-hydroxy-4-methyl-2-(trifluormethyl)pentanamid erhalten. Fp.188-189°C

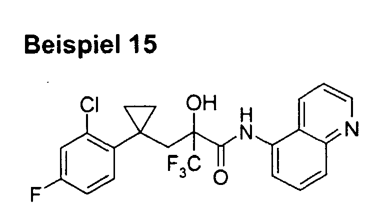

### N-(Chinolin-5-yl)-3-[1-2-chlor-4-fluorphenyl)-cyclopropyl]-2-hydroxy-2-(trifluormethyl)propanamid

### N-(Chinolin-5-yl)-3-[1-(2-chlor-4-fluorphenyl)-cyclopropyl]-2-oxopropanamid

Aus 512 mg 3-[1-(2-Chlor-4-fluorphenyl)-cyclopropyl]-2-oxopropionsäure (WO 02/10143) werden nach der Vorschrift für 4-(1,3-Benzodioxol-4-yl)-N-(chinolin-5-yl)-2-hydroxy-4-methyl-2-(trifluormethyl)pentanamid (Beispiel 12) nach Chromatographie an Kieselgel 535 mg N-(Chinolin-5-yl)-3-[1-(2-chlor-4-fluorphenyl)-cyclopropyl]-2-oxopropanamid als Schaum erhalten.

### N-(Chinolin-5-yl)-3-[1-(2-chlor-4-fluorphenyl)-cyclopropyl]-2-hydroxy-2-(trifluormethyl)propanamid

Aus 535 mg N-(Chinolin-5-yl)-3-[1-(2-chlor-4-fluorphenyl)-cyclopropyl]-2-oxopropanamid werden nach der Vorschrift für 4-(1,3-Benzodioxol-4-yl)-N-(chinolin-5-yl)-2-hydroxy-4-methyl-2-(trifluormethyl)pentanamid (Beispiel 12) nach Chromatographie an Kieselgel 200 mg N-(Chinolin-5-yl)-3-[1(-2-chlor-4-fluorphenyl)-cyclopropyl]-2-hydroxy-2-(trifluormethyl)pentanamid erhalten. Fp.: 220-221°C

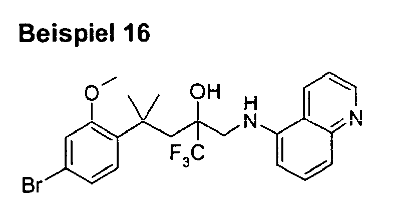

### 4-(4-Brom-2-methoxyphenyl)-1 -(chinolin-5-vlamino)-2-hydroxy-4-methyl-2-(trifluormethyl)pentan-2-ol

Analog zu Beispiel 1 werden 200 mg 2-[2-(4-Brom-2-methoxyphenyl)-2-methylpropyl]-2-(trifluormethyl)oxiran (WO 00/32585) und 5-Aminochinolin umgesetzt. Nach Chromatographie an Kieselgel mit Hexan-Essigester (1+1) werden 43 mg 4-(4-Brom-2-methoxyphenyl)-1-(chinolin-5-ylamino)-2-hydroxy-4-methyl-2-(trifluormethyl)pentan-2-ol erhalten. Fp.: 181°C

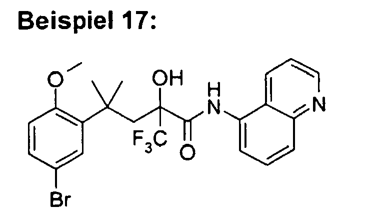

### 4-(5-Brom-2-methoxyphenyl)-N-(chinolin-5-yl)-2-hydroxy-4-methyl-2-(trifluormethyl)pentanamid

### 1-(5-Brom-2-methoxyphenyl)-1-methylethanol

62 ml einer 3 M Methylmagnesiumbromidlösung in Tetrahydrofuran werden in einer Stunde bei 0°C in 18,6 g 5-Brom-2-methoxybenzoesäuremethylester und 180 ml Diethylether eingetropft. Nach 16 Stunden bei Raumtemperatur wird unter Eiskühlung mit gesättigter Ammoniumchloridlösung und Ethylacetat versetzt. Die Ethylacetatphase wird mit Wasser gewaschen, getrocknet (Na₂SO₄) und eingedampft. Man erhält nach Kugelrohrdestillation (Kp.: 140°C/0,04hPa) 16,7 g kristallines 1-(5-Brom-2-methoxyphenyl)-1-methylethanol. Fp.: 66-68°C.

### 4-(5-Brom-2-methoxyphenyl)-4-methyl-2-oxopentansäure-ethylester

9.8 g 1-(5-Brom-2-methoxyphenyl)-1-methylethanol und 15,24 g 2-(Trimethylsilyloxy)-acrylsäureethylester in 150 ml Dichlormethan werden bei - 70°C mit 5,6 ml Zinntetrachlorid versetzt. Nach 20 Minuten bei -70°C wird die Lösung auf eine halbgesättigte Kaliumcarbonatlösung gegossen und mit Dichlormethan versetzt. Die Dichlormethanphase wird mit Kaliumcarbonatlösung, 1 M Salzsäure und Wasser gewaschen, getrocknet (Na₂SO₄) und eingeengt. Nach Kugelrohrdestillation werden 5,2 g 4-(5-Brom-2-methoxyphenyl)-4-methyl-2-oxopentansäure-ethylester erhalten. Kp.: 160°C/0,04 hPa

### 4-(5-Brom-2-methoxyphenyl)-4-methyl-2-oxopentansäure

4-(5-Brom-2-methoxyphenyl)-4-methyl-2-oxopentansäure-ethylester in 30 ml Methanol und 12 ml 1 M Natronlauge werden 1 Stunde bei Raumtemperatur gerührt und das Methanol abdestilliert. Es wird mit Wasser und Hexan versetzt, die Wasserphase unter Eiskühlung mit verdünnter Salzsäure angesäuert und mit Ethylacetat versetzt. Die Ethylacetatphase wird mit Wasser gewaschen, getrocknet (Na₂SO₄) und eingeengt. Nach Kristallisation aus Hexan werden 1,8 g 4-(5-Brom-2-methoxyphenyl)-4-methyl-2-oxopentansäure erhalten. Fp.: 80°C

### 4-(5-Brom-2-methoxyphenyl)-N-(chinolin-5-yl)-4-methyl-2-oxopentanamid

Aus 630 mg 4-(5-Brom-2-methoxyphenyl)-4-methyl-2-oxopentansäure werden nach der Vorschrift für 4-(1,3-Benzodioxol-4-yl)-N-(chinolin-5-yl)-4-methyl-2-oxopentanamid (Beispiel 12) nach Chromatographie an Kieselgel 730 mg 4-(5-Brom-2-methoxyphenyl)-N-(chinolin-5-yl)-4-methyl-2-oxopentanamid erhalten. Fp.: 133-135°C

### 4-(5-Brom-2-methoxyphenyl)-N-(chinolin-5-yl)-2-hydroxy-4-methyl-2-(trifluormethyl)pentanamid

Aus 617 mg 4-(5-Brom-2-methoxyphenyl)-N-(chinolin-5-yl)-4-methyl-2-oxopentanamid werden nach der Vorschrift für 4-(1,3-Benzodioxol-4-yl)-N-(chinolin-5-yl)-2-hydroxy-4-methyl-2-(trifluormethyl)pentanamid (Beispiel 12) nach Chromatographie an Kieselgel 484 mg 4-(5-Brom-2-methoxyphenyl)-N-(chinolin-5-yl)-2-hydroxy-4-methyl-2-(trifluormethyl)pentanamid erhalten. Fp.: 243-245°C

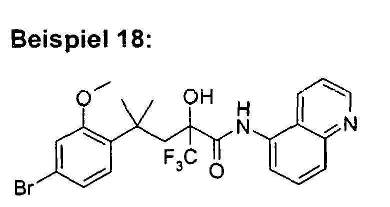

### 4-(4-Brom-2-methoxyphenyl)-N-(chinolin-5-yl)-2-hydroxy-4-methyt-2-(trifluormethyl)pentanamid

### 4-(4-Brom-2-methoxyphenyl)-N-(chinolin-5-yl)-4-methyl-2-oxopentanamid

Aus 630 mg 4-(4-Brom-2-methoxyphenyl)-4-methyl- 2-oxopentansäure (WO 98/54159) werden nach der Vorschrift für 4-(1,3-Benzodioxol-4-yl)-N-(chinolin-5-yl)-4-methyl- 2-oxopentanamid (Beispiel 12) nach Chromatographie an Kieselgel 363 mg 4-(4-Brom-2-methoxyphenyl)-N-(chinolin-5-yl)-4-methyl-2-oxopentanamid erhalten. Fp.: 114-115°C

### 4-(4-Brom-2-methoxyphenyl)-N-(chinolin-5-yl)-2-hydroxy-4-methyl-2-(trifluormethyl)pentanamid

Aus 528 mg 4-(4-Brom-2-methoxyphenyl)-N-(chinolin-5-yl)-4-methyl-2-oxopentanamid werden nach der Vorschrift für 4-(1,3-Benzodioxol-4-yl)-N-(chinolin-5-yl)-2-hydroxy-4-methyl-2-(trifluormethyl)pentanamid (Beispiel 12) nach Chromatographie an Kieselgel 280 mg 4-(4-Brom-2-methoxyphenyl)-N-(chinolin-5-yl)-2-hydroxy-4-methyl-2-(trifluormethyl)pentanamid erhalten. Fp.: 208-209°C

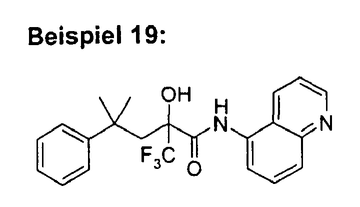

### N-(Chinolin-5-yl)-)-2-hydroxy-4-methyl-4-phenyl-2-(trifluormethyl)pentanamid

### N-(Chinolin-5-yl)-)-4-methyl-2-oxo-4-phenylpentanamid

Aus 515 mg 4-Methyl-2-oxo-4-phenylpentansäure (WO98/54159) werden nach der Vorschrift für 4-(1,3-Benzodioxol-4-yl)-N-(chinolin-5-yl)-4-methyl-2-oxopentanamid (Beispiel 12) nach Chromatographie an Kieselgel 370 mg N-(Chinolin-5-yl)-)-4-methyl-2-oxo-4-phenylentanamid erhalten. Fp.:98-99°C

### N-(Chinolin-5-yl)-)-2-hydroxy-4-methyl-4-phenyl-2-(trifluormethyl)pentanamid

Aus 200 mg N-(Chinolin-5-yl)-4-methyl-2-oxo-4-phenylpentanamid werden nach der Vorschrift für 4-(1,3-Benzodioxol-4-yl)-N-(chinolin-5-yl)-2-hydroxy-4-methyl-2-(trifluormethyl)pentanamid (Beispiel 12) nach Chromatographie an Kieselgel 85 mg N-(Chinolin-5-yl)-2-hydroxy-4-methyl-4-phenyl-2-(trifluormethyl)pentanamid erhalten. Fp.: 181-182°C

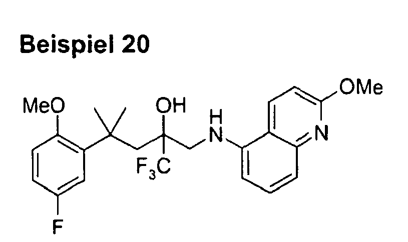

### 4-(5-Fluor-2-methoxvphenyl)-1-(2-methoxychinolin-5-ylamino)-4-methyl-2-(trifluormethyl)pentan-2-ol

### 5-Nitrochinolin-1-oxid

Eine Lösung von 25.5 g (146 mmol) 5-Nitrochinolin in 544 mL Essigsäure und 272 mL 30 proz. wässrige H₂O₂-Lösung werden 100 min auf 62-69 °C erhitzt. Das Reaktionsgemisch wird auf ges. NaCl-Lösung gegossen und mit Essigester extrahiert. Die vereinigten Extrakte werden unter Toluol-Zusatz auf ca. 50 mL eingeengt. Säulenchromatographie an Kieselgel mit Essigester-MeOH liefert 12.3 g des Produkts als gelben Feststoff.
'H-NMR (CDCl₃): δ = 7.5 (dd, 1 H), 7.85 (t, 1 H), 8.45 (d, 1 H), 8.5 (d, 1 H), 8.6 (d, 1 H), 9.15 (d, 1 H).
*2-Methoxy-5-nitrochinolin*

Eine Suspension von 1 g (5 mmol) 5-Nitrochinolin-1-oxid, 1.23 g (6.4 mmol) Toluolsulfonsäurechlorid und 1.4 mL (9.9 mmol) Triethylamin in 30 mL MeOH wird 20 h bei Raumtemp. gerührt. Der Feststoff wird abgesaugt und mit MeOH gewaschen: 565 mg hellgelbes Produkt.
'H-NMR (CDCl₃): δ = 4.1 (s, 3H), 7.15 (d, 1 H), 7.7 (t, 1 H), 8.15 (d, 2H), 8.8 (d, 1 H).
*5-Amino-2-methoxychinolin*

550 mg (2.7 mmol) 2-Methoxy-5-nitrochinolin werden in 15 mL Essigester in Gegenwart von 138 mg 10 proz. Pd-C 5 h in einer Wasserstoffatmosphäre gerührt. Der Ansatz wird filtriert und das Filtrat eingeengt: 520 mg eines hellgelben Öls.
¹H-NMR (CDCl₃): δ = 4.05 (s, 3H), 6.65 (d, 1 H), 6.85 (d, 1 H), 7.3 (d, 1 H), 7.4 (t, 1 H), 8.0 (d, 1 H).
*4-(5-Fluor-2-methoxyphenyl)-2-hydroxy-4-methyl-2-(trifluormethyl)pentanal*

1.5 g (4.8 mmol) 4-(5-Fluor-2-methoxyphenyl)-4-methyl-2-trifluormethylpentan-1,2-diol und 3.4 mL (24.4 mmol) Triethylamin in 17 mL DMSO und 53 mL CH₂Cl₂ werden bei 10 °C portionsweise mit 3 g (18.9 mmol) Pyridin-Schwefeltrioxid-Komplex versetzt. Nach 3 h bei 12-18 °C wird unter Eiskühlung mit ges. NH₄Cl-Lösung hydrolysiert und mit Essigester extrahiert. Die vereinigten Extrakte werden getrocknet (Na₂SO₄) und eingeengt: 1.57 g des Produkts als hellgelbes Öl.
'H-NMR (CDCl₃): δ = 1.4 (s, 3H), 1.5 (s, 3H), 2.25 (d, 1 H), 3.4 (d, 1 H), 3.6 (br., 1 H), 3.85 (s, 3H), 6.8 (dd, 1 H), 6.85-7.0 (m, 2H), 9.05 (s, 1 H).
*4-(5-Fluor-2-methoxyphenyl)-1-(2-methoxychinolin-5-ylamino)- 4-methyl-2-(trifluormethyl)pentan-2-ol*

300 mg (0.97 mmol) 4-(5-Fluor-2-methoxyphenyl)-2-hydroxy-4-methyl-2-(trifluoromethyl)pentanal und 202 mg (1.16 mmol) 5-Amino-2-methoxychinolin in 10 mL Essigsäure werden 5 h unter Rückfluss erhitzt. Man lässt auf Raumtemp. abkühlen, gibt 640 mg (3 mmol) Natriumtriacetoxyborhydrid dazu und rührt 15 h bei Raumtemp. Nach Zugabe von Toluol wird er Ansatz eingeengt, der Rückstand in Essigester aufgenommen, mit ges. NaHCO₃-Lsg. gewaschen, getrocknet und eingeengt. Säulenchromatographie an Kieselgel mit Hexan-Essigester liefert 146 mg des Produkts als farbloses Öl.
¹H-NMR (CDCl₃): δ = 1.45 (s, 3H), 1.55 (s, 3H), 2.3 (d, 1 H), 2.8 (d, 1 H), 3.1 (d, 1 H), 3.2 (s, 1 H), 3.3 (d, 1 H), 3.85 (s, 3H), 4.05 (s, 3H), 5.95 (m, 1 H), 6.8 (m, 2H), 6.9 (td, 1 H), 7.1 (dd, 1 H), 7.35 (m, 2H), 7.85 (d, 1 H).
MS (ES): m/e = 467.

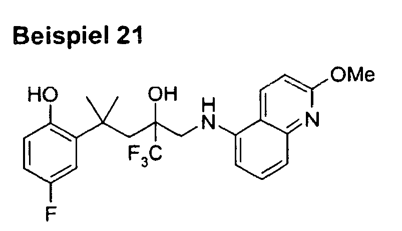

### 4-(5-Fluor-2-hydroxyphenyl)-1-(2-methoxychinolin-5-ylamino)- 4-methyl-2-(trifluormethyl)pentan-2-ol

100 mg (0.21 mmol) 4-(5-Fluor-2-methoxyphenyl)-1-(2-methoxychinolin-5-ylamino)- 4-methyl-2-(trifluormethyl)pentan-2-ol in 9 ml CH₂Cl₂ werden bei Raumtemp. mit 4 ml 1M Bortribromid-CH₂Cl₂-Lösung versetzt. Nach 15 h bei Raumtemp. wird der Ansatz in gesättigte NaHCO₃-Lösung gegossen, 10 Minuten gerührt und mit Essigester extrahiert. Die vereinigten organischen Extrakte werden getrocknet (Na₂SO₄) und im Vakuum eingeengt. Chromatographie an Kieselgel mit Hexan-Essigester liefert 73 mg des Produkts.
¹H-NMR (CDCl₃): δ = 1.5 (s, 3H), 1.6 (s, 3H), 2.35 (d, 1 H), 2.8 (d, 1 H), 3.2 (d, 1 H), 3.3 (br., 1 H), 3.4 (d, 1 H), 4.05 (s, 3H), 6.0 (m, 1 H), 6.7 (dd, 1 H), 6.8 (td, 1 H), 6.85 (d, 1 H), 7.1 (dd, 1 H), 7.35 (m, 2H), 7.95 (d, 1 H).
MS (ES): m/e = 453.

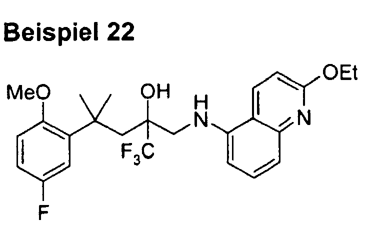

### 1-(2-Ethoxychinolin-5-ylamino)-4-(5-fluor-2-methoxyphenyl)-4-methyl-2-(trifluormethyl)pentan-2-ol

### 2-Ethoxy-5-nitrochinolin

Eine Suspension von 1 g (5 mmol) 5-Nitrochinolin-1-oxid, 1.23 g (6.4 mmol) Toluolsulfonsäurechlorid und 1.4 mL (9.9 mmol) Triethylamin in 30 mL EtOH wird 60 h bei Raumtemp. gerührt. Der Feststoff wird abgesaugt und mit EtOH gewaschen: 870 mg Produkt.
'H-NMR (CDCl₃): δ = 1.45 (t, 3H), 4.55 (q, 2H), 7.1 (d, 1 H), 7.65 (t, 1 H), 8.1 (d, 2H), 8.8 (d, 1 H).
*5-Amino-2-ethoxychinolin*

860 mg (3.9 mmol) 2-Ethoxy-5-nitrochinolin werden in 25 mL Essigester in Gegenwart von 235 mg 10 proz. Pd-C 4.5 h in einer Wasserstoffatmosphäre gerührt. Der Ansatz wird filtriert und das Filtrat eingeengt: 720 mg eines hellgelben Öls.
¹H-NMR (CDCl₃): δ = 1.45 (t, 3H), 4.5 (q, 2H), 6.65 (d, 1 H), 6.85 (d, 1 H), 7.3 (d, 1 H), 7.4 (t, 1H), 8.0 (d, 1 H).
*1-(2-Ethoxychinolin-5-ylamino)-4-(5-fluor-2-methoxyphenyl)-4-methyl-2-(trifluormethyl)pentan-2-ol*

Analog zu Beispiel 20 werden 500 mg (1.6 mmol) 4-(5-Fluor-2-methoxyphenyl)-2-hydroxy-4-methyl-2-(trifluoromethyl)pentanal und 363 mg (1.9 mmol) 5-Amino-2-ethoxychinolin zu 338 mg des Produkts umgesetzt.
¹H-NMR (CDCl₃): δ = 1.4 (t, 3H), 1.45 (s, 3H), 1.65 (s, 3H), 2.3 (d, 1 H), 2.8 (d, 1 H), 3.1 (d, 1 H), 3.2 (s, 1 H), 3.3 (d, 1 H), 3.85 (s, 3H), 4.55 (q, 2H), 5.95 (m, 1H), 6.8 (m, 2H), 6.95 (td, 1 H), 7.1 (dd, 1 H), 7.35 (m, 2H), 7.85 (d, 1 H).
MS (ES): m/e = 481.

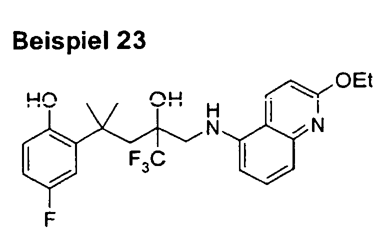

### 1-(2-Ethoxychinolin-5-ylamino)-4-(5-fluor-2-hydroxyphenyl)-4-methyl-2-(trifluormethyl)pentan-2-ol

Analog zu Beispiel 21 werden 200 mg (0.42 mmol) 1-(2-Ethoxychinolin-5-ylamino)-4-(5-fluor-2-methoxyphenyl)-4-methyl-2-(trifluormethyl)pentan-2-ol in 172 mg Produkt umgewandelt.
'H-NMR (CDCl₃): δ = 1.45 (t, 3H), 1.5 (s, 3H), 1.6 (s, 3H), 2.35 (d, 1 H), 2.8 (d, 1 H), 3.2 (d, 1 H), 3.3 (br., 1H), 3.4 (d, 1 H), 4.5 (q, 2H), 6.0 (m, 1 H), 6.7 (dd, 1H), 6.8 (m, 3H), 7.1 (dd, 1 H), 7.35 (m, 2H), 7.95 (d, 1 H).
MS (ES): m/e = 467.

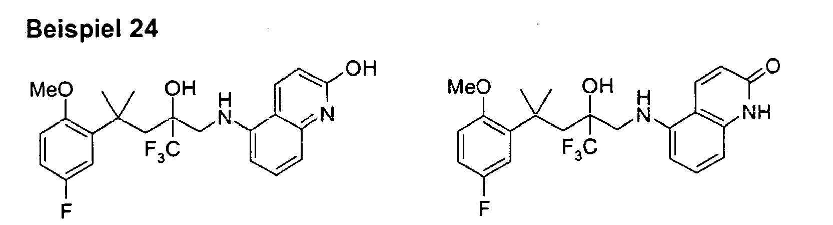

### 4-(5-Fluor-2-methoxyphenyl)-1-(2-hydroxychinolin-5-ylmino)-4-methyl-2-(trifluormethyl)pentan-2-ol/

### 4-(5-Fluor-2-methoxyphenyl)-1 -(2-chinolon-5-ylamino)- 4-methyl-2-(trifluormethyl)pentan-2-ol

### 5-Amino-2-chinolon

1.45 g (8.3 mmol) 5-Amino-2-methoxychinolin werden in 29 mL 6 N HCl 4.5 h unter Rückfluss erhitzt. Man lässt auf Raumtemp. abkühlen, verdünnt mit Wasser, stellt mit NaHCO₃ basisch, extrahiert mit Essigester, trocknet die vereinigten organischen Extrakte (Na₂SO₄) und engt ein. Reinigung des Rückstands durch Chromatographie an Kieselgel mit Hexan-Essigester liefert 670 mg eines gelben Feststoffs.
¹H-NMR ([D]₆-DMSO): δ = 5.85 (s, 2H), 6.25 (d, 1 H), 6.35 (d, 1 H), 6.45 (d, 1 H), 7.1 (t, 1 H), 8.1 (d, 1 H), 11.4 (br.s, 1 H).
*4-(5-Fluor-2-methoxyphenyl)-1-(2-hydroxychinolin-5-ylamino)- 4-methyl-2-(trifluormethyl)pentan-2-oll*
*4-(5-Fluor-2-methoxyphenyl)-1-(2-chinolon-5-ylamino)- 4-methyl-2-(trifluormethyl)pentan-2-ol*

Analog zu Beispiel 20 werden 226 mg (0.73 mmol) 4-(5-Fluor-2-methoxyphenyl)-2-hydroxy-4-methyl-2-(trifluoromethyl)pentanal und 140 mg (0.87 mmol) 5-Amino-2-chinolon zu 200 mg des Produkts umgesetzt.
¹H-NMR ([D]₆-DMSO): δ = 1.35 (s, 3H), 1.55 (s, 3H), 2.05 (d, 1 H), 2.8-3.05 (m, 3H), 3.8 (s, 3H), 5.3 (m, 1 H), 5.7 (d, 1 H), 5.9 (s, 1 H), 6.35 (d, 1 H), 6.55 (d, 1H), 6.9-7.15 (m, 3H), 7.85 (d, 1 H), 11.5 (br.s, 1 H).
MS (ES): m/e = 453.

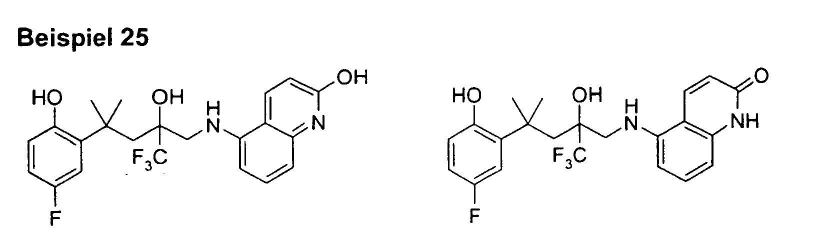

### 4-(5-Fluor-2-hydroxyphenyl)-1-(2-hydroxychinolin-5-ylamino)-4-methyl-2-(trifluormethyl)pentan-2-ol/

### 4-(5-Fluor-2-hydroxyphenyl)-1-(2-chinolon-5-ylamino)- 4-methyl-2-(trifluormethyl)pentan-2-ol

Analog zu Beispiel 21 werden 140 mg (0.31 mmol) 4-(5-Fluor-2-methoxyphenyl)-1-(2-hydroxychinolin-5-ylamino)- 4-methyl-2-(trifluormethyl)pentan-2-ol in 32 mg Produkt umgewandelt.
¹H-NMR ([D]₆-DMSO): δ = 1.4 (s, 3H), 1.55 (s, 3H), 1.9 (d, 1 H), 2.8-3.1 (m, 3H), 5.25 (m, 1H), 5.65 (d, 1 H), 5.9 (s, 1 H), 6.35 (d, 1 H), 6.55 (d, 1 H), 6.75 (dd, 1 H), 6.85 (td, 1 H), 7.0 (d, 1 H), 7.1 (t, 1 H), 7.85 (d, 1 H), 9.75 (br. s, 1 H), 11.5 (br.s, 1H).
MS (ES): m/e = 439.

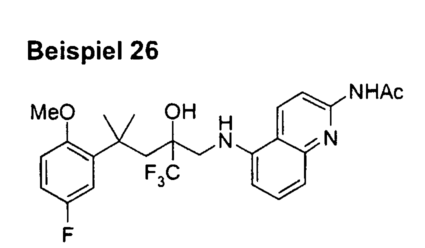

### 1-(2-Acetylaminochinolin-5-ylamino)-4-(5-fluor-2-methoxyphenyl)-4-methyl-2-(trifluormethyl)pentan-2-ol

### 2-Chlor-5-nitrochinolin

Zu 84 mL 100 proz. Salpetersäure werden vorsichtig 69 mL 96 proz. Schwefelsäure getropft. Unter Eiskühlung werden 25 g (152 mmol) 2-Chlorchinolin dazugegeben und der Ansatz 1 h auf 60 °C erwärmt. Nach Abkühlung auf Raumtemp. wird der Ansatz vorsichtig in ein Eis/Wasser-Gemisch eingetragen. Nach 15 min Rühren wird der Feststoff, mit Wasser gewaschen und i. Vak. bei 40 °C getrocknet. Säulenchromatographie an Kieselgel mit Hexan-Essigester liefert 10.7 g eines weißen Feststoffs.
'H-NMR (CDCl₃): δ = 7.65 (d, 1 H), 7.85 (t, 1 H), 8.35 (d, 1 H), 8.4 (d, 1 H), 9.0 (d, 1 H).
*2-Amino-5-nitrochinolin*

450 mg (2.2 mmol) 2-Chlor-5-nitrochinolin, 10 mL 25 proz. Ammoniakwasser und 10 mL THF werden in einem Druckgefäß 8 h bei 120 °C gerührt. Der Ansatz wird mit NaCl-Lösung verdünnt und mit Essigester extrahiert. Die vereinigten organischen Phasen werden getrocknet (Na₂SO₄) und eingeengt: 370 mg Produkt.
'H-NMR ([D]₆-DMSO): δ = 6.9 (br. s, 2H), 7.0 (d, 1 H), 7.65 (t, 1 H), 7.8 (d, 1 H), 7.9 (d, 1 H), 8.35 (d, 1H).
*2-Acetylamino-5-nitrochinolin*

360 mg (1.9 mmol) 2-Amino-5-nitrochinolin werden mit 4 mL (50 mmol) Pyridin und 2 mL (21 mmol) Acetanhydrid 15 h bei Raumtemp. gerührt. Der Ansatz wird in ges. NaHCO₃-Lösung gegossen, 30 min gerührt, mit ges. NaCl-Lösung verdünnt und mit Essigester extrahiert. Die Extrakte werden getrocknet (Na₂SO₄) und eingeengt: 410 mg eines gelben Feststoffs.
¹H-NMR (CDCl₃): δ = 2.3 (s, 3H), 7.75 (t, 1 H), 8.1 (d, 1 H), 8.2 (br. 1 H), 8.25 (d, 1 H), 8.65 (d, 1 H), 9.0 (d, 1H).
*2-Acetylamino-5-aminochinolin*

400 mg (1.7 mmol) 2-Acetylamino-5-nitrochinolin und 105 mg 10 proz. Pd-C werden in 20 mL Essigester-MeOH (3:1) 4 h in einer Wasserstoffatmosphäre bei Raumtemp. gerührt. Der Ansatz wird filtriert, eingeengt und säulenchromatographisch an Kieselgel mit Hexan-Essigester gereinigt: 210 mg Produkt.
¹H-NMR ([D]₆-DMSO): δ = 2.15 (s, 3H), 5.9 (s, 2H), 6.6 (d, 1 H), 6.95 (d, 1 H), 7.35 (t, 1 H), 8.1 (d, 1 H), 8.5 (d, 1 H).
*1-(2-Acetylaminochinolin-5-ylamino)-4-(5-fluor-2-methoxyphenyl)-4-methyl-2-(trifluormethyl)pentan-2-ol*
Analog zu Beispiel 20 werden 263 mg (0.86 mmol) 4-(5-Fluor-2-methoxyphenyl)-2-hydroxy-4-methyl-2-(trifluoromethyl)pentanal und 205 mg (1.0 mmol) 2-Acetylamino-5-aminochinolin zu 197 mg Produkt umgesetzt.
¹H-NMR (CDCl₃): δ = 1.45 (s, 3H), 1.55 (s, 3H), 2.25 (s, 3H), 2.3 (d, 1 H), 2.8 (d, 1 H), 3.15 (dd, 1 H), 3.2 (br., 1H), 3.3 (dd, 1 H), 3.85 (s, 3H), 4.3 (br., 3H), 5.95 (d, 1 H), 6.8 (dd, 2H), 6.9 (td, 1 H), 7.1 (dd, 1 H), 7.2 (d, 1 H), 7.4 (t, 1 H), 8.0 (d, 1 H), 8.3 (m, 2H).
MS (ES): m/e = 494.

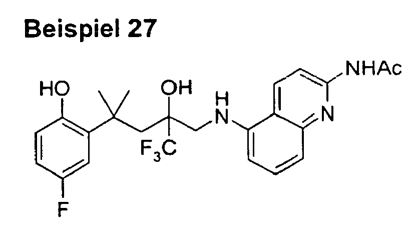

### 1-(2-Acetylaminochinolin-5-ylamino)-4-(5-fluor-2-hydroxyphenyl)-4-methyl-2-(trifluormethyl)pentan-2-ol

Analog zu Beispiel 21 werden 100 mg (0.20 mmol) 1-(2-Acetylaminochinolin-5-ylamino)-4-(5-fluor-2-methoxyphenyl)-4-methyl-2-(trifluormethyl)pentan-2-ol in 79 mg Produkt umgewandelt.
'H-NMR (CDCl₃): δ = 1.5 (s, 3H), 1.6 (s, 3H), 2.2 (s, 3H), 2.35 (d, 1 H), 2.85 (d, 1 H), 3.2 (dd, 1 H), 3.35 (dd, 1 H), 4.4 (br., 3H), 6.05 (d, 1 H), 6.6 (dd, 2H), 6.75 (td, 1 H), 7.1 (dd, 1 H), 7.15 (d, 1 H), 7.4 (t, 1 H), 7.95 (d, 1 H), 8.2 (d, 1 H), 8.35 (br. 1 H).
MS (ES): m/e = 480.

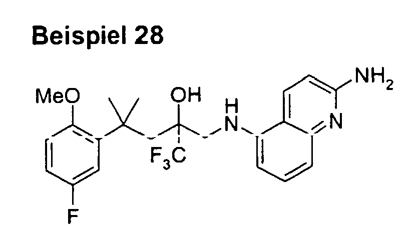

### 1-(2-Aminochinolin-5-ylamino)-4-(5-fluor-2-methoxyphenyl)-4-methyl-2-(trifluormethyl)pentan-2-ol

Eine Lösung von 535 mg (1.05 mmol) 1-(2-Acetylaminochinolin-5-ylamino)-4-(5-fluor-2-methoxyphenyl)-4-methyl-2-(trifluormethyl)pentan-2-ol in 18 mL EtOH-THF (2:1) wird mit 12 mL 3 N Natronlauge 90 min unter Rückfluss erhitzt. Der Ansatz wird mit ges. NaCl verdünnt und mit Essigester extrahiert. Die Extrakte werden getrocknet (Na₂SO₄) und i. Vak. eingeengt. Säulenchromatographie an Kieslegel mit Essigester liefert 380 mg des Produkts als gelbes Öl.
'H-NMR ([D]₆-DMSO): δ = 1.35 (s, 3H), 1.55 (s, 3H), 2.05 (d, 1 H), 2.7-3.0 (m, 3H), 3.8 (s, 3H), 5.05 (m, 1 H), 5.55 (d, 1 H), 6.05 (br., 1H), 6.25 (s, 2H), 6.6 (d, 1 H), 6.75 (d, 1 H), 6.9-7.2 (m, 3H), 7.85 (d, 1 H).
MS (ES): m/e = 452.

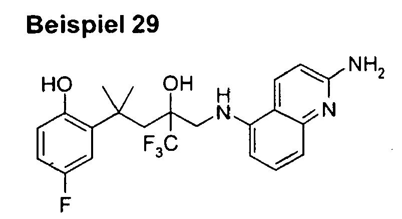

### 1-(2-Aminochinolin-5-ylamino)-4-(5-fluor-2-hydroxyphenyl)-4-methyl-2-(trifluormethyl)pentan-2-ol

Analog zu Beispiel 28 werden 48 mg (0.1 mmol) 1-(2-Acetylaminochinolin-5-ylamino)-4-(5-fluor-2-hydroxyphenyl)-4-methyl-2-(trifluormethyl)pentan-2-ol in 21 mg Produkt umgewandelt.
'H-NMR ([D]₆-DMSO): δ = 1.4 (s, 3H), 1.55 (s, 3H), 1.95 (d, 1 H), 2.85 (dd, 1 H), 3.05 (d, 1 H), 5.05 (m, 1 H), 5.6 (d, 1 H), 6.25 (s, 2H), 6.6 (d, 1 H), 6.7 (m, 2H), 6.8 (m, 1 H), 6.95 (dm, 1 H), 7.1 (t, 1H), 7.85 (d, 1 H).
MS (ES): m/e = 438.

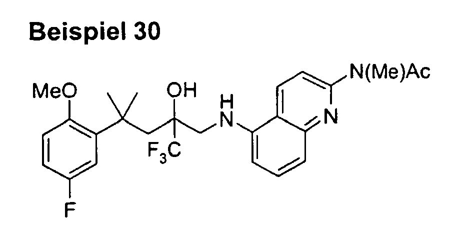

### 1-(2-(Acetyl(methyl)amino)chinolin-5-ylamino)-4-(5-fluor-2-methoxyphenyl)-4-methyl-2-(trifluormethyl)pentan-2-ol

### 2-Methylamino-5-nitrochinolin

1.0 g (4.8 mmol) 2-Chlor-5-nitrochinolin und 20 mL 2 M methanolische Methylamin-Lösung werden in einem Druckgefäß 8 h auf 120 °C erhitzt. Der Ansatz wird nach Zugabe von Toluol eingeengt. Der Rückstand wird säulenchromatographisch an Kieselgel mit Hexan-Essigester gereinigt: 580 mg Produkt.
¹H-NMR (CDCl₃): δ = 3.1 (d, 3H), 4.95 (br., 1H), 6.8 (d, 1 H), 7.55 (t, 1H), 7.95 (2d, 2H), 8.6 (d, 1 H).
*2-Acetyl(methyl)amino-5-nitrochinolin*

580 mg (2.4 mmol) 2-Methylamino-5-nitrochinolin werden mit 4 mL (50 mmol) Pyridin und 2 mL (21 mmol) Acetanhydrid 15 h bei Raumtemp. und 4.5 h bei 60 °C gerührt. Der Ansatz wird mit Essigester verdünnt, in ges. NaHCO₃-Lösung gegossen, 30 min gerührt, und mit Essigester extrahiert. Die Extrakte werden getrocknet (Na₂SO₄) und eingeengt. Der Rückstand wird an Kieselgel mit Hexan-Essigester gereinigt: 660 mg eines gelben Feststoffs.
¹H-NMR (CDCl₃): δ = 2.35 (s, 3H), 3.6 (s, 3H), 7.75 (t, 1 H), 7.9 (d, 1 H), 8.25 (d, 1 H), 8.3 (d, 1 H), 9.0 (d, 1 H).
*2-Acetyl(methyl)amino-5-aminochinolin*

650 mg (2.7 mmol) 2-Acetyl(methyl)amino-5-nitrochinolin und 161 mg 10 proz. Pd-C werden in 25 mL Essigester 2 h in einer Wasserstoffatmosphäre bei Raumtemp. gerührt. Der Ansatz wird filtriert und eingeengt: 490 mg Produkt. ¹H-NMR (CDCl₃): δ = 2.2 (s, 3H), 3.5 (s, 3H), 4.2 (br., 2H), 6.8 (d, 1 H), 7.4 (d, 1H), 7.45 (d, 1 H), 7.5 (t, 1 H), 8.2 (d, 1H).
*1-(2-(Acetyl(methyl)amino)chinolin-5-ylamino)-4-(5-fluor-2-methoxyphenyl)-4-methyl-2-(trifluormethyl)pentan-2-ol*
Analog zu Beispiel 20 werden 576 mg (1.9 mmol) 4-(5-Fluor-2-methoxyphenyl)-2-hydroxy-4-methyl-2-(trifluoromethyl)pentanal und 480 mg (2.2 mmol) 2-Acetyl(methyl)amino-5-aminochinolin zu 330 mg Produkt umgesetzt.
¹H-NMR (CDCl₃): δ = 1.45 (s, 3H), 1.55 (s, 3H), 2.2 (s, 3H), 2.4 (d, 1 H), 2.75 (d, 1 H), 3.15 (dd, 1 H), 3.2 (s, 1 H), 3.35 (dd, 1 H), 3.5 (s, 3H), 3.85 (s, 3H), 4.3 (m, 1H), 6.1 (d, 1 H), 6.8 (dd, 1 H), 6.95 (td, 1 H), 7.1 (dd, 1 H), 7.3-7.5 (m, 3H), 8.0 (d, 1 H).
MS (ES): m/e = 508.

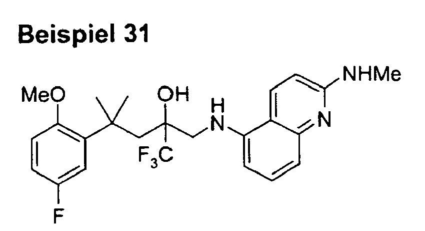

### 4-(5-Fluor-2-methoxyphenyl)-4-methyl-1-(2-(methylamino)chinolin-5-ylamino)-2-(trifluormethyl)pentan-2-ol

Analog zu Beispiel 28 werden 83 mg (0.16 mmol) 1-(2-(Acetyl(methyl)amino)chinolin-5-ylamino)-4-(5-fluor-2-methoxyphenyl)-4-methyl-2-(trifluormethyl)pentan-2-ol in 48 mg Produkt umgewandelt.
'H-NMR (CDCl₃): δ = 1.4 (t, 3H), 1.5 (s, 3H), 2.2 (d, 1 H), 2.75 (d, 1 H), 2.95 (d, 3H), 3.0 (m, 1 H), 3.2 (m, 1 H), 3.8 (s, 3H), 4.0 (m, 1 H), 5.05 (br., 1 H), 5.75 (d, 1 H), 6.5 (d, 2H), 6.75 (dd, 1 H), 6.9 (td, 1 H), 7.05 (dd, 1 H), 7.1 (m, 1 H), 7.2 (m, 1 H), 7.65 (d, 1 H).
MS (ES): m/e = 466.

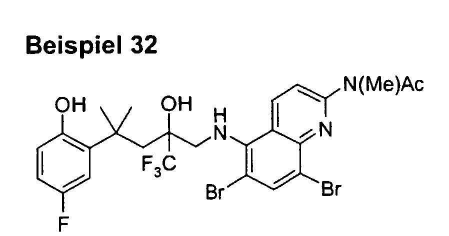

### 1-(2-(Acetyl(methyl)amino)-6 8-dibromchinolin-5-ylamino)-4-(5-fluor-2-hydroxyphenyl)-4-methyl-2-(trifluormethyl)pentan-2-ol

200 mg (0.39 mmol) 1-(2-(Acetyl(methyl)amino)chinolin-5-ylamino)-4-(5-fluor-2-methoxyphenyl)-4-methyl-2-(trifluormethyl)pentan-2-ol in 17 ml CH₂Cl₂ werden bei 0 °C mit 7.6 ml 1 M Bortribromid-CH₂Cl₂-Lösung versetzt. Nach 15 h bei Raumtemperatur werden nochmals mit 7.6 ml 1 M Bortribromid-CH₂Cl₂-Lösung hinzugefügt und 20 h bei Raumtemp. gerührt. Der Ansatz wird in gesättigte NaHCO₃-Lösung gegossen, mit ges. NaCl-Lösung und Essigester verdünnt, 15 Minuten gerührt und mit Essigester extrahiert. Die vereinigten organischen Extrakte werden getrocknet (Na₂SO₄) und eingeengt. Chromatographie mit Hexan-Essigester an Kieselgel liefert 98 mg des Produkts.. 'H-NMR (CDCl₃): δ = 1.4 (t, 3H), 1.55 (s, 3H), 2.3 (d, 1 H), 2.45 (s, 3H), 2.6 (d, 1 H), 2.95 (t, 1 H), 3.25 (dd, 1 H), 3.65 (s, 3H), 3.95 (s, 1 H), 4.1 (m, 1 H), 5.7 (br. s, 1 H), 6.45 (dd, 1 H), 6.6 (td, 1 H), 6.9 (dd, 1 H), 7.6 (d, 1 H), 7.95 (d, 1 H), 8.05 (s, 1 H).
MS (ES): m/e = 650, 652, 654 (1:2:1)

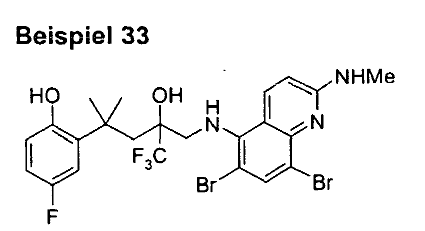

### 1-(6,8-Dibrom-2-(methylamino)chinolin-5-ylamino)-4-(5-fluor-2-hydroxyphenyl)-4-methyl-2-(trifluormethyl)pentan-2-ol

Analog zu Beispiel 28 werden 100 mg (0.15 mmol) 1-(2-(Acetyl(methyl)amino)-6,8-dibromchinolin-5-ylamino)-4-(5-fluor-2-hydroxyphenyl)-4-methyl-2-(trifluormethyl)pentan-2-ol in 97 mg Produkt umgewandelt.
'H-NMR ([D]₆-DMSO): δ = 1.3 (s, 3H), 1.5 (s, 3H), 1.95 (d, 1 H), 2.95 (d, 3H), 3.0 (d, 1 H), 3.15 (dd, 1 H), 4.4 (m, 1 H), 6.15 (s, 1 H), 6.65 (m, 2H), 6.75 (td, 1 H), 6.85 (dd, 1 H), 7.35 d, 1 H), 7.55 (d, 1 H), 7.8 (s, 1 H), 9.55 (s, 1 H).

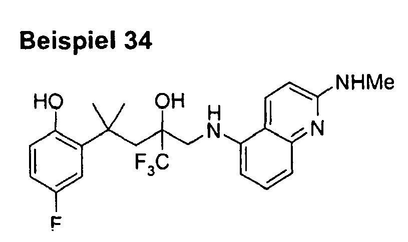

### 4-(5-Fluor-2-hydroxyphenyl)-4-methyl-1 -(2-(methylamino)chinolin-5-ylamino)-2-(trifluormethyl)pentan-2-ol

45 mg (0.07 mmol) 1-(6,8-Dibrom-2-(methylamino)chinolin-5-ylamino)-4-(5-fluor-2-hydroxyphenyl)-4-methyl-2-(trifluormethyl)pentan-2-ol und 50 mg 10 proz. Pd-C werden in 2 mL 90 min unter Wasserstoffatmosphäre gerührt. Der Ansatz wird filtriert und eingeengt. Säulenchromatographie an Kieselgel mit Essigester-MeOH liefert 19 mg des Produkts.
¹H-NMR ([D]₆-DMSO): δ = 1.4 (s, 3H), 1.55 (s, 3H), 1.9 (d, 1 H), 2.8 (d, 3H und m, 1 H), 3.05 (d, 1 H), 3.15 (d, 1 H), 4.95 (m, 1 H), 5.6 (d, 1 H), 5.95 (s, 1 H), 6.6 (d, 1H), 6.75 (dd, 1H), 6.8 (d, 1H), 6.85 (td, 1H), 7.0 (dd, 1H), 7.05 (t, 1H), 7.8 (d, 1 H), 9.7 (s, 1 H).
MS (ES): m/e = 452.

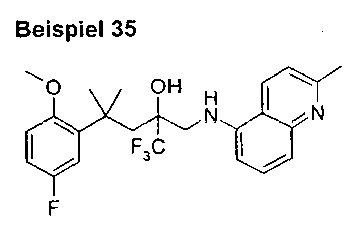

### 4-(5-Fluor-2-methoxyphenyl)-4-methyl-1-(2-methylchinolin-5-ylamino)-2-(trifluormethyl)pentan-2-ol

300 mg (0.97 mmol) 4-(5-Fluor-2-methoxyphenyl)-2-hydroxy-4-methyl-2-(trifluormethyl)pentanal und 184 mg (1.16 mmol) 5-Amino-2-methylchinolin werden in 10 ml Essigsäure über 6 h auf 125°C erhitzt. Nach dem Abkühlen auf Raumtemperatur versetzt man mit 320 mg (1.51 mmol) Natriumtriacetoxyborhydrid und lässt 16 h rühren. Nach der Zugabe von weiteren 320 mg (1.51 mmol) Natriumtriacetoxyborhydrid und 2 Stunden Rühren gibt man Toluol zu und engt im Vakuum ein. Der Rückstand wird in Ethylacetat aufgenommen, die organische Phase mit ges. Natriumhydrogencarbonat und ges. Natriumchlorid Lösung gewaschen und über Natriumsulfat getrocknet. Nach Chromatographie an Kieselgel mit Hexan-Essigester (0-60 %) werden 221 mg des Produkts erhalten.
¹H-NMR (CDCl₃); δ = 1.46 (s, 3H), 1.57 (s, 3H), 2.33 (d, 1H), 2.72 (s, 3H), 2.78 (d, 1 H), 3.12 (dd, 1 H), 3.30 (dd, 1 H), 3.84 (s, 3H), 4.23 (br., 1H), 6.01 (d. 1 H). 6.80 (dd, 1 H), 6.94 (ddd, 1 H), 7.12 (dd, 1 H), 7.21 (d, 1 H), 7.41 (t, 1 H), 7.46 (d, 1 H), 7.88 (d, 1 H).

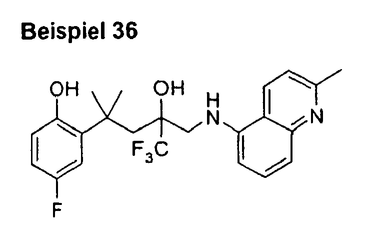

### 4-(5-Fluor-2-hydroxyphenyl)-4-methyl-1-(2-methylchinolin-5-ylamino)-2-(trifluormethyl)-pentan-2-ol

Analog zu Beispiel 35 werden 153 mg (0.34 mmol) 4-(5-Fluor-2-methoxyphenyl)-4-methyl-1-(2-methylchinolin-5-ylamino)-2-(trifluormethyl)-pentan-2-ol
in 17 ml CH₂Cl₂ mit 6.8 ml 1 M Bortribromid-CH₂Cl₂-Lösung umgesetzt. Nach Chromatographie an Kieselgel mit Hexan-Essigester (0-55 %) werden 99 mg des Produkts erhalten.
¹H-NMR (CDCl₃) ; δ = 1.51 (s, 3H), 1.59 (s, 3H), 2.41 (d, 1 H), 2.70 (s, 3H), 2.80 (d, 1 H), 3.24 (dd, 1 H), 3.42 (dd, 1 H), 4.32 (br, 1 H), 6.06 (d, 1 H), 6.63 (dd, 1 H), 6.80 (ddd, 1 H), 7.09 (dd, 1 H), 7.18 (d, 1 H), 7.35 (t, 1 H), 7.42 (d, 1 H), 7.87 (d, 1H).

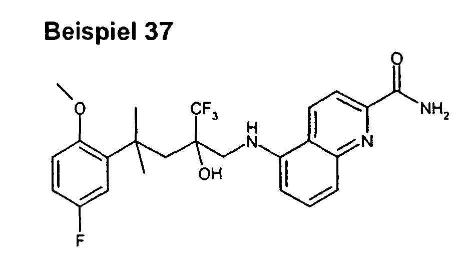

### 5-[4-(5-Fluor-2-methoxyphenyl)-2-hydroxy-4-methyl-2-trifluormethylpentylamino]chinolin-2-carbonsäureamid 5-Aminochinolin-2-carbonsäureamid

840 mg (4.16 mmol) 5-Aminochinolin-2-carbonsäuremethylester werden in 70 mL einer 7N methanolischen Ammoniaklösung gelöst. Es wird 3.5 Stunden bei 40°C, anschließend 20 Stunden bei Raumtemperatur gerührt. Nach Entfernung des Lösungsmittels im Vakuum erfolgt die Reinigung an Kieselgel mit Hexan-Ethylacetat (0-100%) sowie mit Ethylacetat-Methanol (0-10%). Man erhält 690 mg (88% der Theorie) des Produkts
¹H-NMR (DMSO-d₆): δ = 6.11 (s, 2H), 6.78 (d, 1 H), 7.27 (d, 1 H), 7.51 (t, 1 H), 7.70 (s, 1 H), 7.96 (d, 1 H), 8.20 (s, 1 H), 8.68 (d, 1 H). *5-[4-(5-Fluor-2-methoxyphenyl)-2-hydroxy-4-methyl-2-trifluormethylpentylidenamino]-chinolin-2-carbonsäureamid*

Eine Lösung aus 290 mg (1.55 mmol) 5-Aminochinolin-2-carbonsäureamid, 616 mg (2.0 mmol) 4-(5-Fluor-2-methoxyphenyl)-2-hydroxy-4-methyl-2-trifluormethylpentanal 3.80 mL konz. Essigsäure in 30 mL Toluol wird 20 Stunden am Wasserabscheider unter Rückfluss gekocht. Anschließend wird das Lösungsmittel im Vakuum entfernt. Nach Reinigung des Rückstands an Kieselgel mit Hexan-Ethylacetat (0-70%) werden 438 mg (59% der Theorie) des Produkts erhalten.
¹H-NMR (300 MHz, DMSO-d₆): δ = 1.33 (s, 3H), 1.53 (s, 3H), 2.20 (d, 1 H), 3.33 (d, 1 H), 3.77 (s, 3H), 6.31 (s, 1 H), 6.48-6.55 (m, 1 H), 6.70-6.75 (m, 3H), 7.59 (s, 1 H), 7.70 (t, 1 H), 7.82 (br, 1 H), 7.98 (d, 1 H), 8.31 (br, 1 H), 8.82 (d, 1 H).
*5-[4-(5-Fluor-2-methoxyphenyl)*-2*-hydroxy-4-methyl-2-trifluormethylpentylamino]chinolin-2-carbonsäureamid* 253 mg (0.53 mmol) 5-[4-(5-Fluor-2-methoxyphenyl)-2-hydroxy-4-methyl-2-trifluormethylpentylidenamino]chinolin-2-carbonsäureamid werden in 10 mL Tetrahydrofuran-Methanol (50%) gelöst und mit 101 mg (2.65 mmol) Natriumborhydrid versetzt. Nach 20 Stunden wird das Lösungsmittel im Vakuum entfernt. Die anschließende Umkristallisation des Rückstands aus Ethylacetat-Methanol und Reinigung der eingeengten Mutterlauge an Kieselgel mit Hexan-Ethylacetat (0-50%) liefern 116 mg (46% der Theorie) des Produkts.
¹H-NMR (300 MHz, DMSO-d₆): δ = 1.38 (s, 3H), 1.56 (s, 3H), 2.05 (d, 1 H), 2.91-2.96 (m, 2H), 3.07 (d, 1 H), 3.78 (s, 3H), 5.59 (t, 1 H), 5.99 (s, 1 H), 6.02 (d, 1H), 6.91-7.06 (m, 2H), 7.08 (dd, 1H). 7.32 (d, 1 H); 7.46 (t, 1 H), 7.72 (br, 1 H). 8.03 (d,1H),8.21 (br, 1 H), 8.49 (d, 1 H).

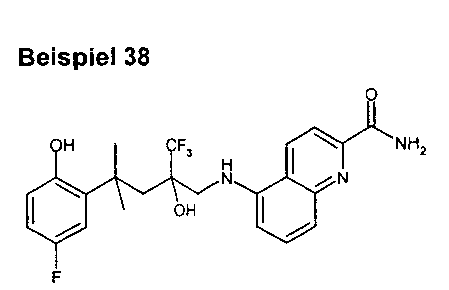

### 5-[4-(5-Fluor-2-hydroxyphenyl)-2-hydroxy-4-methyl-2-trifluormethylpentylamino]chinolin-2-carbonsäureamid

220 mg (0.46 mmol) 5-[4-(5-Fluor-2-methoxyphenyl)-2-hydroxy-4-methyl-2-trifluormethylpentylamino]chinolin-2-carbonsäureamid werden in 5.0 mL Dichlormethan gelöst und mit 9.2 mL einer 1 N Bortribromidlösung in Dichlormethan versetzt. Nach 20 Stunden bei Raumtemperatur erfolgt der Abbruch der Reaktion durch Zugabe von Methanol. Man entfernt das Lösungsmittel im Vakuum, nimmt den Rückstand in gesättigter Natriumhydrogencarbonatlösung und Ethylacetat auf, extrahiert mit Ethylacetat und trocknet die vereinigten organischen Phasen über Natriumsulfat. Nach Entfernen des Lösungsmittels und Reinigung an Kieselgel mit Hexan-Ethylacetat (0-100%) erhält man 60 mg (28 % der Theorie) des Produkts.
¹H-NMR (300 MHz, DMSO-d₆): δ = 1.40 (s, 3H), 1.57 (s, 3H), 1.93 (d, 1 H), 2.98 (dd, 1 H), 3.16-3.20 (m, 2H), 5.52 (br, 1 H), 5.99 (br, 1 H), 6.68 (dd, 1 H), 6.78-6.84 (m, 1 H), 7.01 (dd, 1 H), 7.32 (d, 1 H), 7.45 (t, 1 H), 7.71 (s, 1 H), 8.03 (d, 1 H), 8.21 (s, 1 H), 8.46 (d, 1 H), 9.73 (s, 1 H).

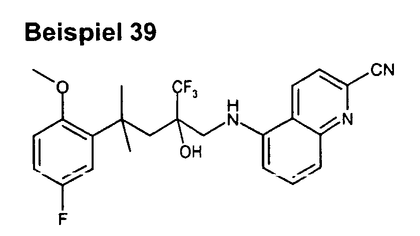

### 5-[4-(5-Fluor-2-methoxyphenyl)-2-hydroxy-4-methyl-2-trifluormethylpentylamino]chinolin-2-carbonsäurenitril

### 5-[4-(5-Fluor-2-methoxyphenyl)-2-hydroxy-4-methyl-2-trifluormethylpentylidenamino]-chinolin-2-carbonsäurenitril

220 mg (0.46 mmol) 5-[4-(5-Fluor-2-methoxyphenyl)-2-hydroxy-4-methyl-2-trifluormethylpentylidenamino]chinolin-2-carbonsäureamid und 1.80 mL (3.2 mmol) Triethylamin werden in 18 mL Dichlormethan gelöst und mit 0.44 mL (1.38 mmol) Trifluoressigsäureanhydrid versetzt. Nach 2 Minuten erfolgt der Abbruch der Reaktion durch Zugabe von Wasser. Man extrahiert dreimal mit Ethylacetat, wäscht die vereinigten organischen Phasen mit einer 1 N-Natriumhydroxidlösung. Nach Trocknen über Natriumsulfat, Entfernen des Lösungsmittels im Vakuum sowie Chromatographie an Kieselgel mit Hexan-Ethylacetat (0-100%) erhält man 190 mg (90% der Theorie) des Produkts.
¹H-NMR (300 MHz, CDCl₃): δ = 1.37 (s, 3H), 1.57 (s, 3H), 2.30 (d, 1H), 3.47 (d, 1H), 3.80 (s, 3H), , 4.69 (s, 1H), 6.38-6.43 (m, 1H), 6.59-6.60 (m, 2H), 6.80 (dd, 1 H), 7.54 (s, 1 H), 7.65 (t, 1 H), 7.74 (d, 1 H), 8.03 (d, 1 H), 8.44 (d, 1 H).
*5-[4-(5-Fluor-2-methoxyphenyl)-2-hydroxy-4-methyl-2-trifluormethylpentylamino]chinolin-2-carbonsäurenitril*
Analog Beispiel 37 werden 90 mg (0.2 mmol) 5-[4-(5-Fluor-2-methoxyphenyl)-2-hydroxy-4-methyl-2-trifluormethylpentylidenamino]-chinolin-2-carbonsäurenitril mit 31 mg (0.8 mmol) Natrium in 5.0 mL Methanol und 1.0 mL Tetrahydrofuran umgesetzt. Nach Aufarbeitung und Chromatographie an Kieselgel mit Hexan-Ethylacetat (0-100%) erhält man 50 mg (54% der Theorie) des Produkts.
¹H-NMR (300 MHz, DMSO-d₆): δ = 1.38 (s, 3H), 1.56 (s, 3H), 2.05 (d, 1 H), 2.88-3.11 (m, 3H), 3.78 (s, 3H), 5.82-5.84 (m, 1 H), 5.94 (s, 1 H), 6.14 (d, 1 H), 6.89-7.09 (m, 3H), 7.31 (d, 1 H), 7.54 (t, 1 H), 7.93 (d, 1 H), 8.61 (d, 1 H).

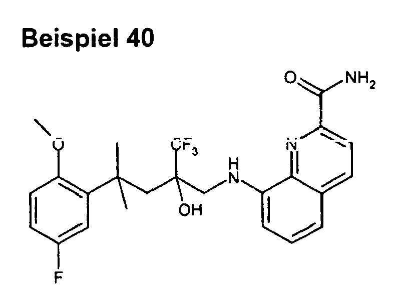

### 8-[4-(5-Fluor-2-methoxyphenyl)-2-hydroxy-4-methyl-2-trifluormethypentylamino]chinolin-2-carbonsäureamid

### 8-Aminochinolin-2-carbonsäureamid

Analog Beispiel 37 werden 120 mg (0.59 mmol) 8-Aminochinolin-2-carbonsäuremethylester mit 10 mL einer 7N methanolischen Ammoniaklösung umgesetzt. Nach Reinigung an Kieselgel mit Hexan-Ethylacetat erhält man 79 mg (72% der Theorie) des Produkts.
¹H-NMR (300 MHz, DMSO-d₆): δ = 6.51 (br, 2H), 6.84 (d, 1 H), 7.04 (d, 1 H), 7.35 (t, 1 H), 7.58 (br, 1 H), 8.03 (d, 1 H), 8.27 (d, 1 H), 8.87 (br, 1 H). *8-[4-(5-Fluor-2-methoxyphenyl)-2-hydroxy-4-methyl-2-trifluormethylpentylidenamino]-chinolin-2-carbonsäureamid* Analog zu Beispiel 37 werden 535 mg (2.86 mmol) eines Gemisches aus 5- und 8-Aminochinolin-2-carbonsäureamid mit 1.06 g (3.43 mmol) 4-(5-Fluor-2-methoxyphenyl)-2-hydroxy-4-methyl-2-(trifluormethyl)pentanal in 4.50 mL konz. Essigsäure und 20 mL Toluol umgesetzt. Nach 40 Stunden erfolgt die Reinigung an Kieselgel mit Hexan-Ethylacetat (0-40%) und man erhält 624 mg (46% der Theorie) des gewünschten Produkts.
¹H-NMR (300 MHz, CDCl₃): δ = 1.37 (s, 3H), 1.60 (s, 3H), 2.27 (d, 1H), 3.46 (d, 1 H), 3.70 (s, 3H), 5.08 (s, 1 H), 5.60 (br, 1 H), 6.34-6.48 (m, 2H), 6.83 (dd, 1 H), 7.00 (d, 1 H), 7.49-7.59 (m, 2H), 7.65-7.78 (m, 2H), 8.32 (s, 2H).
*8-[4-(5-Fluor-2-methoxyphenyl)-2-hydroxy-4-methyl-2-trifluormefhylpenfylamino]chinolin-2-carbonsäureamid*
703 mg (1.47 mmol) 8-[4-(5-Fluor-2-methoxyphenyl)-2-hydroxy-4-methyl-2-trifluormethyl-pentylidenamino]chinolin-2-carbonsäureamid in 10 mL Methanol und 5.0 mL Tetrahydrofuran werden mit 449 mg (11.8 mmol) Natriumborhydrid versetzt. Nach 16 Stunden engt man das Lösungsmittel ein, nimmt den Rückstand in Wasser und Ethylacetat auf, extrahiert mit Ethylacetat und trocknet die vereinigten organischen Phasen über Natriumsulfat. Nach Entfernen des Lösungsmittels und Reinigung an Kieselgel mit Hexan-Ethylacetat (0-50%) sowie mit Dichlormethan-Methanol (0-7%) erhält man 358 mg (51 % der Theorie) des Produkts.
¹H-NMR (300 MHz, DMSO-d₆): δ = 1.39 (s, 3H), 1.58 (s, 3H), 2.10 (d, 1 H), 2.86 (d, 1 H), 3.13 (d, 2H), 3.76 (s, 3H), 5.87 (s, 1 H), 6.24 (d, 1 H), 6.81-6.97 (m, 3H), 7.06-7.11 (m, 2H), 7.32 (t, 1 H), 7.76 (br, 1 H), 8.06 (d, 1 H), 8.31 (d, 1 H), 8.45 (br, 1 H).

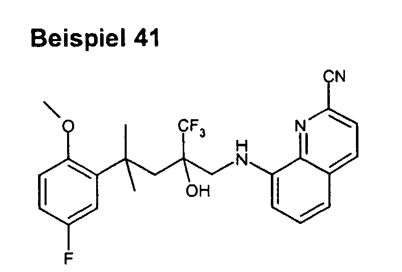

### 8-[4-(5-Fluor-2-methoxyphenyl)-2-hydroxy-4-methyl-2-trifluormethylpentylamino]chinolin-2-carbonsäurenitril

185 mg (0.386 mmol) 8-[4-(5-Fluor-2-methoxyphenyl)-2-hydroxy-4-methyl-2-trifluormethylpentylamino]chinolin-2-carbonsäureamid in 5.0 mL Dimethylformamid werden mit 397 mg (2.80 mmol) Diphosphorpentoxid versetzt. Nach fünf Tagen bei Raumtemperatur werden unlösliche Bestandteile abfiltriert. Das Filtrat wird mit Ethylacetat und gesättigter Natriumchloridlösung verdünnt. Man extrahiert mit Ethylacetat, trocknet die vereinigten organischen Phasen über Natriumsulfat und engt anschließend das Lösungsmittel ein. Dimethylformamid-Reste werden im Hochvakuum entfernt. Nach Reinigung an Kieselgel mit Hexan-Ethylacetat (0-30%) erhält man 102 mg (57% der Theorie) des Produkts.
¹H-NMR (300 MHz, DMSO-d₆): δ = 1.37 (s, 3H), 1.56 (s, 3H), 1.99 (d, 1H), 2.95 (dd, 1 H), 3.03 (d, 1 H), 3.13 (dd, 1 H), 3.79 (s, 3H), 6.16-6.17 (m, 2H), 6.23 (d, 1 H), 6.76-6.79 (m, 2H), 7.01 (dd, 1 H), 7.15 (d, 1 H), 7.43 (t, 1 H), 7.96 (d, 1H), 8.44 (d, 1 H).

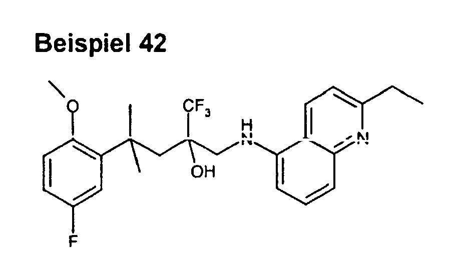

### 1-(2-Ethylchinolin-5-ylamino)-4-(5-fluor-2-methoxyphenyl)-4-methyl-2-(trifluormethyl)pentan-2-ol

### 2-Chlor-5-nitrochinolin

10.0 g (61.1 mmol) 2-Chlorchinolin werden in 34 mL konz. Schwefelsäure gelöst. Bei 0°C werden 8.4 g (76.4 mmol) Kaliumnitrat portionsweise hinzugefügt. Nach 20 Stunden bei Raumtemperatur wird das Reaktionsgemisch auf Eiswasser gegossen, die wässrige Phase wird mit Ethylacetat extrahiert. Die vereinigten organischen Phasen werden mit gesättigter Natriumhydrogencarbonatlösung gewaschen und über Natriumsulfat getrocknet. Nach Entfernen des Lösungsmittels im Vakuum und Chromatographie an Kieselgel mit Hexan-Ethylacetat (10-100%) werden 5.06 g (40% der Theorie) des Produkts erhalten.
¹H-NMR (300 MHz, CDCl₃): δ = 7.62 (d, 1H), 7.84 (t, 1 H), 8.33 (d, 1 H), 8.39 (d, 1 H), 8.98 (d, 1 H).
*5-Nitro-2-vinylchinolin*
5.06 g (24.5 mmol) 2-Chlor-5-nitrochinolin, 1.26 g (4.9 mmol) Triphenylphosphin und 8.0 g (25.2 mmol) Tri-n-butylvinylzinn werden in 60 mL Toluol gelöst. Nach Zugabe von 2.75 g (2.5 mmol) Tris(dibenzylidenaceton)dipalladium lässt man das Reaktionsgemisch 20 Stunden unter Rückfluss kochen. Anschließend wird über Celite filtriert und mit Ethylacetat gewaschen. Das Filtrat wird mit gesättigter Ammoniumchloridlösung versetzt. Man extrahiert mit Ethylacetat und trocknet die vereinigten organischen Phasen über Natriumsulfat. Nach Entfernen des Lösungsmittels im Vakuum und anschließender Chromatographie an Kieselgel mit Hexan-Ethylacetat (5-20%) werden 3.05 g (62% der Theorie) des Produkts erhalten.
¹H-NMR (300 MHz, CDCl₃): δ = 5.78 (d, 1 H), 6.40 (d, 1 H), 7.03 (dd, 1 H), 7.75-7.81 (m, 2H), 8.28-8.38 (m., 2H), 8.95 (d, 1 H).
*2-Ethylchinolin-5-ylamin*
1.0 g (5.0 mmol) 5-Nitro-2-vinylchinolin werden in 30 mL Ethylacetat gelöst. Nach Zugabe von 100 mg Palladium auf Kohle und 50 mg Natriumcarbonat lässt man das Reaktionsgemisch 20 Stunden bei Raumtemperatur unter Wasserstoffatmosphäre rühren. Es wird anschließend über Celite filtriert und mit Ethylacetat gewaschen. Nach Entfernen des Lösungsmittels im Vakuum und Chromatographie an Kieselgel mit Hexan-Ethylacetat (0-100%), dann mit Ethylacetat-Methanol (0-30%) werden 720 mg (84% der Theorie) des Produkts erhalten.
¹H-NMR (300 MHz, DMSO-d₆): δ = 1.29 (t, 3H), 2.86 (q, 2H), 5.87 (br, 2H), 6.63 (d, 1H), 7.09 (d, 1 H), 7.25 (d, 1 H), 7.35 (t, 1 H), 8.41 (d, 1 H). *1-(2-Ethylchinolin-5-ylimino)-4-(5-fluor-2-methoxyphenyl)-4-methyl-2-(trifluormethyl)pentan-2-ol*
Analog zu Beispiel 37 werden 334 mg (1.94 mmol) 2-Ethylchinolin-5-ylamin mit 500 mg (1.62 mmol) 4-(5-Fluor-2-methoxyphenyl)-2-hydroxy-4-methyl-2-trifluormethylpentanal in 2.20 mL konz. Essigsäure und 15 mL Toluol umgesetzt. Nach Reinigung an Kieselgel mit Hexan-Ethylacetat (0-100%) werden 600 mg (80% der Theorie) des entsprechenden Imins erhalten.
¹H-NMR (300 MHz, CDCl₃): δ = 1.38 (s, 3H), 1.41 (t, 3H), 1.57 (s, 3H), 2.30 (d, 1 H), 3.03 (q, 2H), 3.42 (d, 1H), 3.80 (s, 3H), 4.88 (s, 1 H), 6.40-6.56 (m, 3H), 6.81 (dd, 1 H), 7.37 (d, 1 H), 7.47-7.55 (m, 2H), 7.92 (d, 1 H), 8.20 (d, 1 H). *1-(2-Ethylchinolin-5-ylamino)-4-(5-fluor-2-methoxyphenyl)-4-methyl-2-(trifluormethyl)pentan-2-ol*
600 mg (1.3 mmol) 1-(2-Ethylchinolin-5-ylimino)-4-(5-fluor-2-methoxyphenyl)-4-methyl-2-(trifluormethyl)pentan-2-ol in 5.0 mL Methanol werden bei 0°C mit 197 mg (5.2 mmol) Natriumborhydrid versetzt. Nach 2 Stunden bei Raumtemperatur fügt man Wasser hinzu und entfernt Methanol im Vakuum. Man extrahiert die wässrige Phase mit Dichlormethan, trocknet die vereinigten organischen Phasen über Natriumsulfat und entfernt das Lösungsmittel im Vakuum. Nach Reinigung an Kieselgel mit Hexan-Ethylacetat (0-100%) werden 400 mg (66% der Theorie) des Produkts erhalten.
¹H-NMR (300 MHz, CDCl₃): δ = 1.37 (t, 3H), 1.46 (s, 3H), 1.57 (s, 3H), 2.33 (d, 1H), 2.78 (d, 1H), 2.98 (q, 2H), 3.13 (dd, 1H), 3.19 (br, 1H), 3.30 (dd, 1H), 3.84 (s, 3H), 4.24 (br, 1 H), 6.01 (d, 1 H), 6.80 (dd, 1 H), 6.91-6.98 (m, 1 H), 7.12 (dd, 1H),7.23 (d, 1 H), 7.38-7.49 (m, 2H), 7.91 (d, 1 H).

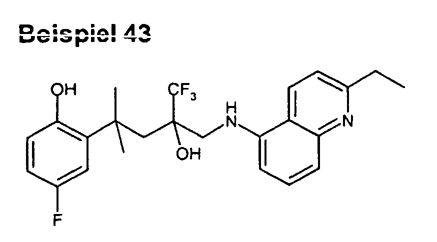

### 1-(2-Ethylchinolin-5-ylamino)-4-(5-fluor-2-hydroxyphenyl)-4-methyl-2-(trifluormethyl)pentan-2-ol

230 mg (0.49 mmol) 1-(2-Ethylchinolin-5-ylimino)-4-(5-fluor-2-methoxyphenyl)-4-methyl-2-(trifluormethyl)pentan-2-ol in 4.5 mL Dichlormethan werden bei 0°C mit 7.30 mL (7.30 mmol) einer 1 M Bortribromidlösung versetzt. Nach 23 Stunden bei Raumtemperatur wird die Reaktion durch Zugabe von 30 mL Methanol zum Abbruch gebracht. Man lässt das Reaktionsgemisch eine Stunde bei Raumtemperatur rühren und entfernt anschließend das Lösungsmittel im Vakuum. Nach Chromatographie an Kieselgel mit Ethylacetat-Methanol (0-10%) werden 60 mg (27% der Theorie) des Produkts erhalten.
¹H-NMR (300 MHz, DMSO-d₆): δ = 1.29 (t, 3H), 1.40 (s, 3H), 1.56 (s, 3H), 1.93 (d, 1 H), 2.87 (q, 2H), 2.95 (d, 1 H), 3.08-3.20 (m, 2H), 5.34 (br, 1 H), 5.89 (d, 1 H), 5.96 (s, 1 H), 6.73 (dd, 1 H), 6.83-6.88 (m, 1 H), 7.01 (dd, 1 H), 7.14 (d, 1 H), 7.27-7.34 (m, 2H), 8.18 (d, 1H), 9.73 (s, 1 H).

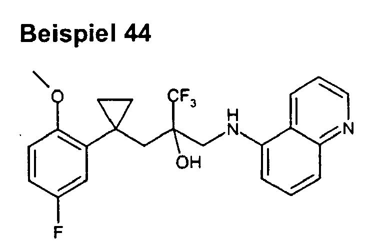

### 1-(Chinolin-5-ylamino)-3-[1-(5-fluor-2-methoxyphenyl)cycloprop-1-yl]-2-(trifluormethyl)propan-2-ol

### 1-(Chinolin-5ylimino)-3-[1-(5-fluor-2-methoxyphenyl)cycloprop-1-yl]-2-(trifluormethyl)propan-2-ol

Analog zu Beispiel 37 werden 362 mg (2.5 mmol) 5-Aminochinolin mit 640 mg (2.09 mmol) 3-[1-(5-Fluor-2-methoxyphenyl)cycloprop-1-yl]-2-hydroxy-2-(trifluormethyl)propanal und 2.80 mL konz. Essigsäure in 19 mL Toluol umgesetzt. Nach 6 Stunden erfolgt die Chromatographie an Kieselgel mit Hexan-Ethylacetat (0-100%) und man erhält 810 mg (90 % der Theorie) des Produkts.
MS (ES+): m/z (r.l. %) = 433 (M+1, 100)
*1-(Chinolin-5-ylamino)-3-[1-(5-fluor-2-methoxyphenyl)cycloprop-1-yl]-2-(trifluormethyl)propan-2-ol*

810 mg (1.87 mmol) 1-(Chinolin-5-ylimino)-3-[1-(5-fluor-2-methoxyphenyl)cycloprop-1-yl]-2-(trifluormethyl)propan-2-ol werden mit 288 mg (7.61 mmol) Natriumborhydrid in 6.0 mL Methanol und 3.0 mL Tetrahydrofuran wie im Beispiel 1 umgesetzt. Nach Chromatographie an Kieselgel mit Hexan-Ethylacetat (0-100%) werden 660 mg (81 % der Theorie) des Produkts erhalten. ¹H-NMR (400 MHz, CDCl₃): δ = 0.85-0.99 (m, 4H), 2.19 (d, 1 H), 2.32 (d, 1 H), 3.19-3.29 (m, 2H), 3.70 (s, 3H), 3.83 (s, 1 H), 4.47 (t, 1 H), 6.31 (d, 1 H), 6.61 (dd, 1 H), 6.84 (ddd, 1 H), 7.06 (dd, 1 H), 7.33 (dd, 1 H), 7.46-7.52 (m, 2H), 8.05 (d, 1H), 8.87 (dd, 1 H).

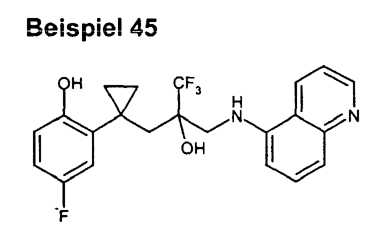

### 1-(Chinolin-5-ylamino)-3-[1-(5-fluor-2-hydroxyphenyl)cycloprop-1-yl]-2-(trifluormethyl)propan-2-ol

Analog zu Beispiel 38 werden 330 mg (0.76 mmol) 1-(Chinolin-5-ylamino)-3-[1-(5-fluor-2-methoxyphenyl)cycloprop-1-yl]-2-(trifluormethyl)propan-2-ol mit 3.80 mL (3.80 mmol) einer 1 M Bortribromidlösung in 6.90 mL Dichlormethan umgesetzt. Nach 2 Stunden erfolgt der Abbruch der Reaktion. Die Umkristallisation aus Ethylacetat und Methanol liefert 292 mg (91 % der Theorie) des Produkts.
¹H-NMR (400 MHz, DMSO-d₆): δ = 0.63-0.65 (m, 1 H), 0.79-0.84 (m, 3H), 1.97 (d, 1 H), 2.62 (d, 1 H), 3.35 (m, 2H), 5.93 (br, 1 H), 6.26 (br, 1 H), 6.42 (dd, 1 H), 6.51 (d, 1 H), 6.64 (td, 1 H), 6.91 (dd, 1 H), 7.31 (d, 1 H), 7.71 (t, 1 H), 7.83 (dd, 1 H), 9.08-9.12 (m, 2H), 9.34 (s, 1 H).

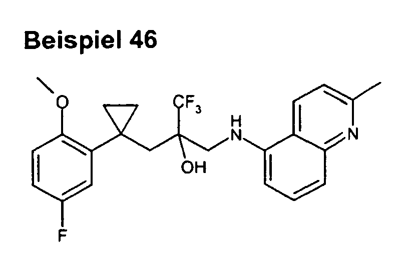

### 3-[1-(5-Fluor-2-methoxyphenyl)cycloprop-1-yl]-1-(2-methylchinolin-5-ylamino)-2-(trifluormethyl)propan-2-ol

### 3-[1-(5-Fluor-2-methoxyphenyl)cycloprop-1-yl]-1-(2-methylchinolin-5-ylimino)-2-(trifluormethyl)propan-2-ol

Analog zu Beispiel 1 werden 352 mg (2.23 mmol) 5-Amino-2-methylchinolin mit 650 mg (2.12 mmol) 3-[1-(5-Fluor-2-methoxyphenyl)cycloprop-1-yl]-2-hydroxy-2-(trifluormethyl)propanal und 2.80 mL konz. Essigsäure in 19 mL Toluol umgesetzt. Nach Chromatographie an Kieselgel mit Hexan-Ethylacetat (0-100%) werden 870 mg (92% der Theorie) des Produkts erhalten.
MS (ES+): m/z (r.I. %) = 447(M+1, 100)
*3-[1-(5-Fluor-2-methoxyphenyl)cycloprop-1-yl]-1-(2-methylchinolin-5-ylamino)-2-(trifluormethyl)propan-2-ol*
870 mg (1.95 mmol) 3-[1-(5-Fluor-2-methoxyphenyl)cycloprop-1-yl]-1-(2-methylchinolin-5-ylimino)-2-(trifluormethyl)propan-2-ol werden analog zu Beispiel 37 mit 96 mg (2.53 mmol) Natriumborhydrid in 6.0 mL Methanol und 3.0 mL Tetrahydrofuran umgesetzt. Nach Umkristallisation aus Hexan und Ethylacetat werden 790 mg (90% der Theorie) des Produkts erhalten.
¹H-NMR (400 MHz, CDCl₃): δ = 0.83-0.97 (m, 4H), 2.19 (d, 1 H), 2.31 (d, 1H), 2.72 (s, 3H), 3.21-3.25 (m, 2H), 3.70 (s, 3H), 3.79 (s, 1 H), 4.41 (t, 1 H), 6.25 (dd, 1 H), 6.61 (dd, 1 H), 6.86 (ddd, 1 H), 7.05 (dd, 1 H), 7.21 (d, 1 H), 7.43-7.44 (m, 2H), 7.94 (d, 1 H).

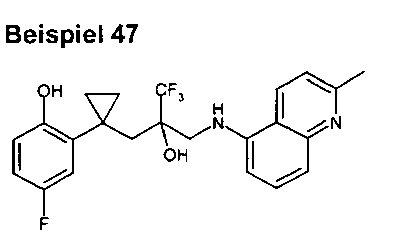

### 3-[1-(5-Fluor-2-hydroxyphenyl)cycloprop-1-yl]-1-(2-methylchinolin-5-ylamino)-2-(trifluormethyl)propan-2-ol

Analog zu Beispiel 38 werden 395 mg (0.88 mmol) 3-[1-(5-Fluor-2-methoxyphenyl)cycloprop-1-yl]-1-(2-methylchinolin-5-ylamino)-2-(trifluormethyl)propan-2-ol mit 4.30 mL (4.3 mmol) einer 1 M Bortribromidlösung in 8.0 mL Dichlormethan umgesetzt. Nach einer Stunde bei 0°C erfolgt der Abbruch der Reaktion. Die anschließende Umkristallisation aus Ethylacetat, Aceton und Methanol liefert 257 mg (67% der Theorie) des Produkts.
MS (ES+): m/z (r.l.%) = 435 (M+1, 100).

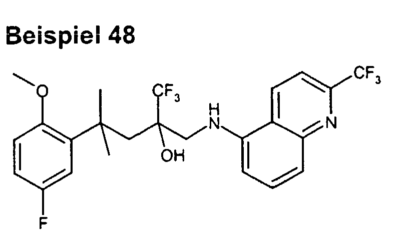

### 4-(5-Fluor-2-methoxyphenyl)-4-methyl-2-trifluormethyl-1-(2-(trifluormethyl)chinolin-5-ylamino)pentan-2-ol

### 2-(Trifluormethyl)chinolin

Nach Literaturvorschrift (Baraznenok, I. L., Nenajdenko, V. G., Balenkova, E. S. *Eur. J. Org. Chem.* **1999,** 937-941) werden 1.2 g (7.18 mmol) (E)-4-(Dimethylamino)-1,1,1-trifluor-3-buten-2-on mit 2.03 g (7.18 mmol) Trifluoressigsäureanhydrid und 1.30 mL (14.36 mmol) Anilin in 72 mL 1,2-Dichlorethan, anschließend in 36 mL Xylol umgesetzt. Nach Aufarbeitung und Reinigung an Kieselgel mit Hexan-Ethylacetat (0-100%) erhält man 1.01 g (71% der Theorie) des Produkts.
*5-Nitro-2-(trifluormethyl)chinolin*
Man löst 1.52 g (7.7 mmol) 2-(Trifluormethyl)chinolin in 7.90 mL konz. Schwefelsäure und fügt bei 0°C 1,47 g Kaliumnitrat portionsweise hinzu. Nach 20 Stunden bei Raumtemperatur wird die Reaktionslösung auf Eis/Wasser gegossen. Man extrahiert mit Ethylacetat, wäscht die vereinigten organischen Phasen mit gesättigter Natriumhydrogencarbonatlösung und trocknet sie über Natriumsulfat. Nach Entfernen des Lösungsmittels und Chromatographie an Kieselgel mit Hexan-Ethylacetat (10-100%) erhält man 390 mg (21% der Theorie) des Produkts.
¹H-NMR (300 MHz, CDCl₃): δ = 7.92-8.00 (m, 2H), 8.53-8.58 (m, 2H), 9.26 (d, 1 H).
*5-Amino-2-(trifluormethyl)chinolin*
390 mg (1.61 mmol) 5-Nitro-2-(trifluormethyl)chinolin werden in 13 mL Methanol gelöst. Nach Zugabe von 39 mg Palladium auf Kohle und 19 mg Kaliumcarbonat lässt man das Reaktionsgemisch 20 Stunden bei Raumtemperatur unter Wasserstoffatmosphäre rühren. Es wird anschließend über Celite filtriert und mit Ethylacetat gewaschen. Nach Entfernen des Lösungsmittels im Vakuum und Chromatographie an Kieselgel mit Hexan-Ethylacetat (0-100%) werden 250 mg (73% der Theorie) des Produkts erhalten.
¹H-NMR (300 MHz, CDCl₃): δ = 4.28 (br, 2H), 6.92 (d, 1 H), 7.58-7.69 (m, 3H), 8.36 (d, 1 H).
*4-(5-Fluor-2-methoxyphenyl)-4-methyl-2-trifluormethyl-1-(2-(trifluormethyl)chinolin-5-ylimino)pentan-2-ol*
Analog zu Beispiel 37 werden 250 mg (1.18 mmol) 5-Amino-2-(trifluormethyl)chinolin mit 438 mg (1.42 mmol) 4-(5-Fluor-2-methoxyphenyl)-2-hydroxy-4-methyl-2-(trifluormethyl)pentanal und 1.30 mL konz. Essigsäure in 20 mL Toluol umgesetzt. Nach Chromatographie an Kieselgel mit Hexan-Ethylacetat (0-100%) werden 500 mg (84% der Theorie) des Produkts erhalten. ¹H-NMR (300 MHz, CDCl₃): δ = 1.38 (s, 3H), 1.58 (s, 3H), 2.31 (d, 1 H), 3.47 (d, 1 H), 3.80 (s, 3H), 4.75 (s, 1 H), 6.39-6.45 (m, 1 H), 6.51-6.58 (m, 2H), 6.82 (dd, 1 H), 7.55 (s, 1 H), 7.64 (t, 1 H), 7.78 (d, 1 H), 8.10 (d, 1 H), 8.49 (d, 1 H).
*4-(5-Fluor-2-methoxyphenyl)-4-methyl-2-trifluormethyl-1-(2-(trifluormethyl)chinolin-5-ylamino)penfan-2-ol*
Analog zu Beispiel 37 werden 500 mg (0.99 mmol) 4-(5-Fluor-2-methoxyphenyl)-4-methyl-2-trifluormethyl-1-(2-(trifluormethyl)chinolin-5-ylimino)-pentan-2-ol mit 154 mg (4.04 mmol) Natriumborhydrid in 5.0 mL Methanol und umgesetzt. Nach Reinigung an Kieselgel mit Hexan-Ethylacetat (0-100%) werden 420 mg (84% der Theorie) des Produkts erhalten.
¹H-NMR (300 MHz, CDCl₃): δ = 1.47 (s, 3H), 1.55 (s, 3H), 2.42 (d, 1H), 2.74 (d, 1 H), 3.03 (s, 1H), 3.16 (dd, 1H), 3.34 (dd, 1 H), 3.85 (s, 3H), 4.38 (dd, 1 H), 6.20 (d, 1 H), 6.79 (dd, 1 H), 6.91-6.97 (m, 1 H), 7.10 (dd, 1 H), 7.52-7.65 (m, 3H), 8.14 (d, 1H).

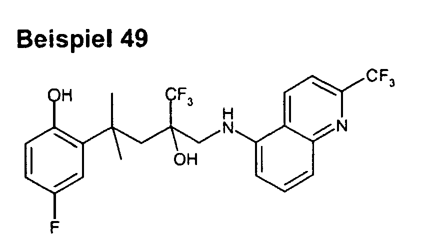

### 4-(5-Fluor-2-hydroxyphenyl)-4-methyl-2-trifluormethyl-1-(2-(trifluormethyl)chinolin-5-ylamino)pentan-2-ol

100 mg (0.20 mmol) 4-(5-Fluor-2-methoxyphenyl)-4-methyl-2-trifluormethyl-1-(2-(trifluormethyl)chinolin-5-ylamino)pentan-2-ol in 2.0 mL Dichlormethan werden bei Raumtemperatur mit 4.0 mL (4.0 mmol) einer 1 M Bortribromidlösung versetzt. Nach 20 Stunden bei Raumtemperatur wird die Reaktion durch Zugabe von 20 mL Methanol zum Abbruch gebracht. Man lässt das Reaktionsgemisch 30 Minuten bei Raumtemperatur rühren und entfernt anschließend das Lösungsmittel im Vakuum. Nach Chromatographie an Kieselgel mit Hexan-Ethylacetat (0-100%) werden 79 mg (80% der Theorie) des Produkts erhalten. ¹H-NMR (300 MHz, CDCl₃): δ = 1.51 (s, 3H), 1.57 (s, 3H), 2.45 (d, 1H), 2.76 (d, 1 H), 3.21-3.27 (m, 2H), 3.42 (dd, 1 H), 4.43 (br, 1 H), 5.60 (br, 1 H), 6.23 (d, 1 H), 6.59 (dd, 1 H), 6.75-6.87 (m, 1H), 7.07 (dd, 1 H), 7.51-7.65 (m, 3H), 8.14 (d, 1 H).

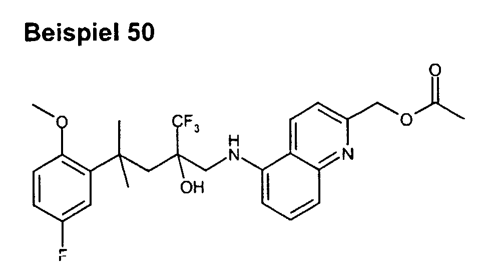

### 1-(2-(Acetoxymethyl)chinolin-5-ylamino)- 4-(5-fluor-2-methoxyphenyl)-4-methyl-2-(trifluormethyl)pentan-2-ol

### 1-(1,2-Dihydroxyethyl)-5-nitrochinolin

Zu einer Lösung aus 3.18 g (15.88 mmol) 5-Nitro-2-vinylchinolin in 140 mL Aceton und 21 mL Wasser fügt man bei 0°C 336 mg (2.5 mmol) N-Methylmorpholin-N-oxidhydrat und 10.22 mL Osmiumteroxidlösung in Isopropanol hinzu und lässt die Reaktionsmischung 24 Stunden bei Raumtemperatur rühren. Dann entfernt man das Lösungsmittel im Vakuum, nimmt den Rückstand in Ethylacetat auf. Die organische Phase wird mit Wasser und gesättigter Natriumchloridlösung gewaschen. Die wässrige Phase wird nochmals mit Dichlormethan, Diethylether und Ethylacetat extrahiert. Die vereinigten organischen Phasen werden über Natriumsulfat getrocknet. Nach Entfernen des Lösungsmittels und Reinigung an Kieselgel mit Hexan-Ethylacetat (0-100%) sowie mit Ethylacetat-Methanol (0-20%) erhält man 1.64 g (44% der Theorie) des Produkts.
¹H-NMR (300 MHz, DMSO-d₆): δ = 3.62-3.81 (m, 2H), 4.78-4.84 (m, 2H), 5.76 (d, 1 H), 7.90-7.96 (m, 2H), 8.37-8.42 (m, 2H), 8.83 (d, 1 H).
*5-Nitrochinolin-2-carbaldehyd*
Zu einer Lösung aus 1.64 g (7.0 mmol) 1-(1,2-Dihydroxyethyl)-5-nitrochinolin in 42 mL Tetrahydrofuran und 7.0 mL Wasser werden 2.99 g (14.0 mmol) Natriumperiodat hinzugefügt. Nach 20 Stunden bei Raumtemperatur fügt man Ethylacetat hinzu und wäscht die organische Phase mit gesättigter Natriumchloridlösung. Nach Trocknen der organischen Phase über Natriumsulfat, Entfernen des Lösungsmittels und Reinigung an Kieselgel mit Hexan-Ethylacetat (0-100%) erhält man 1.40 g (99% der Theorie) des Produkts.
¹H-NMR (300 MHz, CDCl₃): δ = 7.94 (t, 1H), 8.24 (d, 1H), 8.53 (d, 1H), 8.58 (d, 1 H), 9.18 (d, 1 H), 10.24 (s, 1 H).
*2-(Hydroxymethyl)-5-nitrochinolin*
700 mg (3.46 mmol) 5-Nitrochinolin-2-carbaldehyd werden in 13 mL Methanol und 7.0 mL Tetrahydrofuran gelöst und bei 0°C mit 523 mg (13.9 mmol) Natriumborhydrid versetzt. Nach 5 Stunden wird die Reaktion durch Zugabe von Wasser zum Abbruch gebracht. Man entfernt das Lösungsmittel im Vakuum, nimmt den Rückstand in Dichlormethan und Wasser auf, extrahiert die wässrige Phase mit Dichlormethan, trocknet die vereinigten organischen Phasen über Natriumsulfat. Nach Entfernen des Lösungsmittels und Reinigung an Kieselgel mit Hexan-Ethylacetat (0-100%) erhält man 390 mg (55% der Theorie) des Produkts.
¹H-NMR (300 MHz, CDCl₃): δ = 4.13 (br, 1 H), 4.99 (s, 2H), 7.55 (d, 1 H), 7.82 (t, 1 H), 8.36-8.41 (m, 2H), 8.99 (d, 1 H).
*2-(Acetoxymethyl)-5-nitrochinolin*
Man lässt eine Lösung aus 390 mg (1.91 mmol) 2-(Hydroxymethyl)-5-nitrochinolin und 2.5 mL Essigsäureanhydrid in 5.0 mL Pyridin 20 Stunden bei Raumtemperatur rühren. Nach mehrmaligem Coevaporieren mit Toluol und anschließender Chromatographie an Kieselgel mit Hexan-Ethylacetat (0-100%) erhält man 410 mg (87% der Theorie) des Produkts.
¹H-NMR (300 MHz, CDCl₃): δ = 2.23 (s, 3H), 5.42 (s, 2H), 7.70 (d, 1H), 7.81 (t, 1 H), 8.37-8.40 (m, 2H), 9.02 (d, 1 H).
*2-(Acetoxymethyl)-5-aminochinolin*
Unter Wasserstoffatmosphäre lässt man eine Lösung aus 410 mg (1.67 mol) 2-(Acetoxymethyl)-5-nitrochinolin in 61 mL Aceton in Gegenwart von 410 mg Raney-Nickel 2 Stunden bei Raumtemperatur rühren. Man saugt über Celite ab und wäscht mit Aceton nach. Nach Entfernen des Lösungsmittels und anschließender Chromatographie an Kieselgel mit Hexan-Ethylacetat (10-100%) erhält man 230 mg (64 % der Theorie) des Produkts.
¹H-NMR (300 MHz, CDCl₃): δ = 2.19 (s, 3H), 4.20 (br, 2H), 5.37 (s, 2H), 6.81 (dd, 1 H), 7.41 (d, 1 H), 7.51-7.53 (m, 2H), 8.19 (d, 1 H).
*1-(2-(Acetoxymethyl)chinolin-5-ylimino)- 4-(5-fluor-2-methoxyphenyl)-4-methyl-2-(trifluormefhyl)pentan-2-ol*
Analog zu Beispiel 37 werden 230 mg (1.06 mmol) 2-(Acetoxymethyl)-5-aminochinolin mit 273 mg (0.88 mmol) 4-(5-Fluor-2-methoxyphenyl)-2-hydroxy-4-methyl-2-(trifluormethyl)pentanal und 1.20 mL konz. Essigsäure in 20.0 mL Toluol umgesetzt. Nach Chromatographie an Kieselgel mit Hexan-Ethylacetat (0-100%) erhält man 360 mg (81 % der Theorie) des Produkts.
¹H-NMR (300 MHz, CDCl₃) : δ = 1.37 (s, 3H), 1.57 (s, 3H), 2.21 (s, 3H), 2.32 (d, 1H). 3.44 (d, 1H). 3.79 (s, 3H), 4.84 (s, 1 H), 5.40 (s, 2H), 6.42-6.56 (m, 3H), 6.82 (dd, 1 H), 7.45-7.60 (m, 3H), 7.95 (d, 1H), 8.31 (d, 1 H).
*1-(2-(Acetoxymethyl)chinolin-5-ylamino)-4-(5-fluor-2-methoxyphenyl)-4-methyl-2-(trifluormethyl)pentan-2-ol*
Eine Lösung aus 170 mg (0.34 mmol) 1-(2-(Acetoxymethyl)chinolin-5-ylimino)-4-(5-fluor-2-methoxyphenyl)-4-methyl-2-(trifluormethyl)pentan-2-ol in 1.5 mL Methanol und 0.8 mL Tetrahydrofuran wird mit 53 mg (1.38 mmol) Natriumborhydrid versetzt. Nach 3 Stunden bei Raumtemperatur erfolgt der Abbruch der Reaktion durch Zugabe von Wasser. Man extrahiert mit Ethylacetat, wäscht die vereinigten organischen Phasen mit gesättigter Natriumchloridlösung und trocknet sie über Natriumsulfat. Nach Entfernen des Lösungsmittels und Chromatographie an Kieselgel mit Hexan-Ethylacetat (0-100%) erhält man 94 mg (55% der Theorie) des Produkts.
¹H-NMR (300 MHz, CDCl₃): δ = 1.46 (s, 3H), 1.56 (s, 3H), 2.19 (s, 3H), 2.35 (d, 1H), 2.77 (d, 1H), 3.10-3.16 (m, 2H), 3.31 (dd, 1 H), 3.84 (s, 3H), 4.28 (dd, 1H), 5.36 (s, 2H), 6.07 (d, 1 H), 6.80 (dd, 1 H), 6.91-6.98 (m, 1 H), 7.11 (dd, 1 H), 7.37-7.53 (m, 3H), 7.99 (d, 1 H).

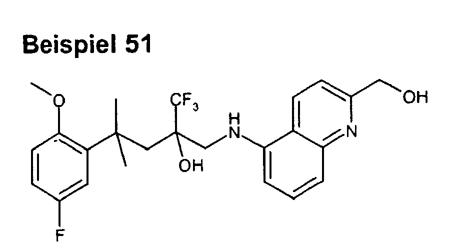

### 4-(5-Fluor-2-methoxyphenyl)-1-(2-(hydroxymethyl)chinolin-5-ylamino)-4-methyl-2-(trifluormethyl)pentan-2-ol

Bei der Umsetzung von 170 mg (0.34 mmol) 1-(2-(Acetoxymethyl)chinolin-5-ylamino)-4-(5-fluor-2-methoxyphenyl)-4-methyl-2-(trifluormethyl)pentan-2-ol mit 53 mg (1.38 mmol) Natriumborhydrid in 1.5 mL Methanol und 0.8 mL Tetrahydrofuran erhält man 43 mg (27% der Theorie) des Produkts (vgl. Beispiel 14).
¹H-NMR (300 MHz, CDCl₃): δ = 1.46 (s, 3H), 1.57 (s, 3H), 2.35 (d, 1 H), 2.77 (d, 1 H), 3.10-3.16 (m, 2H), 3.32 (dd, 1 H), 3.84 (s, 3H), 4.29 (dd, 1 H), 4.41 (br, 1 H), 4.89 (s, 2H), 6.07 (d, 1 H), 6.79 (dd, 1 H), 6.91-6.97 (m, 1 H), 7.12 (dd, 1 H), 7.20 (d,1H), 7.42-7.51 (m,2H), 7.95 (d,1H).

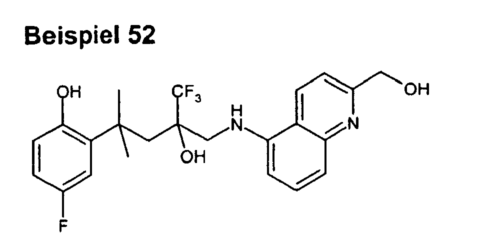

### 4-(5-Fluor-2-hydroxyphenyl)-1-(2-(hydroxymethyl)chinolin-5-ylamino)-4-methyl-2-(trifluormethyl)pentan-2-ol

290 mg (0.62 mmol) 4-(5-Fluor-2-methoxyphenyl)-1-(2-(hydroxymethyl)chinolin-5-ylamino)-4-methyl-2-(trifluormethyl)pentan-2-ol in 7.0 mL Dichlormethan werden bei Raumtemperatur mit 12.5 mL (12.5 mmol) einer 1 M Bortribromidlösung versetzt. Nach 20 Stunden bei Raumtemperatur wird die Reaktion durch Zugabe von Methanol zum Abbruch gebracht. Man entfernt das Lösungsmittel im Vakuum, nimmt den Rückstand in gesättigter Natriumhydrogencarbonatlösung und Ethylacetat auf, extrahiert mit Ethylacetat und trocknet die vereinigten organischen Phasen über Natriumsulfat. Nach Entfernen des Lösungsmittels und Reinigung an Kieselgel mit Hexan-Ethylacetat (0-70%) sowie mit Ethylacetat-Methanol (0-10%) erhält man 160 mg (51 % der Theorie) des Produkts.
¹H-NMR (300 MHz, DMSO-d₆): δ = 1.46 (s, 3H), 1.56 (s, 3H), 1.93 (d, 1 H), 2.94 (dd, 1 H), 3.09-3.21 (m, 2H), 4.67 (d, 2H), 5.34-5.37 (m, 1 H), 5.51 (t, 1 H), 5.91 (d, 1 H), 5.98 (s, 1 H), 6.73 (dd, 1 H), 6.81-6.87 (m, 1 H), 7.01 (dd, 1 H), 7.14 (d, 1 H), 7.32 (t, 1 H), 7.54 (d, 1 H), 8.28 (d, 1 H), 9.74 (s, 1 H).

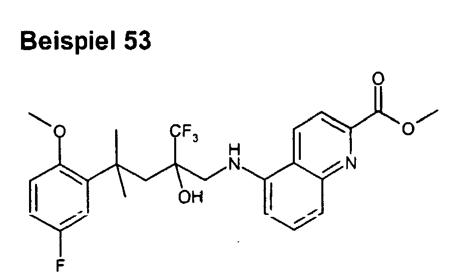

### 5-[4-(5-Fluor-2-methoxyphenly)-2-hydroxy-4-methyl-2-(trifluormethyl)pentylamino]chinolin-2-carbonsäuremethyleste

### 5-Aminochinolin-2-carbonsäuremethylester

Bei 0°C werden 2.8 g (mmol) Kaliumnitrat portionsweise zu einer Lösung aus 3.5 g (20.21 mmol) Chinolin-2-carbonsäure in 12 mL konz. Schwefelsäure hinzugefügt. Nach 4 Tagen bei Raumtemperatur wird das Reaktionsgemisch auf Eiswasser gegossen. Der ausgefallene Feststoff wird abgesaugt, mit wenig Wasser gewaschen und im Hochvakuum getrocknet. Das Rohprodukt wird in 50 mL Methanol gelöst. Nach Zugabe 10 mL konz. Schwefelsäure lässt man die Reaktionslösung 4 Stunden refluxieren, anschließend 36 Stunden bei Raumtemperatur rühren. Die Reaktionslösung wird auf ein Drittel des ursprünglichen Volumens eingeengt und auf Eiswasser gegeben. Man extrahiert mit Ethylacetat, wäscht die vereinigten organischen Phasen mit gesättigter Natriumhydrogencarbonatlösung, dann mit gesättigter Natriumchloridlösung, trocknet sie über Natriumsulfat und entfernt das Lösungsmittel im Vakuum.
Das Rohprodukt wird in 80 mL Methanol-Aceton (50%) gelöst und mit Palladium auf Kohle und Kaliumcarbonat versetzt. Es wird 20 Stunden unter Wasserstoffatmosphäre bei Raumtemperatur gerührt. Nach Absaugen über Celite und Waschen mit Aceton entfernt man das Lösungsmittel im Vakuum und reinigt den Rückstand an Kieselgel mit Hexan-Ethylacetat (0-60%). Man erhält 850 mg (27% der Theorie) des Produkts.
¹H-NMR (300 MHz, CDCl₃): δ = 4.07 (s, 3H), 6.90 (d, 1 H), 7.58 (t, 1 H), 7.75 (d, 1 H), 8.12 (d, 1 H), 8.33 (d, 1 H).
*5-[4-(5-Fluor-2-methoxyphenyl)-2-hydroxy-4-methyl-2-(trifluormethyl)pentylidenamino]-chinolin-2-carbonsäuremethylester*
Analog zu Beispiel 37 werden 200 mg (1.0 mmol) 5-Aminochinolin-2-carbonsäuremethylester mit 371 mg (1.2 mmol) 4-(5-Fluor-2-methoxyphenyl)-2-hydroxy-4-methyl-2-(trifluormethyl)pentanal und 1.10 mL konz. Essigsäure in 20 mL Toluol umgesetzt. Nach Chromatographie an Kieselgel mit Hexan-Ethylacetat (0-100%) erhält man 420 mg (85% der Theorie) des Produkts. ¹H-NMR (300 MHz, CDCl₃): δ = 1.38 (s, 3H), 1.59 (s, 3H), 2.29 (d, 1 H), 3.47 (d, 1 H), 3.80 (s, 3H), 4.10 (s, 3H), 4.79 (s, 1 H), 6.40-6.45 (m, 1 H), 6.53-6.56 (m, 2H), 6.81 (dd, 1 H), 7.54 (s, 1 H), 7.60 (t, 1 H), 8.18 (d, 1 H), 8.23 (d, 1 H), 8.45 (d, 1 H).
*5-(4-(5-Fluor-2-methoxyphenyl)-2-hydroxy-4-methyl-2-(trifluormethyl)pentylamino]chinolin-2-carbonsäuremethylester*
Analog zu Beispiel 40 werden 420 mg (0.85 mmol) 5-[4-(5-Fluor-2-methoxyphenyl)-2-hydroxy-4-methyl-2-(trifluormethyl)pentylidenamino]chinolin-2-carbonsäuremethylester in 10 mL Methanol gelöst und mit 130 mg (3.41 mmol) Natriumborhydrid versetzt. Nach Chromatographie an Kieselgel mit Hexan-Ethylacetat (0-100%) erhält man 75 mg (18% der Theorie) des Produkts. ¹H-NMR (300 MHz, CDCl₃): δ = 1.46 (s, 3H), 1.56 (s, 3H), 2.36 (d, 1H), 2.78 (d, 1 H), 3.04 (s, 1 H), 3.15 (dd, 1 H), 3.50 (dd, 1 H), 3.84 (s, 3H), 4.08 (s, 3H), 4.35 (dd, 1 H), 6.16 (d, 1 H), 6.77 (dd, 1 H), 6.89-6.95 (m, 1 H), 7.10 (dd, 1 H), 7.52 (t, 1 H), 7.71 (d, 1 H), 8.11 (s, 2H).

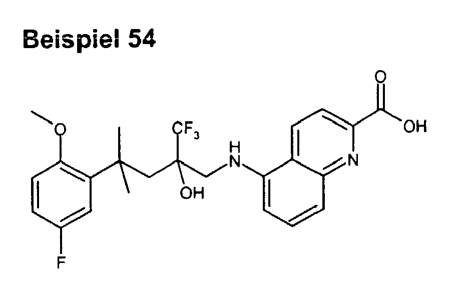

### 5-[4-(5-Fluor-2-methoxyphenyl)-2-hydroxy-4-methyl-2-(trifluormethyl)pentylaminolchinolin-2-carbonsäure

Eine Lösung aus 60 mg (0.12 mmol) 5-[4-(5-Fluor-2-methoxyphenyl)-2-hydroxy-4-methyl-2-(trifluormethyl)pentyamino]chinolin-2-carbonsäuremethylester in 10 mL Methanol wird mit 0.5 mL (0.5 mmol) 1 N-Natronlauge versetzt. Nach 2.5 Stunden entfernt man das Lösungsmittel im Vakuum und reinigt den Rückstand an Kieselgel mit Dichlormethan-Methanol (6-25%). Es werden 47 mg (82% der Theorie) erhalten.
¹H-NMR (300 MHz, DMSO-d₆): δ = 1.38 (s, 3H), 1.54 (s, 3H), 2.04 (d, 1 H), 2.91-3.06 (m, 3H), 3.75 (s, 3H), 5.49 (br, 1 H), 5.94 (d, 1 H), 6.11 (br, 1 H), 6.83-6.88 (m, 1 H), 6.93-6.97 (m, 1 H), 7.07 (dd, 1 H), 7.34 (t, 1 H), 7.44 (d, 1 H), 8.00 (d, 1 H). 8.38 (d, 1 H).

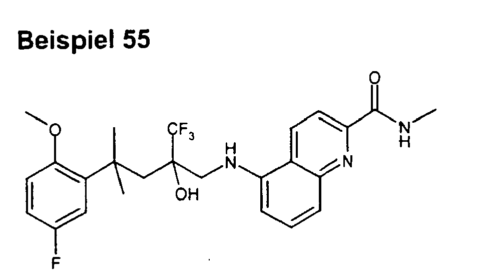

### 5-[4-(5-Fluor-2-methoxyphenyl)-2-hydroxy-4-methyl-2-trifluormethylpentylamino]-chinolin-2-carbonsäuremethylamid

### 5-Aminochinolin-2-carbonsäuremethylamid

76 mg (0.376 mmol) 5-Aminochinolin-2-carbonsäuremethylester werden mit 10 mL einer 2.0 M methanolischer Methylaminlösung gelöst. Nach 90 Minuten unter Rückfluss und weiteren 16 Stunden bei Raumtemperatur entfernt man das Lösungsmittel unter reduziertem Druck und reinigt den Rückstand an Kieselgel mit Hexan-Ethylacetat (0-80%). Es werden 72 mg (95% der Theorie) des Produkts erhalten.
¹H-NMR (300 MHz, DMSO-d₆): δ = 2.87 (d, 3H), 6.10 (s, 2H), 6.77 (d, 1 H), 7.27 (d, 1 H), 7.51 (t, 1 H), 7.95 (d, 1 H), 8.68 (d, 1H), 8.79-8.80 (m, 1 H).
*5-[4-(5-Fluor-2-methoxyphenyl)-2-hydroxy-4-methyl-2-(trifluormethyl)-pentylidenaminoJ-chinolin-2-carbonsäuremethylamid*
Analog zu Beispiel 37 werden 220 mg (1.08 mmol) 5-Aminochinolin-2-carbonsäure-methylamid mit 550 mg (1.78 mmol) 4-(5-Fluor-2-methoxyphenyl)-2-hydroxy-4-methyl-2-(trifluormethyl)pentanal und 3.0 mL konz. Essigsäure in 30 mL Toluol umgesetzt. Nach Chromatographie an Kieselgel mit Hexan-Ethylacetat (0-70%) erhält man 259 mg (48% der Theorie) des Produkts. ¹H-NMR (300 MHz, CDCl₃): δ = 1.37 (s, 3H), 1.56 (s, 3H), 2.29 (d, 1H), 3.11 (d, 3H), 3.48 (d, 1 H), 3.81 (s, 3H), 4.81 (s, 1H), 6.46-6.49 (m, 2H), 6.57 (dd, 1H), 6.78 (dd, 1 H), 7.50 (s, 1 H), 7.56 (t, 1 H), 7.95 (d, 1 H), 8.23 (br, 1 H), 8.34 (d, 1H), 8.46 (d, 1H).
*5-[4-(5-Fluor 2-methoxyphenyl)-2-hydroxy-4-methyl-2-(trifluormethyl)pentylamino]-chinolin-2-carbonsäuremethylamid*
155 mg (0.315 mmol) 5-[4-(5-Fluor-2-methoxyphenyl)-2-hydroxy-4-methyl-2-(trifluormethyl)pentylidenamino]chinolin-2-carbonsäuremethylamid werden in 10 mL Ethanol gelöst und mit 0.09 mL (0.4 mmol) Titantetraethylat sowie 94 mg (2.6 mmol) Natriumborhydrid versetzt. Der Abbruch der Reaktion erfolgt nach 5 Stunden durch Zugabe von gesättigter Natriumchloridlösung und Ethylacetat. Unlösliche Bestandteile werden filtriert. Man extrahiert das Filtrat mit Ethylacetat, trocknet die vereinigten organischen Phasen über Natriumsulfat und entfernt das Lösungsmittel im Vakuum. Nach Chromatographie an Kieselgel mit Hexan-Ethylacetat (0-50%) erhält man 110 mg (71% der Theorie) des Produkts. ¹H-NMR (300 MHz, DMSO-d₆): δ = 1.38 (s, 3H), 1.56 (s, 3H), 2.06 (d, 1 H), 2.86-2.97 (m, 5H), 3.07 (dd, 1 H), 3.78 (s, 3H), 5.98 (s, 1 H), 6.03 (d, 1 H), 6.86-7.05 (m, 2H), 7.07 (dd, 1 H), 7.32 (d, 1H), 7.46 (t, 1 H), 8.03 (d, 1 H), 8.49 (d, 1 H), 8.81 (q, 1H).

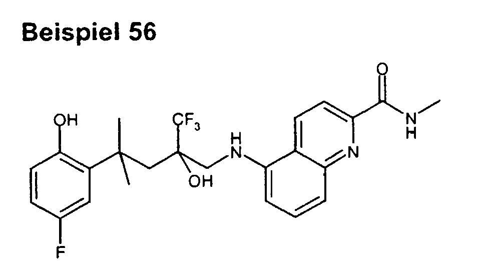

### 5-[4-(5-Fluor-2-hvdroxyhenyl)-2-hydroxy-4-methyl-2-(trifluormethyl)pentylaminol-chinolin-2-carbonsäuremethylamid

Analog zu Beispiel 38 werden 97 mg (0.197 mmol) 5-[4-(5-Fluor-2-methoxyphenyl)-2-hydroxy-4-methyl-2-(trifluormethyl)pentylamino]chinolin-2-carbonsäuremethylamid mit 8.0 mL (8.0 mmol) einer 1 M-Bortribromid-Lösung umgesetzt. Nach Aufarbeitung und Reinigung an Kieselgel mit Hexan-Ethylacetat (0-70%) erhält man 18 mg (18% der Theorie) des Produkts.
¹H-NMR (300 MHz, DMSO-d₆): δ = 1.40 (s, 3H), 1.57 (s, 3H), 1.93 (d, 1 H), 2.87 (d, 3H), 2.97 (dd, 1 H), 3.13-3.20 (m, 2H), 5.51-5.54 (m, 1 H), 5.98 (s, 1 H), 6.04 (d, 1 H), 6.67 (dd, 1 H), 6.76-6.83 (m, 1 H), 7.00 (dd, 1 H), 7.32 (d, 1H), 7.45 (t, 1H), 8.03 (d, 1H), 8.46 (d, 1H); 8.80-8.82 (m, 1H), 9.72 (br, 1H).

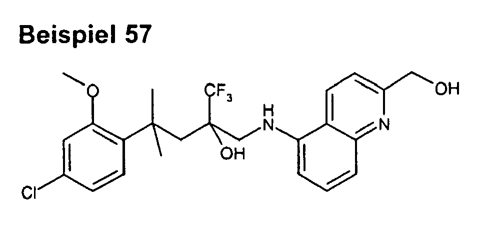

### 4-(4-Chlor-2-methoxyphenyl)-1-(2-(hydroxymethyl)chinolin-5-ylamino)-4-methyl-2-(trifluormethyl)pentan-2-ol

### 5-[4-(4-Chlor-2-methoxyphenyl)-2-hydroxy-4-methyl-2-(trifluormethyl)pentylidenamino]chinolin-2-carbonsäuremethylester

Analog zu Beispiel 1 werden 250 mg (1.24 mmol) 5-Aminochinolin-2-carbonsäuremethylester mit 484 mg (1.49 mmol) 4-(4-Chlor-2-methoxyphenyl)-2-hydroxy-4-methyl-2-(trifluormethyl)pentanal und 1.40 mL konz. Essigsäure in 20 mL Toluol umgesetzt. Nach Chromatographie an Kieselgel mit Hexan-Ethylacetat (0-100%) erhält man 500 mg (79% der Theorie) des Produkts.
¹H-NMR (300 MHz, CDCl₃): δ = 1.36 (s, 3H), 1.58 (s, 3H), 2.28 (d, 1H), 3.45 (d, 1 H), 3.84 (s, 3H), 4.11 (s, 3H), 4.79 (s, 1 H), 6.38 (d, 1 H), 6.48 (dd, 1 H), 6.67 (d, 1 H), 7.00 (d, 1 H), 7.52 (s, 1 H), 7.65 (dd, 1 H), 8.18-8.25 (m, 2H), 8.48 (d, 1 H). *4-(4-Chlor-2-methoxyphenyl)-1-(2-(hydroxymethyl)chinolin-5-ylamino)-4-methyl-2-(trifluormethyl)pentan-2-ol*
Analog zu Beispiel 40 werden 200 mg (0.39 mmol) 5-[4-(4-Chlor-2-methoxyphenyl)-2-hydroxy-4-methyl-2-(trifluormethyl)pentylidenamino]chinolin-2-carbonsäuremethylester in 5.0 mL Methanol gelöst und mit 60 mg (1.56 mmol) Natriumborhydrid versetzt. Nach Chromatographie an Kieselgel mit Hexan-Ethylacetat (0-100%) erhält man 70 mg (37% der Theorie) des Produkts.
¹H-NMR (300 MHz, DMSO-d₆): δ = 1.37 (s, 3H), 1.55 (s, 3H), 2.04 (d, 1 H), 2.82-2.91 (m, 2H), 2.98-3.02 (m, 1 H), 3.81 (s, 3H), 4.67 (d, 2H), 5.39-5.42 (m, 1H), 5.49 (t, 1 H), 5.87 (d, 1 H), 5.95 (s, 1 H), 6.95 (dd, 1 H), 7.01 (d, 1 H), 7.17 (d, 1H), 7.29-7.37 (m, 2H), 7.53-7.55 (d, 1 H), 8.29 (d, 1 H).

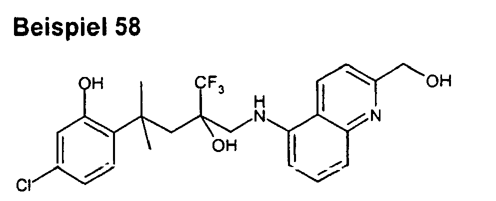

### 4-(4-Chlor-2-hydroxyphenyl)-1-(2-(hydroxymethyl)chinolin-5-ylamino)-4-methyl-2-(trifluormethyl)pentan-2-ol

Analog zu Beispiel 38 werden 110 mg (0.23 mmol) 4-(4-Chlor-2-methoxyphenyl)-1-(2-(hydroxymethyl)chinolin-5-ylamino)-4-methyl-2-(trifluormethyl)pentan-2-ol mit 4.60 mL (4.60 mmol) einer 1 M-Bortribromid-Lösung umgesetzt. Nach Aufarbeitung und Reinigung an Kieselgel mit Hexan-Ethylacetat (0-100%) sowie mit Ethylacetat-Methanol (0-20%) erhält man 54 mg (50% der Theorie) des Produkts.
¹H-NMR (300 MHz, DMSO-d₆): δ = 1.39 (s, 3H), 1.57 (s, 3H), 1.94 (d, 1 H), 2.91 (dd, 1 H), 3.04-3.13 (m, 2H), 4.67 (d, 2H), 5.34-5.37 (m, 1 H), 5.50 (t, 1 H), 5.87 (d, 1 H), 5.97 (s, 1 H), 6.80-6.83 (m, 2H), 7.16 (d, 1 H), 7.34 (t, 1 H), 7.53 (d, 1 H), 8.27 (d, 1 H), 10.20 (s, 1 H).

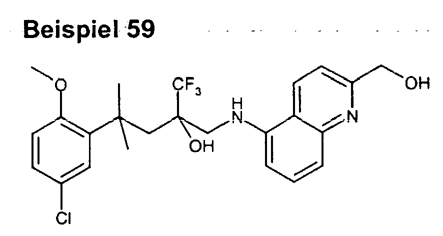

### 4-(5-Chlor-2-methoxyphenyl)-1-(2-(hydroxymethyl)chinolin-5-ylamino)-4-methyl-2-(trifluormethyl)pentan-2-ol

### 5-[4-(5-Chlor-2-methoxyphenyl)-2-hydroxy-4-methyl-2-(trifluormethyl)pentylidenamino]chinolin-2-carbonsäuremethylester

Man löst 224 mg (0.69 mmol) 4-(5-Chlor-2-methoxyphenyl)-2-hydroxy-4-methyl-2-(trifluormethyl)pentanal und 195 mg (0.96 mmol) 5-Aminochinolin-2-carbonsäuremethylester in 20 mL Toluol. Nach Zugabe von 5.0 mL konzentrierter Essigsäure wird 16 Stunden am Wasserabscheider refluxiert. Nach Entfernen des Lösungsmittels nimmt man den Rückstand in 10 mL Tetrahydrofuran auf, fügt 0.3 mL (1.4 mmol) Titantetraethylat hinzu und lässt die Reaktionsmischung erneut refluxieren. Nach Entfernen des Lösungsmittels sowie Reinigung an Kieselgel mit Hexan-Ethylacetat (0-100%) erhält man 120 mg (33% der Theorie) des Produkts.
¹H-NMR (300 MHz, CDCl₃): δ = 1.37 (s, 3H), 1.51 (t, 3H), 1.57 (s, 3H), 2.99 (d, 1 H). 3.49 (d, 1H), 3,81 (s, 3H). 4.57 (q, 2H), 4.83 (s, 1 H), 6.52-6.58 (m, 1H). 6.79 (dd, 1 H), 7.02 (d, 1 H), 7.51 (s, 1 H), 7.61 (t, 1 H), 8.18-8.26 (m, 2H), 8.46 (d, 1 H).
*4-(5-Chlor-2-methoxyphenyl)-1-(2-(hydroxymethyl)chinolin-5-ylamino)-4-methyl-2-(trifluormethyl)pentan-2-ol*
Analog zu Beispiel 40 werden 120 mg (0.23 mmol) 5-[4-(5-Chlor-2-methoxyphenyl)-2-hydroxy-4-methyl-2-trifluormethylpentylidenamino]-chinoline-2-carbonsäureethylester mit 35 mg (0.92 mmol) Natriumborhydrid umgesetzt. Nach Aufarbeitung und Reinigung an Kieselgel mit Hexan-Ethylacetat (0-100%) sowie mit Ethylacetat-Methanol (0-20%) erhält man 61 mg (55% der Theorie) des Produkts
'H-NMR (300 MHz, CDCl₃): δ = 1.47 (s, 3H), 1.59 (s, 3H), 2.29 (d, 1 H), 2.82 (d, 1 H), 3.05-3.16 (m, 2H), 3.29-3.36 (m, 1 H), 4.23-4.26 (m, 1 H), 4.32-4.52 (br, 1H), 4.88 (s, 2H)), 6.04-6.06 (m, 1H), 6.79 (d, 1H), 7.18-7.21 (m, 2H), 7.36 (d, 1H), 7.48-7.50 (m, 2H), 7.95 (d, 1H).

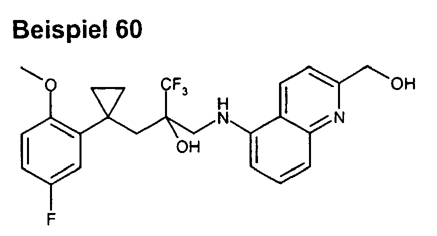

### 3-[1-(5-Fluor-2-methoxyphenyl)cycloprop-1-yl]-1-(2-(hydroxymethyl)chinolin-5-ylamino)-2-(trifluormethyl)propan-2-ol

### 5-{3-[1-(5-Fluor-2-methoxyphenyl)cycloprop-1-yl]-2-hydroxy-2-(trifluormethyl)propylidenamino}chinolin-2-carbonsäuremethylester

Analog zu Beispiel 37 werden 306 mg (1.2 mmol) 5-{3-[1-(5-Fluor-2-methoxyphenyl)cycloprop-1-yl]-2-hydroxy-2-(trifluormethyl)propanal mit 170 mg (0.84 mmol) 5-Aminochinolin-2-carbonsäuremethylester in 4.0 mL konzentrierter Essigsäure und 20 mL Toluol umgesetzt. Nach Aufarbeitung und Reinigung an Kieselgel mit Hexan-Ethylacetat (0-70%) erhält man 204 mg (49% der Theorie) des Produkts.
¹H-NMR (300 MHz, CDCl₃): δ = 0.61-0.68 (m, 1H), 0.76-0.80 (m, 1 H), 0.94-1.00 (m, 1H), 1.06-1.12 (m, 1H), 2.16 (d, 1H), 2.90 (d, 1H), 3.84 (s, 3H), 4.10 (s, 3H), 4.82 (s, 1 H), 6.58 (d, 1 H), 6.63-6.66 (m, 2H), 6.75 (d, 1 H), 7.63 (t, 1 H), 7.74 (s, 1 H), 8.19-8.26 (m, 2H), 8.60 (d, 1 H).
*3-[1-(5-Fluor-2-methoxyphenyl)cycloprop-1-yl]-1-(2-(hydroxymethyl)chinolin-5-ylamino)-2-(trifluormethyl)propan-2-ol*
Analog zu Beispiel 40 werden 200 mg (0.41 mmol) 5-{3-[1-(5-Fluor-2-methoxyphenyl)cycloprop-1-yl]-2-hydroxy-2-
(trifluormethyl)propylidenamino}chinolin-2-carbonsäuremethylester mit 156 mg (4.1 mmol) Natriumborhydrid umgesetzt. Nach Aufarbeitung und Reinigung an Kieselgel mit Hexan-Ethylacetat (0-75%) erhält man 119 mg (63% der Theorie) des Produkts
¹H-NMR (300 MHz, DMSO-d₆): δ = 0.58-0.61 (m, 1 H), 0.78-0.91 (m, 3H), 1.90 (d, 1 H), 2.65 (d, 1 H), 3.17-3.18 (m, 2H), 3.61 (s, 3H), 4.68 (d, 2H), 5.31 (t, 1 H), 5.51 (t, 1 H), 5.97 (s, 1 H), 6.13 (d, 1H), 6.61 (dd, 1 H), 6.85 (td, 1H), 7.01 (dd, 1 H), 7.17 (d, 1 H), 7.38 (t, 1 H), 7.54 (d, 1 H), 8.37 (d, 1 H).

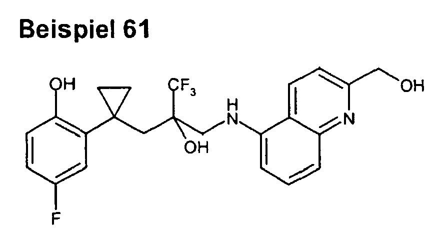

### 3-[1-(5-Fluor-2-hydroxyphenyl)cycloprop-1-yl]-1-(2-(hydroxymethyl)chinolin-5-ylamino)-2-(trifluormethyl)propan-2-ol

Analog zu Beispiel 38 werden 100 mg (0.22 mmol) 3-[1-(5-Fluor-2-methoxyphenyl)cycloprop-1-yl]-1-(2-(hydroxymethyl)chinolin-5-ylamino)-2-(trifluormethyl)propan-2-ol mit 4.40 mL (4.40 mmol) einer 1 M-Bortribromid-Lösung umgesetzt. Nach Aufarbeitung und Reinigung an Kieselgel mit Hexan-Ethylacetat (0-100%) sowie mit Ethylacetat-Methanol (0-20%) erhält man 50 mg (50% der Theorie) des Produkts.
¹H-NMR (300 MHz, DMSO-d₆): δ = 0.66-0.73 (m, 1 H), 0.83-0.88 (m, 3H), 2.04 (d, 1 H), 2.54 (d, 1 H), 3.24-3.26 (m, 2H), 4.68 (d, 2H), 5.32 (t, 1 H), 5.51 (t, 1H), 5.95 (s, 1H), 6.23 (d, 1H), 6.60 (d, 1H), 6.73-6.79 (m, 1H), 6.94 (dd, 1H), 7.16 (d, 1H), 7.39 (t, 1H), 7.54 (d, 1H), 8.33 (d, 1H), 9.49 (s, 1H).

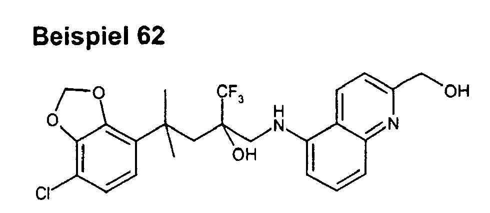

### 4-(7-Chlorbenzo[1,3]dioxol-4-yl)-2-(2-(hydroxymethyl)chinolin-5-ylamino)-4-methyl-2-(trifluormethyl)pentan-2-ol

### 5-[4-(7-Chlorbenzo[1,3]dioxol-4-yl)-2-hydroxy-4-methyl-2-(trifluormethyl)pentylidenamino]chinolin-2-carbonsäuremethylester

Analog zu Beispiel 37 werden 70 mg (0.21 mmol) 4-(7-Chlorbenzo[1,3]dioxol-4-yl)-2-hydroxy-4-methyl-2-(trifluormethyl)pentanal mit 50 mg (0.25 mmol) 5-Aminochinolin-2-carbonsäuremethylester in 0.5 mL konzentrierter Essigsäure und 20 mL Toluol umgesetzt. Nach Aufarbeitung und Reinigung an Kieselgel mit Hexan-Ethylacetat (0-100%) erhält man 60 mg (55% der Theorie) des Produkts.
¹H-NMR (300 MHz, CDCl₃): δ = 1.37 (s, 3H), 1.52 (s, 3H), 2.34 (d, 1H), 3.08 (d, 1 H), 4.11 (s, 3H), 4.83 (s, 1 H), 5.94 (s, 1 H), 6.02 (s, 1 H), 6.37 (d, 1 H), 6.54 (t, 1 H), 7.58-7.70 (m, 2H), 8.20-8.26 (m, 2H), 8.57 (d, 1 H).
*4-(7-Chlorbenzo[1,3]dioxol-4-yl)-2-(2-(hydroxymethyl)chinolin-5-ylamino)-4-methyl-2-(trifluormethyl)pentan-2-ol*
Analog zu Beispiel 40 werden 60 mg (0.11 mmol) 5-[4-(7-Chlorbenzo[1,3]dioxol-4-yl)-2-hydroxy-4-methyl-2-(trifluormethyl)pentylidenamino]chinolin-2-carbonsäuremethylester mit 18 mg (0.46 mmol) Natriumborhydrid umgesetzt. Nach Aufarbeitung und Reinigung an Kieselgel mit Hexan-Ethylacetat (0-100%) sowie mit Ethylacetat-Methanol (0-20%) erhält man 21 mg (39% der Theorie) des Produkts.
¹H-NMR (300 MHz, DMSO-d₆): δ = 1.37 (s, 3H), 1.53 (s, 3H), 2.11 (d, 1 H), 2.55 (d, 1 H), 3.01-3.19 (m, 2H), 4.67 (d, 2H), 5.42-5.46 (m, 1 H), 5.50 (t, 1 H), 5.98-6.08 (m, 4H), 6.86 (s, 2H), 7.18 (d, 1 H), 7.36 (t, 1 H), 7.55 (d, 1 H), 8.32 (d, 1 H).

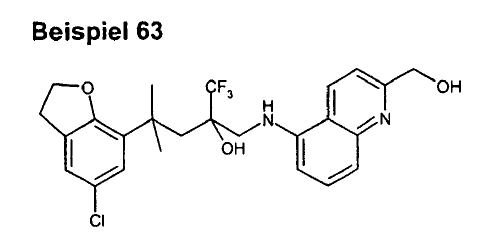

### 4-(5-Chlor-2,3-dihydrobenzofuran-7-yl)-2-(2-(hydroxymethyl)chinolin-5-ylamino)-4-methyl-2-(trifluormethyl)pentan-2-ol

### 5-[4-(5-Chlor-2,3-dihydrobenzofuran-7-yl)-2-hydroxy-4-methyl-2-(trifluormethyl)pentylidenamino]chinolin-2-carbonsäuremethylester

Analog zu Beispiel 37 werden 160 mg (0.47 mmol) 4-(5-Chlor-2,3-dihydrobenzofuran-7-yl)-2-hydroxy-4-methyl-2-(trifluormethyl)pentanal mit 116 mg (0.57 mmol) 5-Aminochinolin-2-carbonsäuremethylester in 1.0 mL konzentrierter Essigsäure und 20 mL Toluol umgesetzt. Nach Aufarbeitung und Reinigung an Kieselgel mit Hexan-Ethylacetat (0-100%) erhält man 110 mg (45% der Theorie) des Produkts.
¹H-NMR (300 MHz, CDCl₃): δ = 1.34 (s, 3H), 1.55 (s, 3H), 2.27 (d, 1H), 2.65-2.73 (m, 1 H), 2.91-3.01 (m, 1 H), 3.35 (d, 1 H), 4.11 (s, 3H), 4.42-4.58 (m, 2H), 4.79 (s, 1 H), 6.58 (s, 1 H), 6.65 (d, 1 H), 6.82 (d, 1 H), 7.61-7.67 (m, 2H), 8.21 (d, 1 H), 8.27 (d, 1H), 8.47 (d, 1H).
*4-(5-Chlor-2,3-dihydrobenzofuran-7-yl)-2-(2-(hydroxymethyl)chinolin-5-ylamino)-4-methyl-2-(trifluormethyl)pentan-2-ol*
Analog zu Beispiel 40 werden 110 mg (0.21 mmol) 5-[4-(5-Chlor-2,3-dihydrobenzofuran-7-yl)-2-hydroxy-4-methyl-2-(trifluormethyl)pentylidenamino]chinolin-2-carbonsäuremethylester mit 32 mg (0.84 mmol) Natriumborhydrid umgesetzt. Nach Aufarbeitung und Reinigung an Kieselgel mit Hexan-Ethylacetat (0-100%) sowie mit Ethylacetat-Methanol (0-30%) erhält man 68 mg (65% der Theorie) des Produkts.
¹H-NMR (300 MHz, DMSO-d₆): δ =1.34 (s. 3H), 1.53 (s, 3H),2,03 (d, 1H), 2.77 (d, 1 H), 2.85-3.12 (m, 4H), 5.32-5.36 (m, 1 H), 5.51 (t, 1H), 5.96 (d, 1 H), 6.02 (s, 1 H), 7.03 (s, 1 H), 7.07 (s, 1 H), 7.18 (d, 1 H), 7.37 (t, 1 H), 7.54 (d, 1 H), 8.29 (d, 1 H).

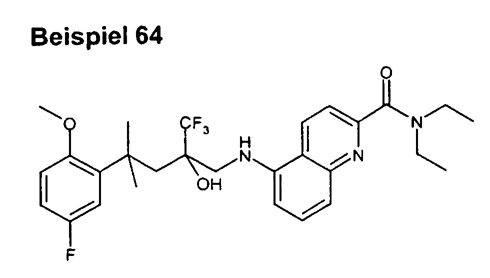

### 5-F4-(5-Fluor-2-methoxyphenyl)-2-hydroxy-4-methyl-2-(trifluormethyl)pentylamino]chinolin-2-carbonsäurediethylamid 5-Aminochinolin-2-carbonsäurediethylamid

Eine Suspension aus 1.06 g (7.95 mmol) Aluminiumchlorid in 35 mL Toluol wird unter Eiskühlung mit 1.70 mL (15.7 mmol) Diethylamin versetzt. Nach einer Stunde bei Raumtemperatur fügt man 350 mg (1.73 mmol) 5-Aminochinolin-2-carbonsäuremethylester hinzu und lässt die Reaktionsmischung 5 Stunden bei 40°C rühren. Der Abbruch der Reaktion erfolgt durch Zugabe von Wasser. Man extrahiert mit Dichlormethan und trocknet die vereinigten organischen Phasen über Natriumsulfat. Nach Entfernen des Lösungsmittels und Reinigung an Kieselgel mit Dichlormethan-Methanol (0-10%) erhält man 210 mg (50% der Theorie) des Produkts.
¹H-NMR (300 MHz, DMSO-d₆): δ = 1.11 (t, 3H), 1.19 (t, 3H), 3.27 (q; 2H), 3.48 (q, 2H), 6.06 (s, 2H), 6.74 (d, 1 H), 7.17 (d, 1 H), 7.42-7.47 (m, 2H), 8.61 (d, 1 H).
*5-(4-(5-Fluor-2-methoxyphenyl)-2-hydroxy-4-methyl-2-(trifluormethyl)pentylidenamino]chinolin-2-carbonsäurediethylamid*
Eine Lösung aus 210 mg (0.86 mmol) 5-Aminochinolin-2-carbonsäurediethylamid, 266 mg (0.86 mmol) 4-(5-Fluor-2-methoxyphenyl)-2-hydroxy-4-methyl-2-(trifluormethyl)pentanal und 0.36 mL (1.73 mmol) Titantetraethylat in 15 mL Tetrahydrofuran wird eine Stunde bei Raumtemperatur, anschließend 3 Stunden bei 80°C gerührt. Nach Entfernen des Lösungsmittels und Reinigung an Kieselgel mit Hexan-Ethylacetat (0-70%) erhält man 230 mg (52% der Theorie) des Produkts.
¹H-NMR (300 MHz, CDCl₃): δ = 1.23 (t, 3H), 1.33 (t, 3H), 1.37 (s, 3H), 1.57 (s, 3H), 3.38-3.50 (m, 3H), 3.63 (q, 2H), 3.81 (s, 3H), 4.82 (s, 1 H), 6.43-6.50 (m, 2H), 6.56 (dd, 1 H), 6.79 (dd, 1 H), 7.50 (s, 1 H), 7.55 (t, 1 H), 7.68 (d, 1 H), 7.99 (d, 1 H), 8.39 (d, 1 H).
*5-[4-(5-Fluor-2-methoxyphenyl)-2-hydroxy-4-methyl-2-(trifluormethyl)pentylamino]chinolin-2-carbonsäurediethylamid*
Eine Lösung aus 230 mg (0.45 mmol) 5-[4-(5-Fluor-2-methoxyphenyl)-2-hydroxy-4-methyl-2-(trifluormethyl)pentylidenamino]chinolin-2-carbonsäurediethylamid und 0.132 mL (0.58 mmol) Titantetraethylat in 14 mL Ethanol wird mit 135 mg (3.55 mmol) Natriumborhydrid versetzt. Nach 6 Stunden bei Raumtemperatur erfolgt der Abbruch der Reaktion durch Zugabe gesättigter Natriumchloridlösung und Ethylacetat. Der entstandene Niederschlag wird über Celite abgesaugt. Das Filtrat wird mit gesättigter Natriumchloridlösung gewaschen und über Natriumsulfat getrocknet. Nach Entfernen des Lösungsmittels und Reinigung an Kieselgel mit Hexan-Ethylacetat (0-70%) erhält man 220 mg (92%) des Produkts.
¹H-NMR (300 MHz, CDCl₃): δ = 1.20 (t, 3H), 1.31 (t, 3H), 1.46 (s, 3H), 1.56 (s, 3H), 2.34 (d, 1 H), 2.78 (d, 1 H), 3.13 (dd, 1 H), 3.19 (s, 1 H), 3.31 (dd, 1 H), 3.41 (q, 2H), 3.60 (q, 2H), 3.84 (s, 3H), 4.33-4.37 (m, 1 H), 6.08 (d, 1 H), 6.79 (dd, 1 H), 6.89-6.96 (m, 1 H), 7.11 (dd, 1 H), 7.42-7.51 (m, 2H), 7.55 (d, 1 H), 8.03 (d, 1 H).

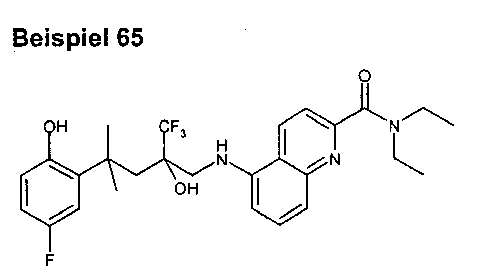

### 5-[4-(5-Fluor-2-hydroxyphenyl)-2-hydroxy-4-methyl-2-(trifluormethyl)pentylamino]-chinolin-2-carbonsäurediethylamid

210 mg (0.39 mmol) 5-[4-(5-Fluor-2-methoxyphenyl)-2-hydroxy-4-methyl-2-(trifluormethyl)pentylamino]chinolin-2-carbonsäurediethylamid werden mit 7.8 mL (7.8 mmol) einer 1 M-Bortribromidlösung in Dichlormethan versetzt. Nach 20 Stunden bei Raumtemperatur erfolgt der Abbruch der Reaktion durch Zugabe von Wasser. Man extrahiert mit Ethylacetat, wäscht die vereinigten organischen Phasen mit gesättigter Natriumhydrogencarbonatlösung und trocknet sie über Natriumsulfat. Nach Entfernen des Lösungsmittels und Reinigung an Kieselgel mit Hexan-Ethylacetat (0-100%) erhält man 56 mg (28%) des Produkts.

¹H-NMR (300 MHz, DMSO-d₆): δ = 1.10 (t, 3H), 1.19 (t, 3H), 1.40 (s, 3H), 1.57 (s, 3H), 1.93 (d, 1 H), 2.96 (dd, 1 H), 3.11-3.29 (m, 4H), 3.48 (q, 2H), 5.48-5.52 (m, 1 H), 5.97 (s, 1 H), 6.01 (d, 1 H), 6.70 (dd, 1 H), 6.79-6.85 (m, 1 H), 7.00 (dd, 1 H), 7.22 (d, 1 H), 7.41 (t, 1 H), 7.51 (d, 1H), 8.40 (d, 1 H), 9.73 (s, 1 H).

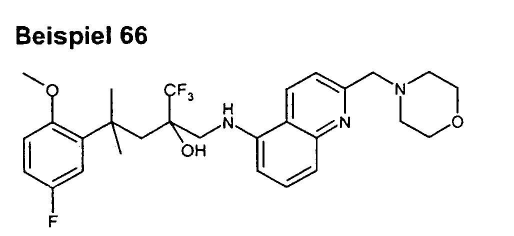

### 4-(5-Fluor-2-methoxyphenyl)-1-(2-(morpholin-4-ylmethyl)chinolin-5-ylamino)-4-methyl-2-(trifluormethyl)pentan-2-ol

### 2-Brommethyl-5-nitrochinolin

Eine Lösung aus 800 mg (4.25 mmol) 5-Nitro-2-methylchinolin in 20 ml Tetrachlormethan wird mit 11 mg (0.04 mmol) Benzoylperoxid und 794 mg (4.46 mmol) N-Bromsuccinimid versetzt. Man lässt die Reaktionsmischung 8 Stunden in Gegenwart von UV-Licht refluxieren. Unlösliche Bestandteile werden abfiltriert, und das Filtrat wird eingeengt. Nach Reinigung an Kieselgel mit Hexan-Ethylacetat (0-100%) erhält man 320 mg (28% der Theorie) des Produkts.
¹H-NMR (300 MHz, CDCl₃): δ = 4.72 (s, 2H), 7.79-7.82 (m, 2H), 8.35-8.39 (m, 2H), 9.02 (d, 1 H).
*2-(Morpholin-4-ylmethyl)-5-nitrochinolin*
460 mg (1.72 mmol) 2-Brommethyl-5-nitrochinolin und 0.54 mL (6.2 mmol) Morpholin werden in 150 mL Toluol gelöst. Nach Zugabe von 1.07 g (7.74 mmol) Kaliumcarbonat lässt man die Reaktionsmischung 2 Stunden refluxieren. Kaliumcarbonat wird abfiltriert, und das Filtrat wird eingeengt. Nach Reinigung an Kieselgel mit Hexan-Ethylacetat (0-100%) erhält man 190 mg (40% der Theorie) des Produkts.
¹H-NMR (300 MHz, CDCl₃): δ = 2.55-2.58 (m, 4H), 3.74-3.77 (m, 4H), 3.86 (s, 2H), 7.78 (t, 1 H), 7.87 (d, 1 H), 8.33-8.39 (m, 2H), 8.95 (d, 1 H).
*5-Amino-2-(morpholin-4-ylmethyl)chinolin*
190 mg (0.7 mmol) 2-(Morpholin-4-ylmethyl)-5-nitrochinolin werden in 10 mL Methanol gelöst. Nach Zugabe von 19 mg Palladium auf Kohle und 19 mg Kaliumcarbonat lässt man das Reaktionsgemisch 4 Stunden bei Raumtemperatur unter Wasserstoffatmosphäre rühren. Es wird anschließend über Celite filtriert und mit Ethylacetat gewaschen. Nach Entfernen des Lösungsmittels im Vakuum und Chromatographie an Kieselgel mit Hexan-Ethylacetat (0-100%) sowie mit Ethylacetat-Methanol (0-100%) werden 134 mg (79% der Theorie) des Produkts erhalten.
¹H-NMR (300 MHz, CDCl₃): δ = 2.55-2.57 (m, 4H), 3.73-3.76 (m, 4H), 3.82 (s, 2H), 4.18 (br, 2H), 6.79 (d, 1 H), 7.45-7.59 (m, 3H), 8.14 (d, 1 H).
*4-(5-Fluor-2-methoxyphenyl)-1-(2-(morpholin-4-ylmethyl)chinolin-5-ylimino)-4-methyl-2-(trifluormethyl)pentan-2-ol*
Analog zu Beispiel 37 werden 180 mg (0.74 mmol) 5-Amino-2-(morpholin-4-ylmethyl)chinolin mit 274 mg (0.89 mmol) 4-(5-Fluor-2-methoxyphenyl)-2-hydroxy-4-methyl-2-(trifluormethyl)pentanal in 0.8 mL konzentrierter Essigsäure und 20 mL Toluol umgesetzt. Nach Reinigung an Kieselgel mit Hexan-Ethylacetat (0-100%) erhält man 220 mg (56% der Theorie) des Produkts.
¹H-NMR (300 MHz, CDCl₃): δ = 1.37 (s, 3H), 1.57 (s, 3H), 2.29 (d, 1H), 2.55-2.58 (m, 4H), 3.46 (d, 1 H), 3.75-3.77 (m, 4H), 3.80 (s, 3H), 3.85 (s, 2H), 4.88 (s, 1 H), 6.38-6.45 (m, 2H), 6.53 (dd, 1 H), 6.79 (dd, 1 H), 7.47-7.52 (m, 2H), 7.67 (d, 1 H), 7.94 (d, 1 H), 8.28 (d, 1 H).
*4-(5-Fluor-2-methoxyphenyl)-9-(2-(morpholin-4-ylmethyl)chinolin-5-ylamino)-4-methyl-2-(frifluormethyl)pentan-2-ol*
Analog zu Beispiel 40 werden 220 mg (0.41 mmol) 4-(5-Fluor-2-methoxyphenyl)-1-(2-(morpholin-4-ylmethyl)chinolin-5-ylamino)-4-methyl-2-(trifluormethyl)pentan-2-ol in 10 mL Methanol gelöst und mit 315 mg (8.25 mmol) Natriumborhydrid versetzt. Nach Aufarbeitung und Reinigung erhält man 109 mg (50% der Theorie) des Produkts.
¹H-NMR (300 MHz, CDCl₃): δ = 1.46 (s, 3H), 1.56 (s, 3H), 2.34 (d, 1H), 2.52-2.55 (m, 4H), 2.77 (d, 1 H), 3.09-3.16 (m, 2H), 3.31 (dd, 1 H), 3.72-3.75 (m, 4H), 3.81 (s, 2H), 3.85 (s, 3H), 4.26-4,29 (m, 1 H), 6.05 (d, 1 H), 6.81 (dd, 1 H), 6.91-6.98 (m, 1 H), 7.11 (dd, 1 H), 7.39-7.56 (m, 3H), 7.94 (d, 1 H).

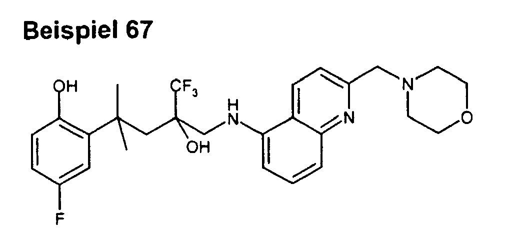

### 4-(5-Fluor-2-hydroxyphenyl)-1-(2-(morpholin-4-ylmethyl)chinolin-5-ylamino)-4-methyl-2-(trifluormethyl)pentan-2-ol

Analog zu Beispiel 38 werden 80 mg (0.15 mmol) 4-(5-Fluor-2-methoxyphenyl)-1-(2-(morpholin-4-ylmethyl)chinolin-5-ylamino)-4-methyl-2-(trifluormethyl)pentan-2-ol werden mit 3.0 mL (3.0 mmol) einer 1M-Bortribromidlösung in Dichlormethan versetzt. Nach Aufarbeitung und Reinigung an Kieselgel mit Hexan-Ethylacetat (0-100%) sowie Ethylacetat-Methanol (0-20%) erhält man 53 mg (68% der Theorie) des Produkts.
¹H-NMR (300 MHz, DMSO-d₆): δ = 1.40 (s, 3H), 1.56 (s, 3H), 2.40-2.43 (m, 4H), 2.93 (dd, 1 H), 3.09-3.20 (m, 2H), 3.58-3.61 (m, 4H), 3.69 (s, 2H), 5.33-5.37 (m, 1 H), 5.93 (d, 1H), 5.96 (br, 1 H), 6.72 (dd, 1 H), 6.80-6.86 (m, 1 H), 6.99 (dd, 1 H), 7.17 (d, 1 H), 7.32 (t, 1 H), 7.51 (d, 1 H), 8.24 (d, 1 H), 9.74 (br, 1 H).

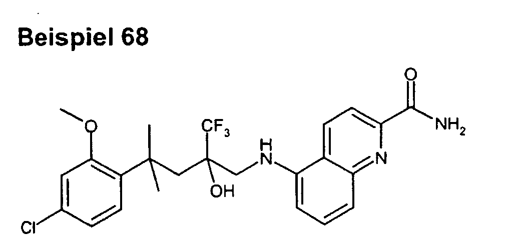

### 5-[4-(4-Chlor-2-methoxyphenyl)-2-hydroxy-4-methyl-2-(trifluomethyl)pentylamino]-chinolin-2-carbonsäureamid

### 5-[4-(4-Chlor-2-methoxyphenyl)-2-hydroxy-4-methyl-2-(trifluomethyl)pentylamino]-chinolin-2-carbonsäureamid

Analog zu Beispiel 37 werden 160 mg (0.87 mmol) 5- Aminochinolin-2-carbonsäureamid mit 341 mg (1.05 mmol) 4-(4-Chlor-2-methoxyphenyl)-2-hydroxy-4-methyl-2-(trifluomethyl)pentanal in 0.96 mL konzentrierter Essigsäure und 20 mL Toluol umgesetzt. Nach Reinigung an Kieselgel mit Hexan-Ethylacetat (0-100%) erhält man 340 mg (79% der Theorie) des Produkts.
¹H-NMR (300 MHz, CDCl₃): δ = 1.37 (s, 3H), 1.58 (s, 3H), 2.27 (d, 1 H), 3.44 (d, 1 H), 3.84 (s, 3H), 4.80 (s, 1 H), 5.71 (br, 1 H), 6.39 (d, 1 H), 6.44 (dd, 1 H), 6.66 (d, 1 H), 6.99 (d, 1 H), 7.51 (s, 1 H), 7.62 (dd, 1 H), 7.99 (d, 1 H), 8.07 (br, 1 H), 8.33 (d, 1 H), 8.49 (d, 1 H).
*5-[4-(4-Chlor-2-methoxyphenyl)-2-hydroxy-4-methyl-2-(trifluormethyl)pentylamino]chinolin-2-carbonsäureamid*
Analog zu Beispiel 40 werden 340 mg (0.69 mmol) 5-[4-(4-Chlor-2-methoxyphenyl)-2-hydroxy-4-methyl-2-(trifluormethyl)pentylidenamino]chinolin-2-carbonsäureamid in 10 mL Methanol gelöst und mehrmals mit 110 mg (2.89 mmol) Natriumborhydrid versetzt. Nach Aufarbeitung und Reinigung an Kieselgel mit Hexan-Ethylacetat (0-100%) erhält man 280 mg (82% der Theorie) des Produkts.
¹H-NMR (300 MHz, DMSO-d₆): δ = 1.37 (s, 3H), 1.56 (s, 3H), 2.06 (d, 1 H), 2.85-3.06 (m, 3H), 3.81 (s, 3H), 5.57-5.61 (m, 1 H), 5.96 (s, 1 H), 6.00 (d, 1 H), 6.95 (dd, 1 H), 6.98 (d, 1 H), 7.29-7.35 (m, 2H), 7.46 (t, 1 H), 7.71 (br, 1 H), 8.04 (d, 1H), 8.21 (br, 1 H), 8.48 (d, 1 H).

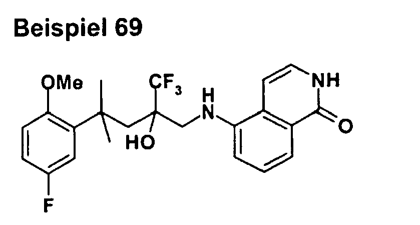

### 5-{[4-(5-Fluor-2-methoxyphenyl)- 2-hydroxy-4-methyl-2-(trifluormethyl)pentyl]aminolisochinolin-1 (2H)-on

### a. 2-Methyl-3-nitrobenzoesäuremethylester

20 g (110,4 mmol) 2-Methyl-3-nitrobenzoesäure werden in 100 ml Methanol nach Zusatz von 2 ml konzentrierter Schwefelsäure 10 Stunden am Rückfluss gekocht. Das beim Abkühlen auskristallisierende Produkt wird abgesaugt und getrocknet. Das Filtrat wird bis zur Trockene eingeengt, der feste Rückstand in Ethylacetat aufgenommen und die Lösung zweimal mit gesättigter Natriumhydrogencarbonatlösung gewaschen. Nach dem Trocknen über Na₂OS₄ erhält man ein weiteres Produkt. Zusammen werden 16,4 g (76,3 %) der gewünschten Verbindung erhalten.

### b. 5-Nitroisocoumarin

16,4 g (84,03 mmol) der unter a. beschriebenen Verbindung werden mit 26,8 g (225,1 mmol) N,N-Dimethylformamiddimethylacetal in 85 ml Dimethylformamid 12 Stunden bei 130° C gerührt. Das Lösungsmittel wird am Rotationsverdampfer abgezogen, der Rückstand in Methyl-tert-butylether aufgenommen und dreimal mit Wasser gewaschen. Nach dem Waschen mit gesättigter NaCl-Lösung wird die organische Phase getrocknet. Nach Abfiltrieren des Trockenmittels und Abrotieren des Lösungsmittels wird der verbleibende Rückstand an Kieselgel (Laufmittel Ethylacetat/Hexan) chromatographiert. Isoliert werden 8,73 g (54,4 %) der gewünschten Verbindung.
MS (CI) m/e (relative Intensität): 209 (M⁺¹⁸, 52), 191 (M⁺, 29), 179 (100)

### c. 5-Nitroisochinolin-1(2H)-on

2,51 g (13,13 mmol) 5- Nitroisocoumarin werden in 100 ml Ethanol gegeben. Unter Druck wird im Autoklaven Ammoniak eingedrückt. Das Produkt fällt aus und wird abgesaugt. Isoliert werden 1,98 g (79,7 %) der gewünschten Verbindung.
MS (Cl) m/e (relative Intensität): 208 (M⁺¹⁸, 60), 191 (M⁺¹, 100), 161 (81)

### d. 5-Aminoisochinolin-1(2H)-on

268,3 mg (1,51 mmol) 5-Nitroisochinolin-1(2H)-on werden mit 376,5 mg Ammoniumchlorid und 2,6 ml Wasser in 14 ml Ethanol und 5,4 ml Tetrahydrofuran gegeben. Nach portionsweiser Zugabe von 1,23 g Zinkpulver (Erwärmung auf 30 bis 35°C) wird zwei Stunden gerührt. Das Reaktionsgemisch wird über Glasfaserfilter abgesaugt und mit Ethylacetat nachgewaschen. Nach dem Waschen des Filtrats mit Wasser und gesättigter Natriumchloridlösung wird die organische Phase wie üblich getrocknet. Abfiltrieren des Trockenmittels und Abrotieren des Lösungsmittels ergeben 196,5 mg (88,1 %) des gewünschten Amins.
MS (Cl) m/e (relative Intensität): 161 (M⁺¹, 100)

### e. 5-{(E/Z)-[4-(5-Fluor-2-methoxyphenyl)-2-hydroxy-4-methyl-2-(trifluormethyl) pentyliden]amino}isochinolin-1 (2H)-on

140,1 mg (0,455 mmol) 4-(5-Fluor-2-methoxyphenyl)-2-hydroxy-4-methyl-2-(trifluormethyl)pentanal werden mit 72,8 mg (0,455 mmol) 5-Aminoisochinolin-1 (2H)-on in 0,74 ml Eisessig über Nacht gerührt. Die Mischung wird bis zur Trockene eingeengt und der Rückstand am Flashmaster (Laufmittel Ethylacetat/Hexan) gesäult. Isoliert werden 103,6 mg (52,5 %) der gewünschten Verbindung.
MS (ES+) m/e (relative Intensität): 451 (M⁺¹, 100)

### f. 5-{[4-(5-Fluor-2-methoxyphenyl)-2-hydroxy-4-methyl-2-(trifluormethyl) pentyl]amino}isochinolin-1 (2H)-on

103,6 mg (0,239 mmol) der unter e. beschriebenen Verbindung werden mit 2,6 ml Dichlorethan und 0,1 ml Eisessig versetzt. Nach Zugabe von 75,9 mg (0,359 mmol) Natriumtriacetoxyborhydrid wird über Nacht gerührt. Die Reaktionsmischung wird mit gesättigter Natriumhydrogencarbonatlösung versetzt und zweimal mit Ethylacetat extrahiert. Die vereinigten organischen Phasen werden mit Wasser und gesättigter Natriumchloridlösung gewaschen, getrocknet, filtriert und eingeengt. Nach Chromatographie am Flashmaster (Laufmittel Ethylacetat/Hexan) werden 36 mg (34,6 %) der gewünschten Verbindung isoliert.
MS (ES+) m/e (relative Intensität): 453 (M⁺¹, 100)

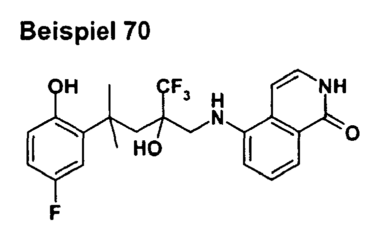

### 5-{[4-(5-Fluor-2-hydroxyphenyl)-2-hydroxy-4-methyl-2-(trifluormethyl)pentyl]amino}isochinolin-1 (2H)-on

30 mg (0,066 mmol) der unter Beispiel 70 f. synthetisierten Verbindung werden mit 0,76 ml einer 1 M Lösung von Bortribromid in Dichlormethan versetzt. Nach vierstündigem Rühren bei Raumtemperatur wird die Reaktionsmischung mit Ethylacetat verdünnt und einmal mit gesättigter Natriumhydrogencarbonatlösung gewaschen. Nach Trocknen der organischen Phase (Natriumsulfat) wird das Lösungsmittel nach dem Abfiltrieren des Trockenmittels abrotiert.

Chromatographie am Flashmaster (Laufmittel Ethylacetat/Hexan) ergibt 15,9 mg (56,7 %) der gewünschten Verbindung.
MS (ES+) m/e (relative Intensität): 439 (M⁺¹, 100)

Analog wurden ausgehend von den entsprechenden Aldehyden synthetisiert:
5-{[4-(3-Fluor-2-hydroxyphenyl)-2-hydroxy-4-methyl-2-(trifluormethyl) pentyl]amino}isochinolin-1 (2H)-on
5-({3-[1-(3-Fluor-2-hydroxyphenyl)cyclopropyl]-2-hydroxy-2-(trifluormethyl)propyl}amino)isochinolin-1 (2H)-on
5-{[4-(4-Chlor-2-hydroxyphenyl)-2-hydroxy-4-methyl-2-(trifluormethyl) pentyl]amino}isochinolin-1 (2H)-on
5-{(4-(5-Chlor-2-hydroxyphenyl)-2-hydroxy-4-methyl-2-(trifluormethyl) pentyl]amino}isochinolin-1 (2H)-on
5-{[4-(2-Chlorphenyl)-2-hydroxy-4-methyl-2-(trifluormethyl) pentyl]amino}isochinolin-1 (2H)-on
(+)-5-{[4-(5-Fluor-2-hydroxyphenyl)-2-hydroxy-4-methyl-2-(trifluormethyl) pentyl]amino}isochinolin-1(2H)-on
(-)-5-{[4-(5-Fluor-2-hydroxyphenyl)-2-hydroxy-4-methyl-2-(trifluormethyl) pentyl]amino}isochinolin-1(2H)-on

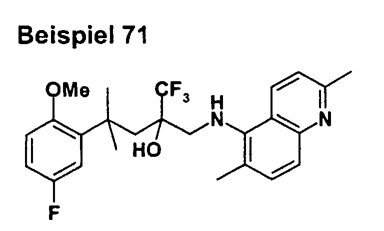

### 5-{[4-(5-Fluor-2-methoxyphenyl)-2-hydroxy-4-methyl-2-(trifluormethyl)pentyl]amino}-2,6-dimethyl-chinolin

### a. 2,6-Dimethyl-5-nitrochinolin

3 g (19,08 mmol) 2,6-Dimethylchinolin werden in 15 ml konzentrierter Schwefelsäure bei 10° C vorgelegt. Nach einer halben Stunde werden eine Lösung von 2,03 g Kaliumnitrat in 11,4 konzentrierter Schwefelsäure zugetropft, und zwar so, dass die Temperatur zwischen 5° und 15° C bleibt. Der Ansatz wird eine Stunde nachgerührt und anschließend auf Eiswasser gegossen. Es wird ammoniakalisch gestellt und der ausgefallene Niederschlag abgesaugt. Nach dem Waschen mit Wasser wird das Rohprodukt in Ethylacetat gelöst und gegen Wasser geschüttelt. Die organische Phase wird wie üblich behandelt. Nach dem Abrotieren des Lösungsmittels bleiben 3,69 g (95,6 %) der gewünschten Verbindung zurück, die ohne weitere Reinigung in die Reduktion eingesetzt werden.
MS (Cl) m/e (relative Intensität): 220 (M⁺¹⁸, 20), 203 (M⁺¹, 100)

### b. 5-Amino-2,6-dimethy-chinolin

3,69 g (18,248 mmol) der nach a. hergestellten Verbindung wird mit 15,19 g (66,85 mmol) Zinn (II)-chlorid dihydrat und 30,4 ml konzentrierter Salzsäure 45 Minuten bei 85° C gerührt. Der Ansatz wird auf Eiswasser gegossen, mit 2 N NaOH basisch gestellt und das Amin mit Ethylacetat extrahiert. Die organischen Extrakte werden mit Sole gewaschen, über Natriumsulfat getrocknet und das Lösungsmittel nach dem Absaugen des Trockenmittels abrotiert. Der verbliebene Rückstand wird an Kieselgel (Laufmittel Ethylacetat/Hexan) chromatographiert. Isoliert werden 2,75 g (87,6 %) der gewünschten Verbindung.
MS (ES+) m/e (relative Intensität): 173 (M⁺¹, 100)

### c. 5-{(E/Z)-[4-(5-Fluor-2-methoxyphenyl)-2-hydroxy-4-methyl-2-(trifluormethyl) pentyliden]amino}2,6-dimethylchinolin

250 mg (0,811 mmol) 4-(5-Fluor-2-methoxyphenyl)-2-hydroxy-4-methyl-2-trifluormethyl)-pentanal werden mit 139,7 mg (0,811 mmol) 5-Amino, 2,6-dimethylchinolin in zehn Milliliter Dichlorethan unter Zusatz von 0,2 ml Trifluoressigsäure und 150 mg Molsieb (4 A) sieben Tage am Rückfluss gekocht. Die Mischung wird über Glasfaserfilter filtriert und das Filtrat bis zur Trockene eingeengt. Der Rückstand wird am Flashmaster (Laufmittel Ethylacetat/Hexan) gesäult. Isoliert werden 134,3 mg (35,8 %) der gewünschten Verbindung.
MS (ES+) m/e (relative Intensität): 463 (M⁺¹, 100)

### d. 5-{[4-(5-Fluor-2-methoxyphenyl)-2-hydroxy-4-methyl-2-(trifluormethyl) pentyl]amino}2,6-dimethylchinolin

134,3 mg (0,29 mmol) der unter c. beschriebenen Verbindung werden in 17 ml Methanol gegeben und mit 1,7 ml Eisessig versetzt. Nach Zugabe von 141,5 mg (2,252 mmol) Natriumcyanoborhydrid wird vier Stunden gerührt. Die Reaktionsmischung wird mit gesättigter Natriumhydrogencarbonatlösung versetzt und zweimal mit Ethylacetat extrahiert. Die vereinigten organischen Phasen werden mit Wasser und gesättigter Natriumchloridlösung gewaschen, getrocknet, filtriert und eingeengt. Nach Chromatographie am Flashmaster (Laufmittel Ethylacetat/Hexan) werden 80 mg (59,3 %) der gewünschten Verbindung isoliert.
MS (ES+) m/e (relative Intensität): 465 (M⁺¹, 100)

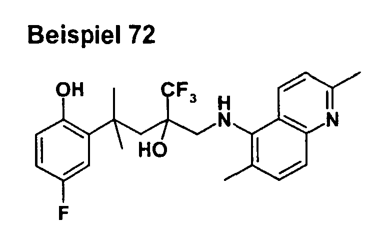

### 5-{[4-(5-Fluor-2-hydroxyphenyl)-2-hydroxy-4-methyl-2-(trifluormethyl)pentyl]amino}-2,6-dimethyl-chinolin

80 mg (0,172 mmol) der unter Beispiel 71 d. synthetisierten Verbindung werden mit zwei Milliliter einer 1 M Lösung von Bortribromid in Dichlormethan versetzt. Nach fünfstündigem Rühren bei Raumtemperatur wird die Reaktionsmischung mit Ethylacetat verdünnt und einmal mit gesättigter Natriumhydrogencarbonatlösung gewaschen. Die organische Phase wird getrocknet (Natriumsulfat) und das Lösungsmittel nach dem Abfiltrieren des Trockenmittels abrotiert. Chromatographie am Flashmaster (Laufmittel Ethylacetat/Hexan) ergibt 58,9 mg (75,9 %) der gewünschten Verbindung.
MS (ES+) m/e (relative Intensität): 451 (M⁺¹, 100)

Analog wurden ausgehend von den entsprechenden Aldehyden und Aminen folgende Verbindungen synthetisiert:
(+)-5-{[4-(5-Fluor-2-hydroxyphenyl)-2-hydroxy-4-methyl-2-(trifluormethyl) pentyl]amino}-2,6-dimethylchinolin
(-)-5-{[4-(5-Fluor-2-hydroxyphenyl)-2-hydroxy-4-methyl-2-(trifluormethyl) pentyl]amino}-2,6-dimethylchinolin
5-{[4-(3-Fluor-2-hydroxyphenyl)-2-hydroxy-4-methyl-2-(trifluormethyl) pentyl]amino}-2,6-dimethylchinolin
5-{[4-(5-Fluor-2-hydroxyphenyl)-2-hydroxy-4-methyl-2-(trifluormethyl) pentyl]amino}-6-chlor-2-methylchinolin
5-{[4-(3-Fluor-2-hydroxyphenyl)-2-hydroxy-4-methyl-2-(trifluormethyl) pentyl]amino}6-chlor-2-methylchinolin 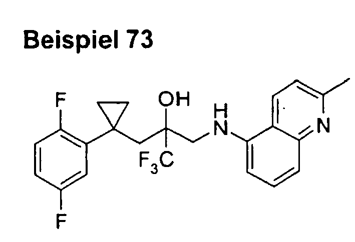
3-[1-(2,5-Difulorphenyl)-cycloprop-1-yl]-1-(2-methylchinolin-5-ylamino)-2-(trifluormethyl)-1-(2-methylchinolin-5-ylamino)-propan-2-ol
Analog zu Beispiel 76 werden 60 mg (1.46 mmol) NaH (60%) und 244 mg (1.23 mmol) 5-Acetylamino-2-methyl-chinolin mit 365 mg (0.81 mmol) Toluol-4-sulfonsäure-3-[1-(2,5-difluorphenyl)-cycloprop-1-yl]-2-(trifluormethyl)-2-hydroxypropyl ester umgesetzt. Das Rohprodukt wird analog Beispiel 76 mit 1 m Natronlauge behandelt. Nach Chromatographie an Kieselgel mit Hexan-Essigester (30-50%) erhält man 67 mg Produkt.
'H-NMR (CDCl₃); δ = 0.80-1.02 (m, 4H), 2.16 (d, 1 H), 2.42 (d, 1 H), 2.21 (s, 3H), 3.27-3.42 (m, 2H), 4.21-4.30 (m, 1 H), 6.30 (dd, 1 H), 6.66-6.81 (m, 2H), 6.95-7.05 (m, 1 H), 7.22 (d, 1 H), 7.40-7.50 (m, 2H), 7.96 (d, 1 H). MS (ESI): 437 (M+H).

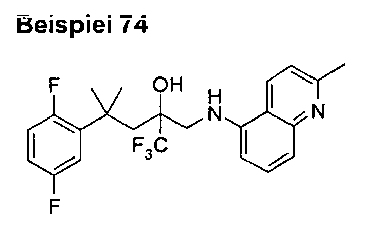

### 4-(2 5-Difluorphenyl)-4-methyl-1-(2-methylchinolin-5-ylamino)-2-(trifluormethyl)-pentan-2-ol

175 mg (0.62 mmol) 4-(2,5-Difluorphenyl)-4-methyl-2-(trifluormethyl)-pentanal und 125 mg (0.78 mmol) 5-Amino-2-methylchinolin werden in 10 ml Toluol und 3 ml Essigsäure 18 h bei RT gerührt und weitere 2 h am Wasserabscheider zum Rückfluss erhitzt. Das Rohprodukt wird analog 79 mit 120 mg (3.16 mmol) NaBH₄ in 5 ml Essigsäure reduziert. Nach Chromatographie an Kieselgel mit Hexan-Essigester (30%) erhält man 45 mg Produkt.
MS (EI): 438 (M⁺)

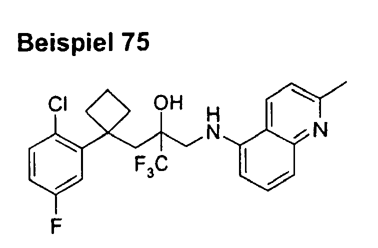

### 3-[1-(2-Chlor-5-fluorphenyl)cyclobut-1-yl]-1-(2-methylchinolin-5-ylamino)-2-(trifluormethyl)-propan-2-ol

Analog zu Beispiel 79 werden 200 mg (0.64 mmol) 3-[1-(2-Chlor-5-fluorphenyl)cyclobut-1-yl]-2-(trifluormethyl)-propanal und 126 mg (0.80 mmol) 5-Amino-2-methylchinolin umgesetzt und das isolierte Rohprodukt mit 102 mg (2.70 mmol) NaBH₄ reduziert. Nach Chromatographie an Kieselgel mit Hexan-Essigester erhält man 165 mg Produkt.
MS (ESI): 467 (M+H); Drehwert des reinen (-)-Enantiomers nach Trennung auf chiraler HPLC: α_{D} = - 31.4.

### Weitere Beispiele:

### Synthese der Diol-Vorstufen:

### Diol-Beispiel a:

### 4-(7-Brom-1,3-benzodioxol-4-yl)-2-hydroxy-4-methyl-2-(trifluormethyl)pentan-1-ol:

### 2,3-Dihydroxy-4-nitrobenzaldehyd:

17.2 g 2-Hydroxy-3-methoxy-4-nitrobenzaldehyd (J. Het. Chem. **33**, (1996), 1171) in 350 ml Dichlormethan werden bei 0°C langsam mit 175 ml Bortribromid Lösung (1 M in Dichlormethan) versetzt und es wird noch weitere 4 h gerührt. Der Ansatz wird auf Eiswasser gegeben, die org. Phase abgetrennt und die wässrige Phase mit Dichlormethan extrahiert. Die vereinten org. Phasen werden mit Wasser gewaschen und das Lösungsmittel im Vakuum entfernt. Man erhält 15.2 g 2,3-Dihydroxy-4-nitrobenzaldehyd als roten Feststoff. Fp. 122-126°C

### 7-Nitro-benzo[1,3]dioxol-4-carbonsäure:

14.2 g 2,3-Dihydroxy-4-nitrobenzaldehyd in 1000 ml DMF werden mit 57.6 ml Bromchlormethan und 50.6 g Cesiumcarbonat bei 100°C für 7 h gerührt. Der Ansatz wird auf 1 N Salzsäure gegeben und mit Essigester extrahiert. Die org. Phase wird mehrfach mit Wasser und Sole gewaschen, mit Natriumsulfat getrocknet und im Vakuum eingeengt. Der erhaltene braune Feststoff wird durch Säulenchromatographie (Kieselgel, Hexan / Essigester 100:0 -> 60:40) aufgereinigt. Man erhält 7.4 g 7-Nitro-benzo[1,3]dioxol-4-carbaldehyd als hellgelben Feststoff. Dieser wird in 400 ml Aceton bei 10°C mit einer Lösung von 11.8 g Kaliumpermanganat in 190 ml Aceton / Wasser (1:1) versetzt. Es wurde 10 h gerührt, auf 2 N Salzsäure gegeben und durch Kieselgur filtriert. Das Aceton wird im Vakuum entfernt und die wässrige Phase mit Natronlauge alkalisch gestellt. Es wird mit Ether extrahiert und dann die wässrige Phase mit Salzsäure angesäuert. Es wird mit Essigester extrahiert, mit Wasser gewaschen, mit Natriumsulfat getrocknet und im Vakuum eingeengt. Man erhält 4.36 g 7-Nitro-benzo[1,3]dioxol-4-carbonsäure als braunen Feststoff. Fp. 230-239°C

### 7-Amino-benzo[1,3]dioxol-4-carbonsäure:

8.05 g 7-Nitro-benzo[1,3]dioxol-4-carbonsäure werden in 650 ml Ethanol mit 800 mg Palladium/Kohle Katalysator unter Normaldruck bei RT mit Wasserstoff umgesetzt. Es wird durch Kieselgur filtriert und im Vakuum eingeengt. Man erhält 6.82 g 7-Amino-benzo[1,3]dioxol-4-carbonsäure als braunen Feststoff. Fp. 196-197°C

### 2-(7-Brom-1,3-benzodioxol-4-yl)-propan-2-ol:

1.95 g 7-Amino-benzo[1,3]dioxol-4-carbonsäure in 25 ml Bromwasserstoffsäure (48%ig) und 20 ml Wasser werden bei 0°C mit einer Lösung von 760 mg Natriumnitrit in 4.2 ml Wasser versetzt. Es wird 15 min. gerührt und dann zu einer Lösung von 2.07 g Kupfer(I)bromid in 5.5 ml Bromwasserstoffsäure (48%ig) gegeben. Es wird für 30 min. auf 100°C erhitzt, auf Wasser gegeben und mit Essigester extrahiert. Es wird mit Sole gewaschen, mit Natriumsulfat getrocknet und im Vakuum eingeengt. Man erhält 2.88 g 7-Brom-benzo[1,3]dioxol-4-carbonsäure als braunen Feststoff. Dieser wird in 50 ml DMF bei 0°C mit 4.08 g Cesiumcarbonat und 0.8 ml Methyliodid umgesetzt. Es wird 4 h bei RT gerührt, auf
Wasser gegeben und mit Essigester extrahiert. Es wird mehrfach mir Wasser und Sole gewaschen, getrocknet und im Vakuum eingeengt. Man erhält 1.81 g 7-Brom-benzo[1,3]dioxol-4-carbonsäuremethylester als roten Feststoff. Dieser wird gelöst in 5 ml Ether und 10 ml THF zu einer Lösung von 5.06 ml Methylmagnesiumchlorid (3 M in THF) und 6 ml Ether bei RT gegeben. Nach 3.5 h wird der Ansatz auf 1 N Salzsäure gegeben, mit Essigester extrahiert, mit Sole gewaschen, getrocknet und im Vakuum eingeengt. Man erhält 1.79 g 2-(7-Brom-1,3-benzodioxol-4-yl)-propan-2-ol als orangefarbenes Öl.
¹H-NMR (CDCl₃), δ (ppm) =1.58 (s, 6H), 6.05 (s, 2H), 6.85 (d, 1 H), 6.97 (d, 1 H)

### 4-(7-Brombenzo[1.3]dioxol-4-yl)-4-methyl-2-oxo-valeriansäure:

1.77 g 2-(7-Brombenzo[1,3]dioxol-4-yl)-propan-2-ol und 2.82 g 2-Trimethylsilyloxy-acrylsäure-ethylester (WO 00/32584 ) in 36 ml Dichlormethan werden bei -70°C mit 1.10 ml Zinn(Vl)chlorid versetzt. Es wird 20 min. bei -70°C gerührt und dann auf ges. Natriumcarbonat Lösung gegeben. Es wird mit Dichlormethan extrahiert, mit Wasser gewaschen, getrocknet und im Vakuum eingeengt. Man erhält 3.15 g 4-(7-Brom-1,3-benzodioxol-4-yl)-4-methyl-2-oxo-valeriansäure-ethylester als Rohprodukt. Dieser wird 4 h bei RT in 57 ml einer Mischung aus 1 N Natronlauge in Ethanol / Wasser umgesetzt. Der Ansatz wird auf Wasser gegeben, mit Ether extrahiert und dann die wässrige Phase mit Salzsäure angesäuert. Es wird mit Essigester extrahiert, mit Wasser gewaschen, mit Natriumsulfat getrocknet und im Vakuum eingeengt. Man erhält 2.1 g 4-(7-Brom-1,3-benzodioxol-4-yl)-4-methyl-2-oxo-valeriansäure als gelbes Öl.
¹H-NMR (CDCl₃), δ (ppm) = 1.46 (s, 6H), 3.44 (s, 2H), 5.98 (s, 2H), 6.66 (d, 1H), 6.94 (d, 1 H)

### 4-(7-Brom-1,3-benzodioxol-4-yl)-2-hydroxy-4-methyl-2-trifluormethyl-pentan-1-ol:

300 mg 4-(7-Brom-1,3-benzodioxol-4-yl)-4-methyl-2-oxo-valeriansäure und 0.1 ml Schwefelsäure (96%ig) in 5 ml Ethanol werden 4 h auf 70°C erhitzt. Dann wird der Ansatz im Vakuum eingeengt und auf Wasser gegeben. Es wird mit Essigester extrahiert, mit Sole gewaschen, getrocknet und im Vakuum eingeengt. Man erhält 272 mg 4-(7-Brom-1,3-benzodioxol-4-yl)-4-methyl-2-oxo-valeriansäure-ethylester als gelbes Öl. Dieser Ester und 0.32 ml Trifluormethyl-trimethylsilan in 4 ml THF werden mit 0.14 ml einer Tetrabutylammoniumfluorid-Lösung (1 M in THF) bei -70°C versetzt. Es wird 1 h bei -70°C, 1.5 h bei -10°C, 1 h bei 0°C und 2 h bei RT gerührt. Es wird noch eine Spatelspitze Tetrabutylammoniumfluorid zugegeben und nach weiterem Rühren für 20 min. auf Wasser gegeben. Es wird mit Essigester extrahiert, mit Wasser gewaschen, mit Natriumsulfat getrocknet und im Vakuum eingeengt. Man erhält 297 mg 4-(7-Brom-1,3-benzodioxol-4-yl)-2-hydroxy-4-methyl-2-trifluormethyl-valeriansäure-ethylester als gelbes Öl. Dieses Öl wird in 5 ml Ether bei 0°C mit 29 mg Lithiumaluminiumhydrid versetzt, 1 h bei 0°C und noch weitere 10 h bei RT gerührt. Der Ansatz wird auf verdünnte Salzsäure gegeben, mit Essigester extrahiert, mit Wasser gewaschen, mit Natriumsulfat getrocknet und im Vakuum eingeengt. Man erhält 164 mg 4-(7-Brom-1,3-benzodioxol-4-yl)-2-hydroxy-4-methyl-2-(trifluormethyl)pentan-1-ol als gelbes Öl.
¹H-NMR (CDCl₃), δ (ppm) = 1.40 (s, 3H), 1.48 (s, 3H), 2.20-2.28 (m, 2H), 2.9 (s, 1 H), 3.42 (d, 1 H), 3.56 (d, 1 H), 6.02 (d, 2H), 6.71 (d, 1 H), 6.94 (d, 1 H) MS (ei) m/e: M⁺ = 384 (⁷⁹Br) /386 (⁸¹Br)

### Diol-Beispiel b:

### 4-(4-Chlorphenyl)-2-hydroxy-4-methyl-2-(trifluormethyl)pentan-1-ol:

### 2-(4-Chlorohenyl)-2-methylpropanal:

10 g 4-Chlorbenzylcyanid und 14.3 ml Methyliodid in 140 ml DMF werden bei 0°C potionsweise mit Natriumhydrid (60%ig in Öl) versetzt. Es wird über Nacht gerührt und dann mit Wasser und Essigester versetzt. Die Phasen werden getrennt und die wässrige Phase mit Essigester extrahiert.
Es wird gründlich mit Wasser extrahiert, mit Sole gewaschen, mit Natriumsulfat getrocknet und im Vakuum eingeengt. Nach Chromatographie an Kieselgel (Hexan / Essigester 95:5) erhält man 11.73 g 2-(4-Chlorphenyl)-2-methylpropionitril als farbloses Öl. Dieser wird in Toluol bei - 78°C langsam mit 55.4 ml Diisobutylaluminiumhydrid Lösung (20% in Toluol) versetzt und nach 4 h bei -78°C wurden 50 ml Essigester zugetropft. Er wird unter Erwärmung auf RT über Nacht gerührt und Wasser zugegeben. Nach filtrieren durch Kieselgur werden die Phasen getrennt und die wässrige Phase mit Essigester extrahiert. Es wird mit Wasser und Sole gewaschen, mit Natriumsulfat getrocknet und im Vakuum eingeengt. Nach Chromatographie an Kieselgel (Hexan / Essigester 95:5) erhält man 10.2 g 2-(4-Chlorphenyl)-2-methylpropanal als farbloses Öl.
¹H-NMR (CDCl₃), δ (ppm) = 1.46 (s, 6H), 7.20 (d, 1H), 7.29-7.43 (m, 3H), 9.48 (s, 1H)

### 4-(4-Chlorphenyl)-4-methyl-2-oxo-valeriansäure:

Eine Lösung von 15.04 g 2-Diethylphosphono-2-ethoxyessigsäure-ethylester in 50 ml Tetrahydrofuran wird unter Eiskühlung innerhalb von 20 Minuten mit 30 ml einer 2 M Lösung von Lithiumdiisopropylamid in Tetrahydrofuran-Heptan-Toluol versetzt und 15 Minuten bei 0 °C gerührt. Innerhalb von 30 Minuten wird eine Lösung von 10.2 g 2-(4-Chlorphenyl)-2-methylpropanal in 50 ml Tetrahydrofuran bei 0°C dazugetropft. Nach 20 Stunden bei RT wird 2 N Schwefelsäure zugegeben, mit Ethylacetat extrahiert, getrocknet (Na₂SO₄) und eingeengt. Das Rohprodukt wird mit 200 ml 2 M Natronlauge / 400 ml Ethanol verseift. Man erhält 13.8 g Säure, die mit 300 ml 2 N Schwefelsäure und 100 ml Eisessig unter starkem Rühren unter Rückfluss 3 Stunden erhitzt wird. Nach Extraktion mit Ethylacetat und Waschen mit Wasser werden 10.9 g 4-(4-Chlorphenyl)-4-methyl-2-oxo-valeriansäure als rotes Öl erhalten.
¹H-NMR (CDCl₃), δ (ppm) = 1.47 (s, 6H), 3.28 (s, 2H), 7.28 (s, 4H), 7.73 (bs, 1H)

### 4-(4-Chlorphenyl)-2-hydroxy-4-methyl-2-(trifluormethyl)-pentan-1-ol:

In Analogie zur Synthese von 4-(7-Brom-1,3-benzodioxol-4-yl)-2-hydroxy-4-methyl-2-(trifluormethyl)pentan-1-ol (Diol Beispiel 1) werden durch Veresterung von 10.9 g 4-(4-Chlorphenyl)-4-methyl-2-oxo-valeriansäure in Ethanol / Schwefelsäure, Umsetzung der Produktes mit Trifluormethyl-trimethylsilan und Tetrabutylammoniumfluorid und Reduktion des gebildeten Hydroxyesters mit Lithiumaluminiumhydrid 4.22 g 4-(4-Chlorphenyl)-2-hydroxy-4-methyl-2-(trifluormethyl)pentan-1-ol als farbloses Öl erhalten.
¹H-NMR (CDCl₃), δ (ppm) = 1.39 (s, 3H), 1.49 (s, 3H), 2.07 (d, 1 H), 2.19 (d, 1 H), 2.83 (bs, 1 H), 3.27 (d, 1 H), 3.41 (d, 1 H), 7.26-7.38 (m, 4H)

### Diol-Beispiel c und d:

### 4-(4-Chlor-2-methoxyphenyl)-2-hydroxy-4-methyl-2-(trifluormethyl)pentan-1-ol und 4-(2-Chlor-4-methoxyphenyl)-2-hydroxy-4-methyl-2-(trifluormethyl)pentan-1-ol

Eine Lösung von 3 g 2-Hydroxy-4-methylen-2-(trifluormethyl)valeriansäureethylester in 22 ml 3-Chloranisol werden bei RT portionsweise mit Aluminiumtrichlorid versetzt. Es wird 48 h gerührt und dann mit 2 N Salzsäure und Hexan versetzt und 1 h gerührt. Es wird mit 2 N Salzsäure und Wasser gewaschen und überschüssiges 3-Chloranisol im Vakuum abdestilliert. Der verbleibende Rückstand wird durch Chromatographie an Kieselgel (Hexan / Essigester 100:0 -> 90:10) gereinigt. Man erhält 2.85 g eines Gemisches von 4-(4-Chlor-2-methoxyphenyl)-2-hydroxy-4-methy-2-(trifluormethyl)valeriansäureethylester und 4-(2-Chlor-4-methoxyphenyl)-2-hydroxy-4-methy-2-(trifluormethyl)valeriansäureethylester als gelbes Öl. Dieses Substanzgemisch wird in 90 ml Ether bei 0°C mit 445 mg Lithiumaluminiumhydrid versetzt und 12 h gerührt. Der Ansatz wird auf gesättigte Natriumhydrogencarbonat-Lösung gegeben, durch Kieselgur filtriert, die Phasen werden getrennt und die wässrige Phase mit Essigester extrahiert. Es wird mit Wasser und Sole gewaschen, mit Natriumsulfat getrocknet und im Vakuum eingeengt. Nach Chromatographie an Kieselgel (Hexan / Essigester 95:5) erhält man als 1. Fraktion 1.87 mg 4-(4-Chlor-2-methoxyphenyl)-2-hydroxy-4-methyl-2-(trifluormethyl)pentan-1-ol und als 2. Fraktion 160 mg 4-(2-Chlor-4-methoxyphenyl)-2-hydroxy-4-methyl-2-(trifluormethyl)pentan-1-ol als Farblose Öle.
1. Fraktion: ¹H-NMR (CDCl₃), δ (ppm) =1.41 (s, 3H), 1.51 (s, 3H), 2.24 (d, 1H), 2.51 (d, 1 H), 2.84 (bs, 1 H), 3.36 (d, 1 H), 3.48 (d, 1 H), 3.85 (s, 3H), 6.88 (d, 1 H), 6.92 (dd, 1H), 7.24 (d, 1H)
2. Fraktion: ¹H-NMR (CDCl₃), δ (ppm) = 1.52 (s, 3H), 1.62 (s, 3H), 2.18 (d, 1 H), 2.76 (d, 1 H), 2.93 (bs, 1 H), 3.33 (d, 1 H), 3.55 (d, 1 H), 3.80 (s, 3H), 6.78 (dd, 1 H), 6.90 (d, 1 H), 7.38 (d, 1 H)

Über die oben beschriebenen Verfahren Diol-Beispiel a bis Diol-Beispiel d wurden folgende Verbindungen hergestellt: Tabelle I

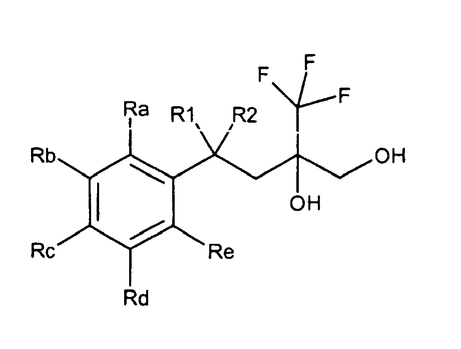

**Tabelle I**

| Bsp. | Diol Synthese-Verfahren | R1 / R2 | Ra-Re (≠H) | MS |
|---|---|---|---|---|
| e | b | -CH₂-CH₂- | Ra = Cl | M⁺+1 = 295 (³⁵Cl), 297 (³⁷Cl); (esi) |
| f | a | CH₃, CH₃ | Ra-Rb = -O-CH₂-O- | M⁺ = 306 (ei) |
| g | b | CH₃, CH₃ | Ra = Cl | M⁺ = 296 (³⁵Cl), 298 (³⁷Cl); (ei) |
| h | b | CH₃, CH₃ | Rb = Cl | M⁺ = 296 (³⁵Cl), 298 (³⁷Cl) ; (ei) |
| i | a | CH₃, CH₃ | Ra-Rb = -O-CH₂-O-, Rc = Cl | M⁺ = 340 (³⁵Cl), 342 (³⁷Cl); (ei) |
| j | b | CH₃, CH₃ | Ra = Rc = Cl | M⁺ = 330 (2x³⁵Cl), 332 (³⁷Cl+³⁵Cl), 334 (2x³⁷Cl); (ei) |
| k | b | CH₃, CH₃ | Ra = CF₃, Rd = F | M⁺ = 348 (ei) |
| l | a | CH₃, CH₃ | Ra = OCH₃, Rb = Rd = F | M⁺+1 = 329 (esi) |
| m | b | CH₃, CH₃ | Ra = Rd = F | M⁺ = 298 (ci) |
| n | b | -(CH₂)₃- | Ra = Cl, Rd = F | M⁺+NH₄ = 344 (ci) |
| o | b | CH₃, CH₃ | Ra = Cl, Rd = F | 314, 316 (EI⁺) |
| p | b | -(CH₂)₂- | Ra = Cl, Rc = F | 312, 314 (EI⁺) |
| q | b | -(CH₂)₂- | Ra = Cl, Rd = F | 312, 314 (EI⁺) |
| r | b | -(CH₂)₃- | Ra = OCH₃, Rd = F | 322, 324 (EI⁺) |
| s | b | CH₃, CH₃ | Ra = Cl, Rc = F | 378 (-Cl, EI⁺) |
| t | c | CH₃, CH₃ | Ra = OCH₃, Rd = Br | 370, 372 (EI⁺) |

### Beispiel 76:

### (+/-)-4-(7-Brom-1,3-benzodioxol-4-yl)-4-methyl-1-(2-methylchinolin-5-ylamino)-2-(trifluormethyl)pentan-2-ol

Eine Lösung von 160 mg 4-(7-Brom-1,3-benzodioxol-4-yl)-2-hydroxy-4-methyl-2-(trifluormethyl)pentan-1-ol und 111 mg 4-Toluolsulfonsäurechlorid in 1.5 ml Pyridin werden 70 h bei 0°C gerührt. Es wird im Vakuum eingeengt, in 2 N Salzsäure aufgenommen und mit Essigester extrahiert. Es wird mit 2 N Salzsäure und Wasser gewaschen, mit Natriumsulfat getrocknet und im Vakuum eingeengt. Man erhält 188 mg 4-Toluolsulfonsäure-[4-(7-brom-1,3-benzodioxol-4-yl)-2-hydroxy-4-methyl-2-(trifluormethyl)]pentylester als gelbes Öl. Diese werden zu einer Lösung gegeben, die aus 94 mg N-Acetyl-5-amino-2-methylchinolin und Natriumhydrid (60%ig in Öl) in 1.5 ml DMF bei 0°C bereitet und 1.5 h gerührt wird. Es wird 7 h auf 50°C erwärmt. Es wird noch weitere 70 h bei RT gerührt und auf gesättigte Natriumhydrogencarbonat-Lösung gegeben. Es wird mit Essigester extrahiert, mit Wasser und Sole gewaschen, mit Natriumsulfat getrocknet und im Vakuum eingeengt. Nach Chromatographie an Kieselgel (Hexan / Essigester 100:0 -> 85:15) erhält man 74 mg N-Acetyl-(7-brom-1,3-benzodioxol-4-yl)-4-methyl-1-[(2-methylchinolin-5-yl)amino]-2-(trifluormethyl)pentan-2-ol als gelbes Öl. Diese wird mit 4 ml Ethanol und 1.2 ml 1 N Natronlauge für 8 h auf 100°C erhitzt. Man engt im Vakuum ein und extrahiert mit Essigester. Es wird mit Wasser und Sole gewaschen, mit Natriumsulfat getrocknet und im Vakuum eingeengt. Nach Chromatographie an Kieselgel (Hexan / Essigester 100:0 -> 85:15) erhält man 51 mg der Titelverbindung als gelben Schaum.
¹H-NMR (CDCl₃), δ (ppm) = 1.46 (s, 3H), 1.58 (s, 3H), 2.29 (d 1 H), 2.50 (d, 1 H), 2.74 (s, 3H), 3.13 (bs, 1 H), 3.18 (dd, 1 H), 3.35 (dd, 1 H), 4.30 (bs, 1 H), 5.98 (s, 2H), 6.11 (d, 1H), 6.80 (d, 1H), 7.00 (d, 1H). 7.21 (d, 1 H), 7.49 (d, 1 H), 7.52 (d, 1H), 7.95 (d, 1H).

### Beispiel 77, 78:

### (-)-4-(7-Brom-1,3-benzodioxol-4-yl)-4-methyl-1-[(2-methylchinolin-5-yl)amino]-2-(trifluormethyl)pentan-2-ol (+)-4-(7-Brom-1,3-benzodioxol-4-yl)-4-methyl-1-[(2-methylchinolin-5-yl)amino]-2-(trifluormethyl)pentan-2-ol

Trennung von (+/-)-4-(7-Brom-1,3-benzodioxol-4-yl)-4-methyl-1-[(2-methylchinolin-5-yl)amino]-2-(trifluormethyl)pentan-2-ol :
Das Enantiomerengemisch wird durch Chromatographie an chiralem Trägermaterial (CHIRALPAK AD^{®}, Fa DAICEL) mit Hexan / Ethanol / Triethylamin (98 : 2 : 0.1, vvv) getrennt. Man erhält so das (-)-Enantiomer: MS (ei): M⁺ = 524 (⁷⁹Br) 526 (⁸¹Br), [α]_{D} -50.4° (c = 0.5, CHCl₃) und das
(+)-Enantiomer: MS (ei): M⁺ = 524 (⁷⁹Br) 526 (⁸¹Br).

### Beispiel 79:

### (+/-)-4-(4-Chlorphenyl)-4-methyl-1-[(2-methylchinolin-5-yl)amino]-2-(trifluormethyl)pentan-2-ol

0,674 ml Oxalylchlorid in 16.8 ml Dichlormethan werden bei -78°C mit 1.15 ml DMSO in 3.4 ml Dichlormethan versetzt. Nach 5 min. werden2 g 4-(4-Chlorphenyl)-2-hydroxy-4-methyl-2-(trifluormethyl)pentan-1-ol in 6.8 ml Dichlormethan bei -78°C zugetropft. Nach 15 min. wird mit 4.7 ml Triethylamin versetzt und langsam auf RT erwärmt. Es wird mit Wasser und Sole gewaschen, mit Natriumsulfat getrocknet und im Vakuum eingeengt. Nach Chromatographie an Kieselgel (Hexan / Essigester 98:2) erhält man 1.07 g 4-(4-Chlorphenyl)-2-hydroxy-4-methyl-2-(trifluormethyl)-pentanal als farbloses Öl. Davon wurden 300 mg mit 194 mg 5-Amino-2-methylchinoplin in 9 ml Eisessig für 4.5 h auf 130°C erhitzt. Nach Zugaben von Toluol wird am Vakuum eingeengt. Der Rückstand wird in Methanol aufgenommen und bei 0°C mit 153 mg Natriumborhydrid versetzt. Man lässt 5 h bei RT rühren und gibt Wasser zu. Der Ansatz wird im Vakuum eingeengt , mit Essigester extrahiert, mit Wasser und Sole gewaschen, mit Natriumsulfat getrocknet und im Vakuum eingeengt. Nach Chromatographie an Kieselgel (Hexan / Essigester 100:0 -> 85:15) erhält man 163 mg der Titelverbindung als gelben Feststoff. Fp.: 137-139°C

### Beispiel 80, 81:

### (-)-4-(4-Chlorphenyl)-4-methyl-1-[(2-methylchinolin-5-yl)amino]-2-(trifluormethyl)pentan-2-ol (+)-4-(4-Chlorphenyl)-4-methyl-1-[(2-methylchinolin-5-yl)amino]-2-(trifluormethyl)pentan-2-ol Trennung von (+/-)-4-(4-Chlorphenyl)-4-methyl-1-[(2-methylchinolin-5-yl)amino]-2-(trifluormethyl) pentan-2-ol:

Das Enantiomerengemisch wird durch Chromatographie an chiralem Trägermaterial (CHIRALPAK AD^{®}, Fa DAICEL) mit Hexan / Ethanol (97 : 3, vvv) getrennt. Man erhält so das
(-)-Enantiomer: MS (esi): M⁺+1 = 437 (³⁵Cl) 439 (³⁷Cl), [α]_{D} -47.0° (c = 0.75, CHCl₃) und das
(+)-Enantiomer: MS (esi): M⁺+1 = 437 (³⁵Cl) 439 (³⁷Cl).

### Beispiel 82:

### 4-(1,3-Benzodioxol-4-yl)-4-methyl-1-[(chinolin-5-yl)amino]-2-(trifluormethyl)pentan-2-ol

Eine Lösung con 2.44 g 4-(1,3-Benzodioxol-4-yl)-2-hydroxy-4-methyl-2-(trifluormethyl)-pentan-1-ol und 1.72 g 4-Toluolsulfonsäurechlorid in 18 ml Pyridin werden 70 h bei 9°C gerührt. Es wird im Vakuum eingeengt, in 2 N Salzsäure aufgenommen und mit Essigester extrahiert. Es wird mit 2 N Salzsäure und Wasser gewaschen, mit Natriumsulfat getrocknet und im Vakuum eingeengt. Nach Chromatographie an Kieselgel (Hexan / Essigester 100:0 -> 90:10) erhält man 3.97 g 4-Toluolsulfonsäure-[4-(1,3-benzodioxol-4-yl)-2-hydroxy-4-methyl-2-(trifluormethyl)]-pentylester als farbloses Öl. Davon werden 1.35 g in 40 ml THF mit 118 mg Natriumhydrid (60%ig in Öl) versetzt und 4 h auf 60°C erhitzt. Es wird Wasser zugegeben und mit Essigester extrahiert. Es wird mit Wasser und Sole gewaschen, mit Natriumsulfat getrocknet und im Vakuum eingeengt. Nach Chromatographie an Kieselgel (Hexan / Essigester 98:2) erhält man 630 mg 2-[2-(1,3-benzodioxol-4-yl)-2-(methylpropyl)]-2-(trifluormethyl)oxiran als gelbes Öl. Davon werden 230 mg mit 230 mg 5-Aminochinolin in 5 ml DMSO 5 h auf 120°C erhitzt. Es wird Wasser zugegeben und mit Essigester extrahiert. Es wird mit Wasser und Sole gewaschen, mit Natriumsulfat getrocknet und im Vakuum eingeengt. Nach Chromatographie an Kieselgel (Dichlormethan / Aceton 99:1) erhält man 27 mg der Titelverbindung. MS (esi): M⁺+1 = 433.

Nach oben beschriebenen Verfahren wurden folgende Verbindungen der Tabelle II erhalten:

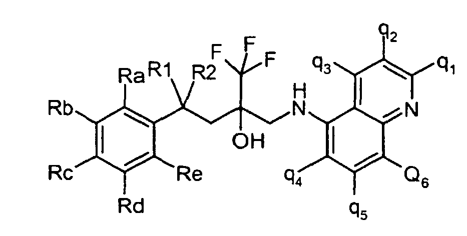

| **Bsp.** | **Synthese Verfahren** | **R1/R2** | **Ra-Re (≠H)¹⁾** | **q1-q6 (≠H)²⁾** | **Analytik³⁾** | **Isomerie** |
|---|---|---|---|---|---|---|
| 83 | Bsp. 76 | CH₃/CH₃ | Ra-Rb = -O-CH₂-O- | q1 = CH₃ | MS: M⁺ = 477 (ei) | Racemat |
| 84 | Bsp. 77,78 | CH₃/CH₃ | Ra-Rb = -O-CH₂-O- | q1 = CH₃ | MS: M⁺ = 477 (ei) | -51.2 (c=1, CHCl₃) |
| 85 | Bsp. 77,78 | CH₃/CH₃ | Ra-Rb = -O-CH₂-O- | q1 = CH₃ | MS: M⁺ = 477 (ei) | +54.7 (c=0.5, CHCl₃) |
| 86 | Bsp. 79 | CH₃/CH₃ | Rb = Cl | | MS: M⁺ = 422 /424(ei) | Racemat |
| 87 | Bsp. 80,81 | CH₃/CH₃ | Rb = Cl | | MS: M⁺ = 422 /424(ei) | -28.1 (c= 0.35, MeOH/CHCl 3) |
| 88 | Bsp. 80,81 | CH₃/CH₃ | Rb = Cl | | MS: M⁺ = 422 /424(ei) | +26.7 (c=0.5, CHCl₃) |
| 89 | Bsp. 79 | CH₃/CH₃ | Rc = Cl | | MS: M⁺+1 = 423 / 425 (esi) | Racemat |
| 90 | Bsp. 79 | CH₃/CH₃ | Ra=Cl | | Fₚ= 186-187°C | Racemat |
| 91 | Bsp. 80,81 | CH₃/CH₃ | Ra = Cl | | MS: M⁺+1 = 423 / 425 (esi) | -26.0 (c=0.5, CHCl₃) |
| 92 | Bsp. 80,81 | CH₃/CH₃ | Ra = Cl | | MS: M⁺ = 422 / 424(ei) | +17.3 (c=1.1, CHCl₃) |
| 93 | Bsp. 79 | CH₃/CH₃ | Ra = Cl | q1 = CH₃ | MS: M⁺ = 436 / 438(ei) | Racemat |
| 94 | Bsp. 80,81 | CH₃/CH₃ | Ra = Cl | q1 = CH₃ | MS: M⁺ = 436 /438(ei) | -15.5 (c=0.5, CHCl₃) |
| 95 | Bsp. 80,81 | CH₃/CH₃ | Ra = Cl | q1 = CH₃ | MS: M⁺ = 436 /438(ei) | +18.7 (c=0.6, CHCl₃) |
| 96 | Bsp. 76 | CH₃/CH₃ | Ra-Rb = -O-CH₂-O-, Rc = Cl | q1 = CH₃ | MS: M⁺ = 480 / 482 (ei) | Racemat |
| 97 | Bsp. 77,78 | CH₃/CH₃ | Ra-Rb = -O-CH₂O-, Rc = Cl | q1 = CH₃ | MS: M⁺ = 480 / 482 (ei) | -29.0 (c=0.45, CHCl₃) |
| 98 | Bsp. 77,78 | CH₃/CH₃ | Ra-Rb = -O-CH₂-O-, Rc = Cl | q1 = CH₃ | MS: M⁺ = 480 / 482 (ei) | (+)-Enantiomer |
| 99 | Bsp. 79 | CH₃/CH₃ | Rb = Cl | q1 = CH₃ | MS: M⁺ = 436 / 438(ei) | Racemat |
| 100 | Bsp. 80,81 | CH₃/CH₃ | Rb = Cl | q1 = CH₃ | MS: M⁺ = 436 / 438(ei) | -34.6 (c=1.0, CHCl₃) |
| 101 | Bsp. 80,81 | CH₃/CH₃ | Rb = Cl | q1 = CH₃ | MS: M⁺ = 436 / 438(ei) | (+)-Enantiomer |
| 102 | Bsp.79 | CH₃/CH₃ | Ra = Rc = Cl | q1 = CH₃ | MS: M⁺ = 470, 472, 474 (ei) | Racemat |
| 103 | Bsp. 80,81 | CH₃/CH₃ | Ra=Rc=Cl | q¹=CH₃ | MS: M⁺ = 470, 472, 474 (ei) | (-)-Enantiomer |
| 104 | Bsp. 80,81 | CH₃/CH₃ | Ra=Rc=Cl | q1=CH₃ | MS: M⁺ = 470, 472, 474 (ei) | +15.7 (c=0.5. CHCl₃) |
| 105 | Bsp. 79 | CH₃/CH₃ | Ra=CF₃, Rd=F | q1=CH₃ | MS: M⁺= 488 (ei) | Racemat |
| 106 | Bsp. 80,81 | CH₃/CH₃ | Ra = CF₃, Rd = F | q1 = CH₃ | MS: M⁺ = 488 (ei) | -20.5 (c=1.0, CHCl₃) |
| 107 | Bsp. 80,81 | CH₃/CH₃ | Ra = CF₃, Rd = F | q1 = CH₃ | MS: M⁺ = 488 (ei) | (+)-Enatiomer |
| 108 | Bsp. 79 | -CH₂-CH₂- | Ra = Cl | q1 = CH₃ | MS: M⁺+1 = 435, 437 (ci) | Racemat |
| 109 | Bsp. 80,81 | -CH₂-CH₂- | Ra = Cl | q1 = CH₃ | MS: M⁺ = 434, 436 (ei) | -6.4 (c=0.5, CHCl₃) |
| 110 | Bsp. 80,81 | -CH₂-CH₂- | Ra = Cl | q1 = CH₃ | MS: M⁺ = 434, 436 (ei) | +5.0 (c=0.5, CHCl₃) |
| 111 | Bsp. 76 | CH₃/CH₃ | Ra = OCH₃, Rc = Cl | q1 = CH₃ | MS: M⁺ = 466, 468 (ei) | Racemat |
| 112 | Bsp. 77,78 | CH₃/CH₃ | Ra=OCH₃, Rc=Cl | q1 = CH₃ | MS: M⁺ = 466, 468 (ei) | (-)-Enantiomer |
| 113 | Bsp. 77,78 | CH₃/CH₃ | Ra = OCH₃, Rc = Cl | q1 = CH₃ | MS: M⁺ = 466, 468 (ei) | +34.1 (c=1.0, CHCl₃) |
| 114 | Bsp. 82 | CH₃/CH₃ | Ra-Rb = -O-CH₂-O- | q1=Cl | MS: M⁺+1= 467, 469 (ci) | Racemat |
| 115 | Bsp. 76 | CH₃/CH₃ | Ra = OCH₃, Rb = Rd = F | q1 = CH₃ | MS: M⁺+1 = 469 (esi) | Racemat |
| 116 | Bsp. 77,78 | CH₃/CH₃ | Ra = OCH₃, Rb = Rd = F | q1 = CH₃ | MS: M⁺ = 468 (ei) | -11.0 (c=0.45, CHCl₃) |
| 117 | Bsp. 77,78 | CH₃/CH₃ | Ra = OCH₃, Rb = Rd = F | q1 = CH₃ | MS: M⁺ = 468 (ei) | (+)-Enantiomer |
| 118 | Bsp. 4 | CH₃/CH₃ | Ra = OH, Rb = Rd = F | q1 = CH₃ | MS: M+1 = 455 (esi) | -10.0 (c=0.3, CHCl₃) |
| 119 | Bsp. 4 | CH₃/CH₃ | Ra = OH, Rb = Rd = F | q1 = CH₃ | MS: M⁺+1 = 455 (esi) | (+)-Enantiomer |
| 120 | Bsp. 4 | CH₃/CH₃ | Ra = OH, Rc = Cl | q1 = CH₃ | Fp.: 107-108°C | (-)-Enantiomer |
| 121 | Bsp. 4 | CH₃/CH₃ | Ra = OH, Rc = Cl | q1 = CH₃ | Fp.: 106-107°C | (+)-Enantiomer |
| 122 | Bsp. 71 | CH₃/CH₃ | Ra = OCH₃, Rd = F | q1 = CH₃, q6 = F | MS: M⁺+1 = 469 (esi) | Racemat |
| 123 | Bsp.4 | CH₃/CH₃ | Ra = OH, Rd = F | q1 = CH₃, q6 = F | MS: M⁺+1 = 455 (esi) | Racemat |
| 124 | Bsp. 79 | CH₃/CH₃ | Ra = OCH₃, Rd = F | q1 = CH₃, q4 = F | MS: M⁺+1 = 469 (esi) | Racemat |
| 125 | Bsp. 4 | CH₃/CH₃ | Ra = OH, Rd = F | q1 = CH₃, q4 = F | MS: M⁺+1 = 455 (esi) | Racemat |
| 126 | Bsp. 79 | CH₃/CH₃ | Ra-Rb = -O-CH₂-O- | q¹ = CH₃, q6 = F | MS: M⁺+1 = 463 (esi) | Racemat |
| 127 | Bsp. 79 | CH₃/CH₃ | Rb = Cl | q1 = OH* | MS: M⁺+1 = 439, 441 (esi) | Racemat |
| 128 | Bsp.79 | -CH₂-CH₂- | Ra=Cl | q1=OH* | MS: M+1 = 435, 437 (ci) | Racemat |
| 129 | Bsp.79 | CH₃/CH₃ | Ra-Rb=-O-CH₂-O- | q1 = OH* | MS:M⁺=448 (ei) | Racemat |
| 130 | Bsp. 79 | CH₃/CH₃ | Ra=OCH₃, Rc=Cl | q1=OH* | MS:M⁺+1= 469, 471 (esi) | Racemat |
| 131 | Bsp. 76 | -CH₂-CH₂- | Ra = Rd = F | q1 = CH₃ | MS: M⁺+1 = 437 (esi) | Racemat |
| 132 | Bsp.79 | CH₃/CH₃ | Ra = Rd = F | q1 = CH₃ | MS: M⁺+1 = 438 (esi) | Racemat |
| 133 | Bsp. 77,78 | CH₃/CH₃ | Ra = Rd = F | q1 = CH₃ | | (-)-Enantiomer |
| 134 | Bsp. 77,78 | CH₃/CH₃ | Ra = Rd = F | q1 = CH₃ | | +27.1 (c=0.33, CHCl₃) |
| 135 | Bsp.79 | -(CH₂)₃- | Ra = Rd = F | q1 = CH₃ | MS: M⁺+1 = 451 (ci) | |
| 136 | Bsp. 79 | -(CH₂)₃- | Ra = Cl, Rd = F | q1 = CH₃ | MS: M⁺+1 = 467 (esi) | Racemat |
| 137 | Bsp. 77,78 | -(CH₂)₃- | Ra = Cl, Rd = F | q1 = CH₃ | | (+)-Enantiomer |
| 138 | Bsp. 77,78 | -(CH₂)₃- | Ra = Cl, Rd = F | q1 = CH₃ | | -29.2 (c=0.61, CHCl₃) |
| 139 | Bsp. 77,78 | CH₃/CH₃ | Ra=OCH₃.Rc=Br | | Fp:131-134°C, MS: 496, 498 (EI⁺) | -32.7° c=0.5, THF |
| 140 | Bsp. 77,78 | CH₃/CH₃ | Ra=OCH₃, Rc=Br | | Fp:132-135°C, MS: 496, 498 (EI⁺) | +36.7° c=0.5, THF |
| 141 | Bsp. 4 | CH₃/CH₃ | Ra=OH, Rc=Br | | Fp: 105°C, MS: 482, 484 (EI⁺) | Racemat |
| 142 | Bsp. 77,78 | CH₃/CH₃ | Ra=OH, Rc=Br | | MS: 482,484 (El+) | (-) Enantiomer |
| 143 | Bsp. 77,78 | CH₃/CH₃ | Ra=OH, Rc=Br | | MS: 482, 484 (EI⁺) | +34.5° c=0.5, THF |
| 144 | Bsp. 76 | CH₃/CH₃ | Ra=OCH₃, Rc=Br | q1=CH₃ | Fp:146-147°C MS:510, 512 (EI⁺) | Racemat |
| 145 | Bsp. 77,78 | CH₃/CH₃ | Ra=OCH₃, Rc=Br | q1=CH₃ | MS:510, 512 (EI⁺) | (-) Enantiomer |
| 146 | Bsp. 77,78 | CH₃/CH₃ | Ra=OCH₃, Rc=Br | q1=CH₃ | MS:510, 512 (El⁺) | +38.5° c=0.5, THF |
| 147 | Bsp. 4 | CH₃/CH₃ | Ra=OH Rc=Br | q1=CH₃ | MS: 496, 498 (EI⁺) | Racemat |
| 148 | Bsp. 77,78 | CH₃/CH₃ | Ra=OH Rc=Br | q1=CH₃ | MS: 496, 498 (EI⁺) | (-) Enantiomer |
| 149 | Bsp. 77,78 | CH₃/CH₃ | Ra=OH Rc=Br | q1=CH₃ | MS: 496, 498 (EI⁺) | +41.2° c=0.5, THF |
| 150 | Bsp. 76 | CH₃/CH₃ | Ra=OCH₃ Rd=Br | | Fp:138°C, MS: 496, 498 (EI⁺) | Racemat |
| 151 | Bsp. 4 | CH₃/CH₃ | Ra=OH, Rd=Br | | MS: 482, 484 | Racemat |
| 152 | Bsp. 77,78 | CH₃/CH₃ | Ra=OH, Rd=Br | | Fp:124-126°C, MS: 482, 484 | (+) Enantiomer |
| 153 | Bsp. 77,78 | CH₃/CH₃ | Ra=OH, Rd=Br | | Fp:124-126°C, MS: 482, 484 | -45.0° c=0.5, THF |
| 154 | Bsp. 76 | CH₃/CH₃ | Ra=OCH₃, Rd=Br | q1=CH₃ | Fp:155°C, MS: 510, 512 (EI⁺) | Racemat |
| 155 | Bsp. 77,78 | CH₃/CH₃ | Ra=OH, Rd=Br | q1=CH₃ | MS: 496, 498 (EI⁺) | (+) Enantiomer |
| 156 | Bsp. 77,78 | CH₃/CH₃ | Ra=OH Rd=Br | q1=CH₃ | MS: 496, 498 (EI⁺) | -42.0° c=0.5, THF |
| 157 | Bsp. 76 | CH₃/CH₃ | Ra=Cl, Rd=F | | Fp:180-182°C, MS: 440, 442 (EI⁺) | Racemat |
| 158 | Bsp. 77,78 | CH₃/CH₃ | Ra=Cl, Rd=F | | Fp:141-142°C, MS: 440, 442 (EI⁺) | (+) Enantiomer |
| 159 | Bsp. 77,78 | CH₃/CH₃ | Ra=Cl, Rd=F | | Fp:142°C, MS: 440, 442 (El⁺ | (-) Enantiomer |
| 160 | Bsp. 76 | CH₃/CH₃ | Ra=Cl, Rd=F | q1=CH₃ | Fp:132-133°C, MS: 454, 456 (El⁺) | Racemat |
| 161 | Bsp. 77,78 | CH₃/CH₃ | Ra=Cl, Rd=F | q1=CH₃ | Fp:178°C, MS: 454, 456 (El⁺) | (+) Enantiomer |
| 162 | Bsp. 77,78 | CH₃/CH₃ | Ra=Cl, Rd=F | q1=CH₃ | Fp:177°C, MS: 454, 456 (El⁺) | -3.4° c=0.5, THF |
| 163 | Bsp. 76 | -CH₂-CH₂. | Ra=Cl, Rd=F | | Fp:171 °C, MS: 438, 440 (EI⁺) | Racemat |
| 164 | Bsp.76 | -CH₂-CH₂. | Ra=Cl, Rd=F | q1=CH₃ | Fp:171°C, MS: 452, 454 (EI⁺) | Racemat |
| 165 | Bsp. 76 | CH₃/CH₃ | Ra=Cl, Rc=F | | Fp:164-166°C, MS: 440(EI⁺) | Racemat |
| 166 | Bsp. 76 | CH₃/CH₃ | Ra=Cl, Rc=F | q1=CH₃ | Fp:128-130°C, MS: 454, 456 (EI⁺) | Racemat |
| 167 | Bsp. 77,78 | CH₃/CH₃ | Ra=CI, Rc=F | q1=CH₃ | Fp:128°C, MS: 454, 456 (EI⁺) | -1.5° c=0.5, THF |
| 168 | Bsp. 77,78 | CH₃/CH₃ | Ra=CI, Rc=F | q1=CH₃ | Fp:128°C, MS: 454, 456 (EI⁺) | (+) Enantiomer |
| 169 | Bsp. 76 | -CH₂-CH₂- | Ra=CI, Rc=F | | Fp:172-173°C, MS: 452, 454 (EI⁺) | Racemat |
| 170 | Bsp. 76 | -(CH₂)₃- | Ra=OCH₃, Rd=F | | Fp:130°C, MS: 448, 449 (EI⁺) | Racemat |
| 171 | Bsp. 77,78 | -(CH₂)₃- | Ra=OCH₃, Rd=F | | Fp:132-134°C, MS: 448, 449 (EI⁺) | -18.0° c=0.5, THF |
| 172 | Bsp. 77,78 | -(CH₂)₃- | Ra=OCH₃, Rd=F | | Fp:133-134°C, MS: 448, 449 (EI⁺) | (+) Enantiomer |
| 173 | Bsp. 4 | -(CH₂)₃- | Ra=OH, Rd=F | | MS: 434, 435 (EI⁺) | Racemat |
| 174 | Bsp. 77,78 | -(CH₂)₃- | Ra=OH, Rd=F | | MS: 434 (EI⁺) | -18.1° c=0.5, THF |
| 175 | Bsp. 77,78 | -(CH₂)₃- | Ra=OH, Rd=F | | MS: 434 (EI⁺) | (+) Enantiomer |
| 176 | Bsp. 76 | -(CH₂)₃- | Ra=OCH₃, Rd=F | | Fp:188°C, MS:462, 463 (El⁺) | Racemat |
| 177 | Bsp. 77,78 | -(CH₂)₃- | Ra=OCH₃, Rd=F | | Fp:188°C, MS:462, 463 (EI⁺) | -13.2° c=0.4, CHCl₃ |
| 178 | Bsp. 77,78 | -(CH₂)₃- | Ra=OCH₃, Rd=F | | Fp:188°C, MS:462, 463 (EI⁺) | (+) Enantiomer |
| 179 | Bsp. 4 | -(CH₂)₃- | Ra=OH, Rd=F | q1=CH₃ | MS: 448, 449 | Racemat |
| 180 | Bsp. 77,78 | -(CH₂)₃- | Ra=OH, Rd=F | q1=CH₃ | MS: 448, 449 | -12.0° c=0.4, CHCl₃ |
| 181 | Bsp. 77,78 | -(CH₂)₃- | Ra=OH, Rd=F | q1=CH₃ | MS: 448, 449 | (+) Enantiomer |
| 182 | Bsp. 82 | CH₃/CH₃ | | q6=Br | Fp:176°C, MS:466, 466 (EI⁺) | Racemat |
| 183 | Bsp. 79 | CH₃/CH₃ | Ra=OCH₃, Rd=F | q6=Br | Fp:200°C, MS:514,516 (El⁺) | Racemat |
| 184 | Bsp. 4 | CH₃/CH₃ | Ra=OH, Rd=F | q6=Br | MS:500, 502 (El⁺) | Racemat |
| 185 | Bsp. 79 | CH₃/CH₃ | Ra=OCH₃, Rd=F | q6=Cl | Fp:188-189°C, MS:470, 472 (EI⁺) | Racemat |
| 186 | Bsp. 4 | CH₃/CH₃ | Ra=OH, Rd=F | q6=Cl | Fp:184°C, MS:456, 458 (EI⁺) | Racemat |
| 187 | Bsp. 79 | CH₃/CH₃ | Ra=OCH₃, Rd=F | q4=Cl | Fp:132°C, MS:470, 472 (EI⁺) | Racemat |
| 188 | Bsp. 4 | CH₃/CH₃ | Ra=OH, Rd=F | q4=Cl | MS:456, 458 (EI⁺) | Racemat |

| | | | | | | |
|---|---|---|---|---|---|---|
| ¹⁾ **Ra-Re (≠H)** bedeutet, das in der Spalte alle die Reste Ra-Re aufgeführt sind, die nicht Wasserstoff bedeuten; alle nicht aufgeführten Reste Ra-Re = Wasserstoff ²⁾ **q1-q6 (≠H)** bedeutet, dass alle Reste q1-q6 aufgeführt sind, die nicht Wasserstoff bedeuten; alle nicht aufgeführten Reste q1-q6 = Wasserstoff. ³⁾Analytik: MS = Massenspektrometrie, Fp. = Schmelzpunkt (Festpunkt) *OH soll darauf hinweisen, dass die Verbindung als Tautomerengleichgewicht vorliegen kann Tabelle II | | | | | | |

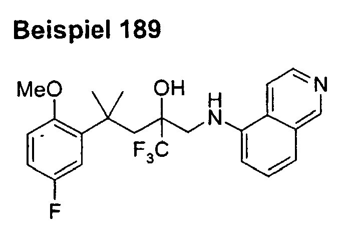

### 4-(5-Fluor-2-methoxyphenyl)-1-(isochinolin-5-ylamino)-4-methyl-2-(trifluormethyl)pentan-2-ol

Analog zu Beispiel 20 werden 500 mg (1.6 mmol) 4-(5-Fluor-2-methoxyphenyl)-2-hydroxy-4-methyl-2-(trifluormethyl)pentanal und 500 mg (1.9 mmol) 5-Aminoisochinolin in 16 ml Essigsäure zum 4-(5-Fluor-2-methoxyphenyl)-4-methyl-1-(isochinolin-5-ylimino)-2-(trifluormethyl)-pentan-2-ol umgesetzt und im Anschluss mit 509 mg (2.4 mmol) Natriumtriacetoxyborhydrid reduziert. Nach Chromatographie an Kieselgel mit Hexan-Essigester (0-60%) werden 221 mg Produkt erhalten.
¹H-NMR (CDCl₃); δ = 1.46 (s, 3H), 1.60 (s, 3H), 2.33 (d, 1 H), 2.81 (d, 1 H), 3.12 (dd, 1H), 3.24 (br, 1H), 3.30 (dd, 1 H), 3.84 (s, 3H), 4.32 (br, 1 H), 6.19 (dd, 1 H), 6.78 (dd, 1 H), 6. 92 (td, 1 H), 7.13 (dd, 1 H), 7.38 (m, 3H), 8.44 (d, 1 H), 9.12 (s, 1 H).

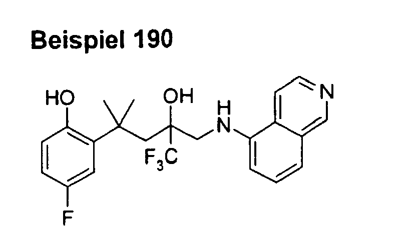

### 4-(5-Fluor-2-hydroxyphenyl)-1-(isochinolin-5-yl)-4-methyl-2-(trifluormethyl)pentan-2-ol

Analog zu Beispiel 2 werden 100 mg (0.15 mmol) 4-(5-Fluor-2-methoxyphenyl)-1-(isochinolin-5-ylamino)-4-methyl-2-(trifluormethyl)pentan-2-ol mit 4.6 ml 1 M Bortribromid-CH₂Cl₂-Lösung umgesetzt. Nach Chromatographie an Kieselgel mit Hexan-Essigester (0-80 %) erhält man 13 mg des Produkts.
'H-NMR (CD₃OD); δ = 1.48 (s, 3H), 1.67 (s, 3H), 1.98 (d, 1 H), 3.00 (d, 1 H), 3.25 (d, 1 H), 3.43 (d, 1 H), 6.22 (dd, 1 H), 6.40 (dd, 1 H), 6.53 (td, 1 H), 7.01 (dd, 1H), 7.34 (m, 2H), 7.71 (d, 1 H), 8.37 (d, 1 H), 9.06 (s, 1 H).

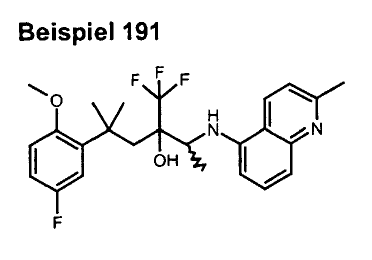

### 5-(5-Fluor-2-methoxyphenyl)-5-methyl-2-(2-methylchinolin-5-ylamino)-3.(trifluormethyl)hexan-3-ol

### 5-(5-Fluor-2-methoxyphennyl)-5-methyl-3-(trifluormethyl)hexan-2,3-diol

Zu der Lösung von 3,6 g (11,7 mmol) 4-(5-Fluor-2-methoxyphenyl)-2-hydroxy-4-methyl-2-(trifluormethyl)pentanal in 150ml Diethylether werden bei +2° C, 8ml 3M Methylmagnesiumchlorid-Tetrahydrofuran-Lösung zugetropft. 1 h bei +2°C und 2h bei Raumtemperatur nachgerührt. Anschließend unter Eiskühlung mit ges. Ammoniumchlorid Lösung hydrolisiert, die organische Phase abgetrennt, über Natriumsulfat getrocknet und eingeengt. Säulenchromatographie an Kieselgel mit Hexan-Essigester liefert 2,23 g des Produktes.

### 5-(5-Fluor-2-methoxyphenyl)-3-hydroxy-5-methyl-3-(trifluormethyl)hexan-2-on

1,46 g (4,5 mmol) 5-(5-Fluor-2-methoxyphenyl)-5-methyl-3-(trifluormethyl)hexan-2,3-diol und 3,14 ml (22,5 mmol) Triethylamin in 16,3 ml DMSO und 50 ml Methylenchlorid werden bei Raumtemperatur portionsweise mit 4,3 g (27 mmol) Pyridin-Schwefeltrioxid-Komplex versetzt. Nach 20 h bei Raumtemperatur wird unter Eiskühlung mit ges. Ammoniumchlorid Lösung hydrolisiert und mit Diethylether extrahiert. Die vereinigten Extrakte werden getrocknet (Natriumsulfat ) und eingeengt. Säulenchromatographie an Kieselgel mit Hexan-Essigester liefert 1,04 g des Produktes.

### 5-(5-Fluor-2-methoxyphenyl)-5-methyl-2-(2-methylchinolin-5-ylimino)-3-(trifluormethyl)hexan-3-ol

645 mg (2 mmol ) 5-(5-Fluor-2-methoxyphenyl)- 3-hydroxy-5-methyl-3-(trifluormethyl) hexan-2-on werden in 4 ml Tetrahydrofuran unter Stickstoff mit 0,84 ml (4 mmol ) Titantetraethylat und 348 mg (2,2 mmol) 5-Amino-2-methylchinolin 24 h unter Rückfluß erhitzt. Nach Abkühlen auf Raumtemperatur wird das Reaktionsgemisch in 20 ml ges. NaCl-Lösung eingerührt, 50 ml Essigester zugegeben und 30 min. gerührt, über Celite abgesaugt, mit Essigester und mit Tetrahydrofuran nachgewaschen. Die organische Phase wird abgetrennt, getrocknet (Natriumsulfat) und eingeengt. Chromatographie an Kieselgel mit Hexan-Essigester liefert 578 mg des Produktes

### 5-(5-Fluor-2-methoxyphenyl)-5-methyl-2-(2-rnethylchinolin-5-ylamino)-3-(trifluormethyl)hexan-3-ol

231 mg (0,5 mmol) 5-(5-Fluor-2-methoxyphenyl)-5-methyl-2-(2-methylchinolin-5-ylimino)-3-(trifluromethyl)hexan-3-ol werden in 10 ml Tetrahydrofuran und 4 ml Ethanol mit 0,21 ml (1 mmol) Titantetraethylat versetzt. Anschließend werden 875 mg (23,1 mmol) Natriumborhydrid portionsweise innerhalb von 4 Tagen bei 65° C Reaktionstemperatur zugegeben. Das Reaktionsgemisch wird nach abkühlen auf RT mit 20 ml ges. NaCl-Lösung versetzt, 1 h bei RT gerührt, über Celite filtriert, mit Essigester und Tetrahydrofuran nachgewaschen. Die organische Phase wird abgetrennt, mit gesättigter NaCl-Lösung gewaschen, getrocknet und eingeengt. Säulenchromatographie an Kieselgel mit Hexan-Essigester liefert 68 mg des Produktes als Gemisch der beiden möglichen Diastereoisomere.

## Patentansprüche

1. Verbindungen der allgemeinen Formel 1 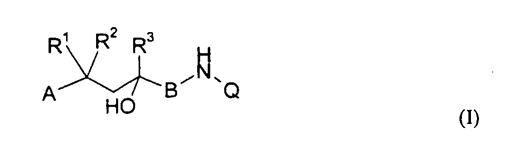 worin
A für eine Aryl-, eine Benzyl- oder eine Phenethylgruppe steht, wobei die Aryl-, Benzyl- oder Phenethylgruppe gegebenenfalls substituiert sein kann durch einen oder mehrere Reste aus der Gruppe C₁-C₅-Alky), C₁-C₅-Alkoxy, C₁-C₅-Alkylthio, C₁-C₅-Perfluoralkyl, Halogen, Hydroxy, Cyano, Nitro, -O-(CH₂)ₙ-O-, -O-(CH₂)ₙ-CH₂-, -O-CH=CH-, -(CH₂)ₙ₊₂-wobei n = 1 oder 2 ist und die endständigen Sauerstoffatome und/oder Kohlenstoffatome mit direkt benachbarten Ring-Kohlenstoffatomen verknüpft sind,
oder NR⁴R⁵,
wobei R⁴ und R⁵ unabhängig voneinander Wasserstoff, C₁-C₅-Alkyl oder (CO)-C₁-C₅-Alkyl sein können,
R¹ und R² unabhängig voneinander ein Wasserstoffatom, eine Methyl- oder Ethylgruppe oder gemeinsam mit dem Kohlenstoffatom der Kette einen C₃-C₆-Cycloalkylring,
R³ eine C₁-C₃-Alkylgruppe oder eine gegebenenfalls teilweise oder vollständig fluorierte C₁-C₃-Alkylgruppe,
B eine gegebenenfalls durch eine Methyl- oder Ethylgruppe substituierte Methylengruppe oder eine Carbonylgruppe und
Q eine über eine beliebige Position verknüpfte Chinolinyl- oder Isochinolinylgruppe bedeutet, die gegebenenfalls substituiert sein kann durch einen oder mehrere Reste aus der Gruppe C₁-C₅-Alkyl, die gegebenenfalls substituiert sein kann durch 1-3 Hydroxygruppen und/oder 1-3 COOR⁶-Gruppen, C₁-C₅-Alkoxy, C₁-C₅-Alkylthio, C₁-C₅-Perfluoralkyl, Halogen, Hydroxy, ein Carbonylsauerstoffatom, Cyano, Nitro, oder NR⁴R⁵,
wobei R⁴ und R⁵ unabhängig voneinander Wasserstoff, C₁-C₅-Alkyl oder (CO)-C₁-C₅-Alkyl sein können,
COOR⁶, wobei R⁶ Wasserstoff oder eine C₁-C₅-Alkylgruppe bedeuten,
(CO)NR⁷R⁸, wobei R⁷ und R⁸ unabhängig voneinander Wasserstoff oder eine C₁-C₅-Alkylgruppe bedeuten, oder (C₁-C₅-Alkylen)-O-(CO)-(C₁-C₅)alkyl sowie deren Racemate oder getrennt vorliegenden Stereoisomeren, und gegebenenfalls deren physiologisch verträgliche Salze.

2. Verbindungen gemäß Anspruch 1, worin Q eine über eine beliebige Position verknüpfte Chinolinyl- oder lsochinolinylgruppe bedeutet, die gegebenenfalls substituiert sein kann durch einen oder mehrere Reste aus der Gruppe C₁-C₅-Alkyl, die gegebenenfalls substituiert sein kann durch 1-3 Hydroxygruppen und/oder 1-3 COOR⁶-Gruppen,
ein Carbonylsauerstoffatom,
COOR⁶, wobei R⁶ Wasserstoff oder eine C₁-C₅-Alkylgruppe bedeuten, (CO)NR⁷R^{B}, wobei R⁷ und R⁸ unabhängig voneinander Wasserstoff oder eine C₁-C₅-Alkylgruppe bedeuten,
oder eine (C₁-C₅-Alkylen)-O-(CO)-(C₁-C₅)alkylgruppe bedeutet.

3. Verbindungen der allgemeinen Formel I gemäß Anspruch 1 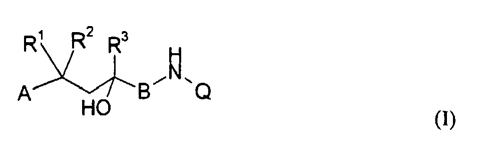 worin
A für eine Aryl-, eine Benzyl- oder eine Phenethylgruppe steht, wobei die Aryl-, Benzyl- oder Phenethylgruppe gegebenenfalls substituiert sein kann durch einen oder mehrere Reste aus der Gruppe C₁-C₅-Alkyl, C₁-C₅-Alkoxy, C₁-C₅-Alkylthio, C₁-C₅-Perfluoralkyl, Halogen, Hydroxy, Cyano, Nitro, -O-(CH₂)ₙ-O-, -O-(CH₂)ₙ-CH₂-, -O-CH=CH-, -(CH₂)ₙ₊₂-wobei n = 1 oder 2 ist und die endständigen Sauerstoffatome und/oder Kohlenstoffatome mit direkt benachbarten Ring-Kohlenstoffatomen verknüpft sind, oder NR⁴R⁵,
wobei R⁴ und R⁵ unabhängig voneinander Wasserstoff, C₁-C₅-Alkyl oder (CO)-C₁-C₅-Alkyl sein können,
R¹ und R² unabhängig voneinander ein Wasserstoffatom, eine Methyl- oder Ethylgruppe oder gemeinsam mit dem Kohlenstoffatom der Kette einen C₃-C₆-Cycloalkylring,
R³ eine C₁-C₃-Alkylgruppe oder eine gegebenenfalls teilweise oder vollständig fluorierte C₁-C₃-Alkylgruppe,
B eine gegebenenfalls durch eine Methyl- oder Ethylgruppe substituierte Methylengruppe oder eine Carbonylgruppe und
Q eine über eine beliebige Position verknüpfte Chinolinyl- oder Isochinolinylgruppe bedeutet, die gegebenenfalls substituiert sein kann durch einen oder mehrere Reste aus der Gruppe C₁-C₅-Alkyl, C₁-C₅-Alkoxy, C₁-C₅-Alkylthio, C₁-C₅-Perfluoralkyl, Halogen, Hydroxy, Cyano, Nitro, oder NR⁴R⁵,
wobei R⁴ und R⁵ unabhängig voneinander Wasserstoff, C₁-C₅-Alkyl oder (CO)-C₁-C₅-Alkyl sein können sowie deren Racemate oder getrennt vorliegenden Stereoisomeren, und gegebenenfalls deren physiologisch verträgliche Salze.

4. Verbindungen gemäß Anspruch 2 oder 3, wobei B eine Methylengruppe bedeutet.

5. Verbindungen gemäß Anspruch 2 oder 3, wobei A ein Arylrest ist.

6. Verbindungen gemäß Anspruch 5, wobei A ein Arylrest ist, der gegebenenfalls substituiert sein kann durch einen oder mehrere Reste aus der Gruppe C₁-C₅-Alkyl, C₁-C₅-Alkoxy, C₁-C₅-Perfluoralkyl, Halogen, Hydroxy, Nitro, -O-(CH₂)ₙ-O-, -O-(CH₂)ₙ-CH₂-, -O-CH=CH-, -(CH₂)ₙ₊₂-
wobei n = 1 oder 2 ist und die endständigen Sauerstoffatome und/oder Kohlenstoffatome mit direkt benachbarten Ring-Kohlenstoffatomen verknüpft sind.

7. Verbindungen gemäß Anspruch 2 oder 3, wobei R¹ und R² gemeinsam mit dem Kohlenstoffatom der Kette einen C₃-C₆-Cycloalkylring bedeuten.

8. Verbindungen gemäß Anspruch 2 oder 3, **dadurch gekennzeichnet, dass** Q eine über eine beliebige Position verknüpfte gegebenenfalls substituierte Chinolinylgruppe bedeutet.

9. Verbindungen gemäß Anspruch 5, **dadurch gekennzeichnet, dass** A ein durch eine Hydroxygruppe oder eine Methoxygruppe und ein Halogenatom substituierter Phenylrest ist.

10. Verbindungen gemäß Anspruch 1, **dadurch gekennzeichnet, dass** es sich um die (+)-Enantiomeren handelt.

11. Verbindungen gemäß Anspruch 1, **dadurch gekennzeichnet, dass** es sich um die (-)-Enantiomeren handelt.

12. Verwendung der Verbindungen gemäß Anspruch 1 zur Herstellung von Arzneimitteln.

13. Verwendung der Verbindungen gemäß Anspruch 1 zur Herstellung eines Arzneimittels zur Behandlung von entzündlichen Erkrankungen.

14. Pharmazeutische Präparate enthaltend mindestens eine Verbindung nach Anspruch 1 oder deren Gemische sowie pharmazeutisch verträgliche Träger.

15. Verfahren zur Herstellung der Verbindungen der allgemeinen Formel I gemäß Anspruch 1, **dadurch gekennzeichnet, dass** für den Fall, dass B = CO ist,
entweder
A1)
eine α-Ketosäure der Formel (II) 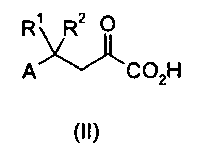 worin A, R¹ und R² die in Anspruch 1 angegebenen Bedeutungen haben,
mit einem Amin der Formel
Q-NH₂
worin Q ein gegebenenfalls substituiertes Chinolin, Isochinolin oder (partial)-hydriertes Chinolin oder (partial)-hydriertes Isochinolin gemäß Anspruch 1 bedeutet,
zum α-Ketoamid (III) 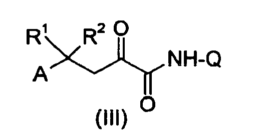 worin worin A, R¹, R² und Q die in Anspruch 1 angegebenen Bedeutungen haben,
gegebenenfalls in Gegenwart von dehydratisierenden
Kupplungsreagenzien oder nach Überführung der Säurefunktion in ein Säurechlorid in dem Fachmann bekannter Weise,
umgesetzt wird,
das anschließend
entweder
mit einer Alkylmetallverbindung
oder
mit einer Siliziumverbindung der Formel (IV)
(R⁹)₃-Si-R³ (IV)
worin R³ für eine C₁-C₃-Alkylgruppe oder eine gegebenenfalls teilweise oder vollständig fluorierte C₁-C₃-Alkylgruppe steht, und R⁹ eine C₁-C₅-Alkylgruppe bedeutet,
in Gegenwart eines Katalysators zu Verbindungen der allgemeinen Formel (I) umgesetzt wird, oder
A2)
eine α-Ketosäure (II) 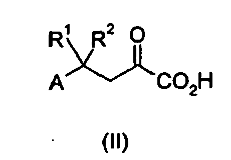 nach dem Fachmann bekannter Methode verestert wird, der erhaltene α-Ketoester (V) 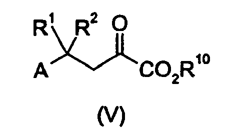 worin A, R¹ und R² die oben genannten Bedeutungen haben und R¹⁰ C₁-C₄-Alkyl bedeutet,
dann wie in A1) beschrieben mit einer Alkylmetallverbindung oder einer Siliziumverbindung der Formel (IV) zu einer Verbindung der allgemeinen Formel (VI) 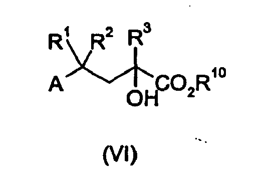 worin R¹⁰ C₁-C₄-Alkyl bedeutet,
umgesetzt wird,
gegebenenfalls der Ester nach dem Fachmann bekannter Methode verseift wird, und die Verbindung der Formel (VI)
dann mit einem Amin der Formel
Q-NH₂
worin Q Chinolin, Isochinolin oder (partial)-hydriertes Chinolin oder (partial)-hydriertes Isochinolin bedeutet,
unter Verwendung eines Aktivierungsreagenzes, gegebenenfalls in Gegenwart eines Katalysators
zu einer Verbindung der Formel (I) umgesetzt wird,
oder
für den Fall, dass B eine gegebenenfalls durch eine Methyl- oder Ethylgruppe substituierte Methylengruppe bedeutet,
B1)
entweder
eine Verbindung der Formel (VII) oder (VIII) 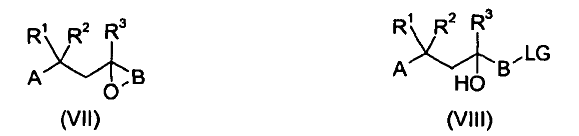 worin A, R¹, R² und R³ die oben angegebenen Bedeutungen haben, B für eine gegebenenfalls durch eine Methyl- oder Ethylgruppe substituierte Methylengruppe und LG für eine beliebige Fluchtgruppe steht,
mit einer Verbindung der Formel (IX) oder (X) 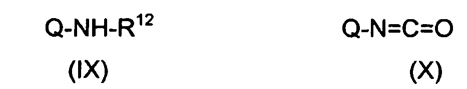 worin R¹² ein Wasserstoffatom, eine C₁-C₅-Acylgruppe oder eine Alkoxygruppe oder eine Aryloxycarbonylgruppe bedeutet und Q ein Chinolin, Isochinolin oder (partial)-hydriertes Chinolin oder (partial)-hydriertes Isochinolin bedeutet,
umgesetzt wird und ein gegebenenfalls intermediär gebildetes Oxazolidinon gespalten wird, um eine Verbindung der Formel (I) zu erhalten, oder
B2)
Verbindungen der Formeln (VII) oder (VIII) mit Stickstoffnukleophilen umgesetzt werden,
und gegebenenfalls anschließend mit dem Fachmann bekannten Reagenzien reduziert wird oder eine Übergangsmetall-katalysierte Hydrogenolyse durchgeführt wird, um Verbindungen der Formel (XI) 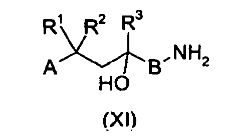 zu erhalten,
wobei die Reste A, B, R¹, R² und R³ die oben angegebenen Bedeutungen haben
die dann gegebenenfalls unter Basenkatalyse oder
Übergangsmetallkatalyse mit einem nach dem Fachmann bekannter
Methode halogenierten Derivat des Chinolins, Isochinolins oder (partial)-hydrierten Chinolins oder (partial)-hydrierten Isochinolins
umgesetzt wird, oder
B3)
indem nach dem Fachmann bekannten Methoden hergestellte Verbindungen der Formel (XII) 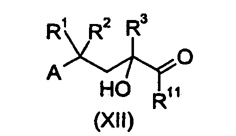 worin die Reste A, R¹, R² und R³ die oben angegebenen Bedeutungen haben und R¹¹ Methyl oder Ethyl bedeuten,
unter Bedingungen der reduktiven Aminierung mit Verbindungen der Formel
Q-NH₂
worin Q die oben angegebenen Bedeutung hat,
zu Verbindungen der allgemeinen Formel (I) umgesetzt werden.

## Claims

1. Compounds of the general formula I 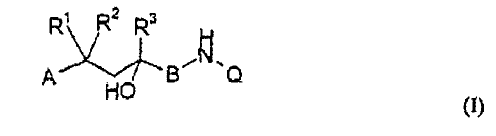 wherein
A stands for an aryl group, a benzyl group or a phenethyl group, which aryl, benzyl or phenethyl group optionally can be substituted by one or more radicals from the group C₁-C₅-alkyl, C₁-C₅-alkoxy, C₁-C₅-alkylthio, C₁-C₅-perfluoroalkyl, halogen, hydroxy, cyano, nitro, -O-(CH₂)ₙ-O-, -O-(CH₂)ₙ-CH₂-, -O-CH=CH-, -(CH₂)ₙ₊₂-, where n = 1 or 2 and the terminal oxygen atoms and/or carbon atoms are linked to directly adjacent ring-carbon atoms,
or NR⁴R⁵,
where R⁴ and R⁵, independently of one another, can be hydrogen, C₁-C₅-alkyl or (CO)-C₁-C₅-alkyl,
R¹ and R², independently of one another, mean a hydrogen atom, a methyl or ethyl group or together with the carbon atom of the chain a C₃-C₆-cycloalkyl ring,
R³ means a C₁-C₃-alkyl group or an optionally partially or completely fluorinated C₁-C₃-alkyl group,
B means an optionally methyl- or ethyl-substituted methylene group or a carbonyl group and
Q means a quinolinyl or isoquinolinyl group that is linked via any position and that optionally can be substituted by one or more radicals from the group
C₁-C₅-alkyl, which optionally can be substituted by 1-3 hydroxy groups and/or 1-3 COOR⁶ groups,
C₁-C₅-alkoxy, C₁-C₅-alkylthio, C₁-C₅-perfluoroalkyl, halogen, hydroxy, a carbonyl-oxygen atom, cyano, nitro or NR⁴R⁵,
where R⁴ and R⁵, independently of one another, can be hydrogen, C₁-C₅-alkyl or (CO)-C₁-C₅-alkyl,
COOR⁶, where R⁶ means hydrogen or a C₁-C₅-alkyl group,
(CO)NR⁷R⁸, where R⁷ and R⁸, independently of one another, mean hydrogen or a C₁-C₅-alkyl group,
or (C₁-C₅-alkylene)-O-(CO)-(C₁-C₅)alkyl
and also their racemates or separately present stereoisomers, and if appropriate their physiologically compatible salts.

2. Compounds according to Claim 1, wherein Q means a quinolinyl or isoquinolinyl group that is linked via any position and that optionally can be substituted by one or more radicals from the group
C₁-C₅-alkyl, which optionally can be substituted by 1-3 hydroxy groups and/or 1-3 COOR⁶ groups,
a carbonyl-oxygen atom,
COOR⁶, where R⁶ means hydrogen or a C₁-C₅-alkyl group,
(CO)NR⁷R⁸, where R⁷ and R⁸, independently of one another, mean hydrogen or a C₁-C₅-alkyl group,
or a (C₁-C₅-alkylene)-O-(CO)-(C₁-C₅)alkyl group.

3. Compounds of the general formula I according to Claim 1 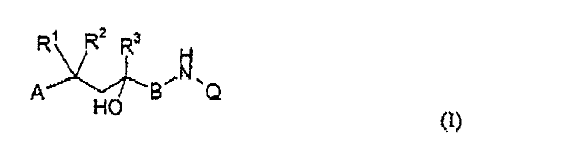 wherein
A stands for an aryl group, a benzyl group or a phenethyl group, which aryl, benzyl or phenethyl group optionally can be substituted by one or more radicals from the group C₁-C₅-alkyl, C₁-C₅-alkoxy, C₁-C₅-alkylthio, C₁-C₅-perfluoroalkyl, halogen, hydroxy, cyano, nitro, -O-(CH₂)ₙ-O-, -O-(CH₂)ₙ-CH₂-, -O-CH=CH-, - (CH₂) ₙ₊₂-, where n = 1 or 2 and the terminal oxygen atoms and/or carbon atoms are linked to directly adjacent ring-carbon atoms,
or NR⁴R⁵,
where R⁴ and R⁵, independently of one another,
can be hydrogen, C₁-C₅-alkyl or (CO)-C₁-C₅-alkyl,
R¹ and R², independently of one another, mean a hydrogen atom, a methyl or ethyl group or together with the carbon atom of the chain a C₃-C₆-cycloalkyl ring,
R³ means a C₁-C₃-alkyl group or an optionally partially or completely fluorinated C₁-C₃-alkyl group,
B means an optionally methyl- or ethyl-substituted methylene group or a carbonyl group and
Q means a quinolinyl or isoquinolinyl group that is linked via any position and that optionally can be substituted by one or more radicals from the group C₁-C₅-alkyl, C₁-C₅-alkoxy, C₁-C₅-alkylthio, C₁-C₅-perfluoroalkyl, halogen, hydroxy, cyano, nitro or NR⁴R⁵,
where R⁴ and R⁵, independently of one another, can be hydrogen, C₁-C₅-alkyl or (CO) -C₁-C₅-alkyl,
and also their racemates or separately present stereoisomers, and if appropriate their physiologically compatible salts.

4. Compounds according to Claim 2 or 3 where B means a methylene group.

5. Compounds according to Claim 2 or 3 where A is an aryl radical.

6. Compounds according to Claim 5 where A is an aryl radical which optionally can be substituted by one or more radicals from the group C₁-C₅-alkyl, C₁-C₅-alkoxy, C₁-C₅-perfluoroalkyl, halogen, hydroxy, nitro, -O- (CH₂)ₙ-O-, -O-(CH2)ₙ-CH₂-, -O-CH=CH-, -(CH₂)ₙ₊₂-, where n = 1 or 2 and the terminal oxygen atoms and/or carbon atoms are linked to directly adjacent ring-carbon atoms.

7. Compounds according to Claim 2 or 3 where R¹ and R² together with the carbon atom of the chain mean a C₃-C₆-cycloalkyl ring.

8. Compounds according to Claim 2 or 3, **characterized in that** Q means an optionally substituted quinolinyl group linked via any position.

9. Compounds according to Claim 5, **characterized in that** A is a phenyl radical that is substituted by a hydroxy group or a methoxy group and a halogen atom.

10. Compounds according to Claim 1, **characterized in that** they are the (+)-enantiomers.

11. Compounds according to Claim 1, **characterized in that** they are the (-)-enantiomers.

12. Use of the compounds according to Claim 1 in the manufacture of pharmaceutical agents.

13. Use of the compounds according to Claim 1 in the manufacture of a pharmaceutical agent for treating inflammatory diseases.

14. Pharmaceutical preparations that contain at least one compound according to Claim 1 or mixtures thereof and also pharmaceutically compatible vehicles.

15. Process for producing the compounds of the general formula I according to Claim 1, **characterized in that** for B = CO
either
A1)
an α-keto acid of the formula (II) 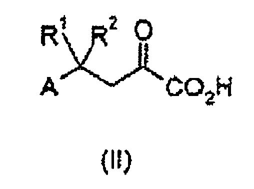 wherein A, R¹ and R² are each as defined in Claim 1,
is reacted with an amine of the formula
Q-NH₂
wherein Q means an optionally substituted quinoline, isoquinoline or (partially) hydrogenated quinoline or (partially) hydrogenated isoquinoline according to Claim 1,
to form the α-keto amide (III) 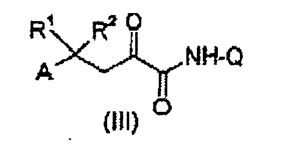 wherein A, R¹, R² and Q are each as defined in Claim 1,
if appropriate in the presence of dehydrating coupling reagents or after conversion of the acid function into an acid chloride in a conventional manner,
which is subsequently reacted
either
with an alkyl metal compound
or
with a silicon compound of the formula (IV)
(R⁹)₃-Si-R³ (IV)
wherein R³ stands for a C₁-C₃-alkyl group or an optionally partially or completely fluorinated C₁-C₃-alkyl group,
and R⁹ means a C₁-C₅-alkyl group,
in the present of a catalyst to form compounds of the general formula (I),
or
A2)
an α-keto acid (II) 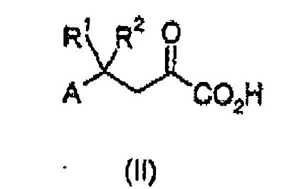 is esterified by a conventional method, the resulting α-keto ester (V) 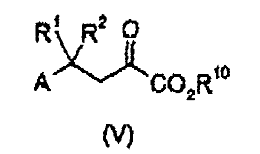 wherein A, R¹ and R² are each as defined above and R¹⁰ means C₁-C₄-alkyl,
is then reacted as described in A1) with an alkyl metal compound or a silicon compound of the formula (IV) to form a compound of the general formula (VI) 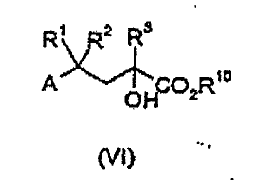 wherein R¹⁰ means C₁-C₄-alkyl,
if appropriate the ester is saponified by a conventional method, and the compound of the formula (VI)
is then reacted with an amine of the formula
Q-NH₂
wherein Q means quinoline, isoquinoline or (partially) hydrogenated quinoline or (partially) hydrogenated isoquinoline,
by using an activating reagent, if appropriate in the presence of a catalyst,
to form a compound of the formula (I),
or
for B meaning an optionally methyl- or ethyl-substituted methylene group,
B1)
either
a compound of the formula (VII) or (VIII) 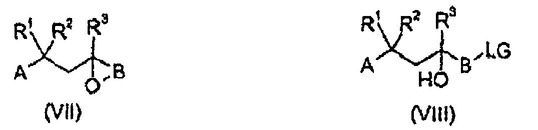 wherein A, R¹, R² and R³ are each as defined above, B stands for an optionally methyl- or ethyl-substituted methylene group and LG stands for any leaving group,
is reacted with a compound of the formula (IX) or (X) 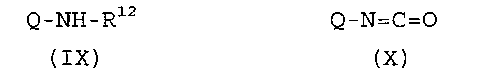 where R¹² means a hydrogen atom, a C₁-C₅-acyl group or an alkoxy group or an aryloxycarbonyl group and Q means a quinoline, isoquinoline or (partially) hydrogenated quinoline or (partially) hydrogenated isoquinoline,
and any oxazolidinone intermediate formed is cleaved to obtain a compound of the formula (I),
or
B2) compounds of the formulae (VII) or (VIII) are reacted with nitrogen nucleophiles,
and if appropriate is subsequently reduced using conventional reagents or a transition metal catalysed hydrogenolysis is carried out to obtain compounds of the formula (XI) 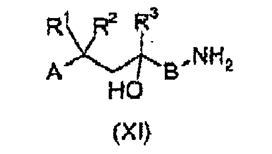 where the radicals A, B, R¹, R² and R³ are each as defined above,
which is then reacted if appropriate under base catalysis or
transition metal catalysis with a conventionally halogenated derivative of quinoline, of isoquinoline or of (partially) hydrogenated quinoline or of (partially) hydrogenated isoquinoline, or
B3) by conventionally produced compounds of the formula (XII) 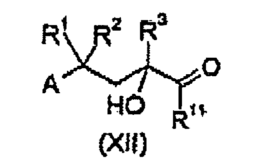 wherein the radicals A, R¹, R² and R³ are each as defined above and R¹¹ means methyl or ethyl,
being reacted with compounds of the formula
Q-NH₂
wherein Q is as defined above,
under conditions of reductive amination to form compounds of the general formula (I).

## Revendications

1. Composés de formule générale I 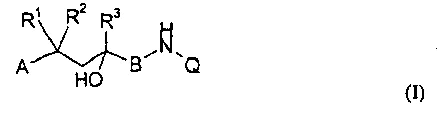 dans laquelle
A représente un groupe aryle, benzyle ou phénéthyle, où le groupe aryle, benzyle ou phénéthyle peut le cas échéant être substitué par un ou plusieurs radicaux du groupe formé par C₁-C₅-alkyle, C₁-C₅-alcoxy, C₁-C₅-alkylthio, C₁-C₅-perfluoroalkyle, halogène, hydroxy, cyano, nitro, -O-(CH₂)ₙ-O-, -O-(CH₂)ₙ-CH₂-, -O-CH=CH-, -(CH₂)ₙ₊₂-, où n = 1 ou 2 et les atomes d'oxygène et/ou les atomes de carbone en position terminale sont liés avec des atomes de carbone cycliques directement adjacents,
ou NR⁴R⁵
où R⁴ et R⁵ peuvent représenter, indépendamment l'un de l'autre, hydrogène, C₁-C₅-alkyle ou (CO)-C₁-C₅-alkyle
R¹ et R² signifient, indépendamment l'un de l'autre, un atome d'hydrogène, un groupe méthyle ou éthyle ou, avec l'atome de carbone de la chaîne, un cycle C₃-C₆-cycloalkyle,
R³ signifie un groupe C₁-C₃-alkyle ou un groupe C₁-C₃-alkyle le cas échéant partiellement ou complètement fluoré,
B signifie un groupe méthylène le cas échéant substitué par un groupe méthyle ou éthyle ou un groupe carbonyle et
Q signifie un groupe quinoléinyle ou isoquinoléinyle, lié via une position quelconque qui peut le cas échéant être substitué par un ou plusieurs radicaux du groupe formé par
C₁-C₅-alkyle, qui peut le cas échéant être substitué par 1-3 groupes hydroxy et/ou 1-3 groupes COOR⁶,
C₁-C₅-alcoxy, C₁-C₅-alkylthio, C₁-C₅-perfluoroalkyle, halogène, hydroxy, un atome d'oxygène carbonyle, cyano, nitro, ou NR⁴R⁵, où R⁴ et R⁵ peuvent représenter, indépendamment l'un de l'autre, hydrogène, C₁-C₅-alkyle ou (CO)-C₁-C₅-alkyle,
COOR⁶, où R⁶ signifie hydrogène ou un groupe C₁-C₅-alkyle,
(CO)NR⁷R⁸, où R⁷ et R⁸ signifient indépendamment l'un de l'autre hydrogène ou un groupe C₁-C₅-alkyle,
ou (C₁-C₅-alkylène) -O- (CO) - (C₁-C₅) alkyle
ainsi que leurs racémates ou leurs stéréo-isomères séparés et le cas échéant leurs sels physiologiquement acceptables.

2. Composés selon la revendication 1, dans lesquels Q signifie un groupe quinoléinyle ou isoquinoléinyle lié via une position quelconque, qui peut le cas échéant être substitué par un ou plusieurs radicaux du groupe formé par
C₁-C₅-alkyle, qui peut le cas échéant être substitué par 1-3 groupes hydroxy et/ou 1-3 groupes COOR⁶,
un atome d'oxygène carbonyle,
COOR⁶, où R⁶ signifie hydrogène ou un groupe C₁-C₅-alkyle,
(CO)NR⁷R⁸, où R⁷ et R⁸ signifient indépendamment l'un de l'autre hydrogène ou un groupe C₁-C₅-alkyle,
ou un groupe (C₁-C₅-alkylène)-O-(CO)-(C₁-C₅) alkyle.

3. Composés de formule générale I selon la revendication 1 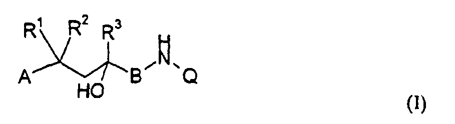 dans laquelle
A représente un groupe aryle, benzyle ou phénéthyle, où le groupe aryle, benzyle ou phénéthyle peut le cas échéant être substitué par un ou plusieurs radicaux du groupe formé par C₁-C₅-alkyle, C₁-C₅-alcoxy, C₁-C₅-alkylthio, C₁-C₅-perfluoroalkyle, halogène, hydroxy, cyano, nitro, -O-(CH₂)ₙ-O-, -O-(CH₂)ₙ-CH₂-, -O-CH=CH-, - (CH₂)ₙ₊₂-, où n = 1 ou 2 et les atomes d'oxygène et/ou les atomes de carbone en position terminale sont liés avec des atomes de carbone cycliques directement adjacents,
ou NR⁴R⁵,
où R⁴ et R⁵ peuvent représenter, indépendamment l'un de l'autre, hydrogène, C₁-C₅-alkyle ou (CO)-C₁-C₅-alkyle
R¹ et R² signifient, indépendamment l'un de l'autre, un atome d'hydrogène, un groupe méthyle ou éthyle ou, avec l'atome de carbone de la chaîne, un cycle C₃-C₆-cycloalkyle,
R³ signifie un groupe C₁-C₃-alkyle ou un groupe C₁-C₃-alkyle le cas échéant partiellement ou complètement fluoré,
B signifie un groupe méthylène le cas échéant substitué par un groupe méthyle ou éthyle ou un groupe carbonyle et
Q signifie un groupe quinoléinyle ou isoquinoléinyle lié via une position quelconque, qui peut le cas échéant être substitué par un ou plusieurs radicaux du groupe C₁-C₅-alkyle, C₁-C₅-alcoxy, C₁-C₅-alkylthio, C₁-C₅-perfluoroalkyle, halogène, hydroxy, cyano, nitro, ou NR⁴R⁵,
où R⁴ et R⁵ peuvent représenter, indépendamment l'un de l'autre, hydrogène, C₁-C₅-alkyle ou (CO)-C₁-C₅-alkyle
ainsi que leurs racémates ou leurs stéréo-isomères séparés et le cas échéant leurs sels physiologiquement acceptables.

4. Composés selon la revendication 2 ou 3, où B signifie un groupe méthylène.

5. Composés selon la revendication 2 ou 3, où A représente un radical aryle.

6. Composés selon la revendication 5, où A représente un radical aryle, qui peut le cas échéant être substitué par un ou plusieurs radicaux du groupe formé par C₁-C₅-alkyle, C₁-C₅-alcoxy, C₁-C₅-perfluoroalkyle, halogène, hydroxy, nitro, -O-(CH₂)ₙ-O-, -O- (CH₂)ₙ-CH₂-, -O-CH=CH-, - (CH₂) ₙ₊₂-, où n = 1 ou 2 et les atomes d'oxygène et/ou les atomes de carbone en position terminale sont liés aux atomes de carbone de cycle directement adjacents.

7. Composés selon la revendication 2 ou 3, où R¹ et R² signifient, avec l'atome de carbone de la chaîne, un cycle C₃-C₆-cycloalkyle.

8. Composés selon la revendication 2 ou 3, **caractérisés en ce que** Q signifie un groupe quinoléyle le cas échéant substitué lié via une quelconque position.

9. Composés selon la revendication 5, **caractérisés en ce que** A représente un radical phényle substitué par un groupe hydroxy ou un groupe méthoxy et un atome d'halogène.

10. Composés selon la revendication 1, **caractérisés en ce qu'**il s'agit des énantiomères (+).

11. Composés selon la revendication 1, **caractérisés en ce qu'**il s'agit des énantiomères (-).

12. Utilisation des composés selon la revendication 1 pour la préparation de médicaments.

13. Utilisation des composés selon la revendication 1 pour la préparation d'un médicament destiné au traitement de maladies inflammatoires.

14. Préparations pharmaceutiques contenant au moins un composé selon la revendication 1 ou leurs mélanges ainsi que des supports pharmaceutiquement acceptables.

15. Procédé pour la préparation de composés de formule générale I selon la revendication 1, **caractérisé en ce que**,
pour le cas où B = CO,
soit
A1)
on transforme un α-cétoacide de formule (II) 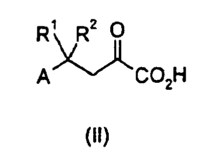 dans laquelle A, R¹ et R² ont les significations indiquées dans la revendication 1,
avec une amine de formule
Q-NH₂
dans laquelle Q signifie quinoléine, isoquinoléine ou quinoléine (partiellement) hydrogénée ou isoquinoléine (partiellement) hydrogénée le cas échéant substituée selon la revendication 1,
en α-cétoamide (III) 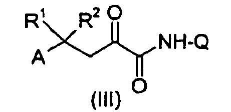 dans laquelle A, R¹, R² et Q ont les significations indiquées dans la revendication 1,
le cas échéant en présence de réactifs de couplage de déshydratation ou après transformation de la fonction acide en chlorure d'acide de manière connue par l'homme du métier,
qui est ensuite transformé
soit
avec un composé alkylmétal
ou
avec un composé de silicium de formule (IV)
(R⁹)₃-Si-R³ (IV)
dans laquelle R³ représente un groupe C₁-C₃-alkyle ou un groupe C₁-C₃-alkyle le cas échéant partiellement ou complètement fluoré et R⁹ signifie un groupe C₁-C₅-alkyle,
en présence d'un catalyseur en composés de formule générale (I),
ou
A2)
on estérifie un α-cétoacide (II) 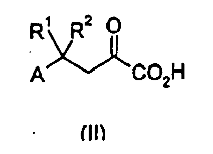 selon un procédé connu par l'homme du métier, puis on transforme l'α-cétoester (V) obtenu 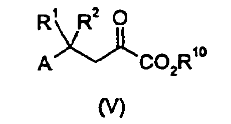 où A, R¹ et R² ont les significations susmentionnées et R¹⁰ signifie C₁-C₄-alkyle,
comme décrit dans A1) avec un composé alkylmétal ou un composé de silicium de formule (IV) en un composé de formule générale (VI) 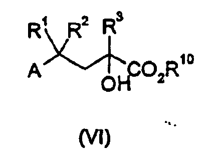 dans laquelle R¹⁰ signifie C₁-C₄-alkyle,
on saponifie le cas échéant l'ester selon le procédé connu par l'homme du métier, et on transforme ensuite le composé de formule (VI) avec une amine de formule
Q-NH₂
dans laquelle Q signifie quinoléine, isoquinoléine ou quinoléine (partiellement) hydrogénée ou isoquinoléine (partiellement) hydrogénée,
avec utilisation d'un réactif d'activation, le cas échéant en présence d'un catalyseur en un composé de formule (I),
ou
pour le cas où B signifie un groupe méthylène le cas échéant substitué par méthyle ou éthyle,
B1)
soit
on transforme un composé de formule (VII) ou (VIII) 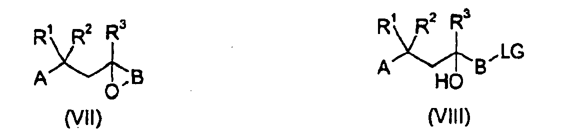 dans laquelle A, R¹, R² et R³ ont les significations susmentionnées, B représente un groupe méthylène le cas échéant substitué par un groupe méthyle ou éthyle et LG représente un groupe partant quelconque,
avec un composé de formule (IX) ou (X) 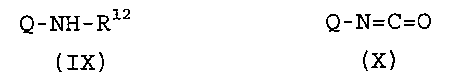 dans lesquelles R¹² représente un atome d'hydrogène, un groupe C₁-C₅-acyle ou un groupe alcoxy ou un groupe aryloxycarbonyle et Q signifie quinoléine, isoquinoléine ou quinoléine (partiellement) hydrogénée ou isoquinoléine (partiellement) hydrogénée,
et on dissocie une oxazolidinone le cas échéant formée de manière intermédiaire, de manière. à obtenir un composé de formule (I),
ou
B2)
on transforme des composés de formules (VII) ou (VIII) avec des nucléophiles azotés,
et on réduit ensuite le cas échéant avec des réactifs connus par l'homme du métier ou on réalise une hydrogénolyse catalysée par un métal de transition, de manière à obtenir des composés de formule (XI) 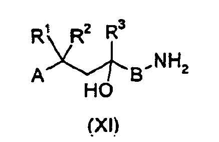 les radicaux A, B, R¹ R² et R³ ayant les significations susmentionnées
qui sont ensuite transformés, le cas échéant sous une catalyse par une base ou par un métal de transition avec un dérivé, halogéné selon un procédé connu par l'homme du métier, de la quinoléine, de l'isoquinoléine ou de la quinoléine (partiellement) hydrogénée ou de l'isoquinoléine (partiellement) hydrogénée
ou
B3)
**en ce que** des composés de formule (XII) préparés selon des procédés connus par l'homme du métier 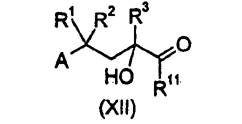 dans laquelle A, R¹, R² et R³ ont les significations susmentionnées et R¹¹ signifie méthyle ou éthyle,
sont transformés dans des conditions d'une amination réductive avec des composés de formule
Q-NH₂
dans laquelle Q a la signification susmentionnée, en composés de formule générale (I).
